# EUROPEAN PATENT APPLICATION

(11) **EP 4 837 921 A1**
(43) Date of publication of application: **11.11.2026**
(21) Application number: 25175474.3
(22) Date of filing: 09.05.2025
(51) Int. Cl.: C07K 16/30, C07K 16/28, A61K 39/00, A61P 35/00

(54) **ANTIGEN BINDING PROTEINS SPECIFICALLY BINDING MAGE-A3**

(71) Applicant: BioCopy AG, 4057 Basel (CH)
(72) Inventor: Krauss, Jan, 80331 Munich (DE); Heiß, Kirsten, 79268 Bötzingen (DE); Kilb, Normann, 79211 Denzlingen (DE); Gross, Tabea, 79104 Freiburg (DE); Jünemann, Gloria, 77960 Seelbach (DE); Schuster, Simon, 79241 Ihringen (DE); Reinemann, Christine, 04416 Markkleeberg (DE)
(74) Representative: Krauss, Jan

(57) **Abstract**

The present invention relates to antigen binding proteins that specifically bind to a MAGE-A3 antigenic peptide wherein said antigenic peptide is in a complex with a major histocompatibility complex (MHC) protein. The present invention further relates to nucleic acid molecules encoding an antigen binding protein, vectors comprising the nucleic acid molecules, or recombinant or genetically modified cells comprising and optionally expressing the nucleic acid molecule or vector. The invention further relates to pharmaceutical compositions comprising the antigen binding proteins, nucleic acids, vectors or cells in accordance with the invention, and their use as a medicament in the prevention or treatment of a cancer and/or a metastasis thereof.

## Description

The present invention relates to antigen binding proteins that specifically bind to a MAGE-A3 antigenic peptide wherein said antigenic peptide is in a complex with a major histocompatibility complex (MHC) protein. The present invention further relates to nucleic acid molecules encoding an antigen binding protein, vectors comprising the nucleic acid molecules, or recombinant or genetically modified cells comprising and optionally expressing the nucleic acid molecule or vector. The invention further relates to pharmaceutical compositions comprising the antigen binding proteins, nucleic acids, vectors or cells in accordance with the invention, and their use as a medicament in the prevention or treatment of a cancer and/or a metastasis thereof.

### Background of the invention

The MAGE-A3 Peptide 112-120 (KVAELVHFL (SEQ ID NO: 1)) is a peptide corresponding to MAGE-A3 (Melanoma-associated antigen 3, UniProt: P43357) from Homo sapiens (human)), amino acids 112-120. The MAGE-A3 peptide is used to stimulate human MAGE-A3-specific CD8+ T cells. KVAELVHFL is a linear peptidic epitope also studied as part Melanoma-associated antigen 9 (UniProt:P43362) of Homo sapiens (human).

MAGE proteins are CT (cancer testis) antigens, and there are about 60 proteins in the MAGE family that can be subdivided into type I (present only on the X-chromosome, MAGE-A, B, and C) and type II (MAGE D-L and NECDIN). Under normal conditions they are mostly found in the testis and placenta. Nevertheless, they are found at high levels in several cancer types, such as melanoma, brain, and breast cancer, and are involved in the development of resistance to chemotherapy, cell motility, and cell survival. Expression of MAGE proteins tends to correlate with a poor prognosis. MAGE are intracellular proteins, with MAGE-A1 being mostly cytosolic, making them poor targets for strategies such as CAR-T cell therapy. MAGE proteins are degraded in the proteosome, and the peptides created can then be found on the cell membrane in combination with MHC (major histocompatibility complex) I.

The presentation on the cell surface in this form makes them an attractive target for TCR (T cell receptor)-T cell therapy. Several clinical trials are ongoing, and either alone or in combination with other forms of cancer therapy the use of TCR-T cells targeting MAGE-A antigens may become a promising therapeutical avenue

MAGE-A3 is a protein and member of the MAGE-A gene family. Expression in normal tissue is in general restricted to the immune privileged testis and placenta. The normal function of MAGE-A3 in healthy cells is unknown. The presence of the antigen on tumor cells has been associated with worse prognosis. In one study, high levels of MAGE-A3 in lung adenocarcinoma were associated with shorter survival.

MAGE-A3 is a tumor-specific protein, and has been identified in many tumors including melanoma, non-small cell lung cancer, hematologic malignancies, among others. Currently, a cancer vaccine targeting MAGE-A3 is developed. The vaccine is a fusion protein of MAGE-A3 and *Haemophilus influenzae* protein D, combined with an immunoadjuvant.

Chinnasamy N, et al. (in: A TCR targeting the HLA-A*0201-restricted epitope of MAGE-A3 recognizes multiple epitopes of the MAGE-A antigen superfamily in several types of cancer. J Immunol. 2011 Jan 15;186(2):685-96. doi: 10.4049/jimmunol.1001775. Epub 2010 Dec 13. PMID: 21149604; PMCID: PMC6292200) employed a transgenic mouse model that expresses the human HLA-A*0201 molecule. Mice were immunized with two HLA-A*0201-restricted peptides of MAGE-A3: 112-120 (KVAELVHFL) (SEQ ID NO: 1) or MAGE-A3: 271-279 (FLWGPRALV) (SEQ ID NO: 65), and T cell clones were generated. MAGE-A3-specific TCR α- and β-chains were isolated and cloned into a retroviral vector. Expression of both TCRs in human PBLs demonstrated Ag-specific reactivity against a range of melanoma and nonmelanoma tumor cells. The TCR against MAGE-A3 : 112-120 was selected for further development based on superior reactivity against tumor target cells. Interestingly, peptide epitopes from MAGE-A3 and MAGE-A12 (and to a lesser extent, peptides from MAGE-A2 and MAGE-A6) were recognized by PBLs engineered to express this TCR. To further improve TCR function, single amino acid variants of the CDR3 α-chain were generated. Substitution of alanine to threonine at position 118 of the α-chain in the CDR3 region of the TCR improved its functional avidity in CD4 and CD8 cells.

Chen L, et al. (in: A humanized TCR retaining authentic specificity and affinity conferred potent anti-tumour cytotoxicity. Immunology. 2018 Sep;155(1):123-136. doi: 10.1111/imm.12935. Epub 2018 May 7. PMID: 29645087; PMCID: PMC6099166) disclose a TCR that was generated previously specific for human tumor testis antigen peptide MAGE-A3₁₁₂₋₁₂₀ (KVAELVHFL) HLA-A*02 complex. They developed an approach to humanize the murine TCR by replacing the mouse framework with sequences of folding optimized human TCR variable domains for retaining binding affinity. The resultant humanized TCR exhibited higher affinity and conferred better anti-tumor activity than its parent murine MAGE-A3 TCR (SRm1).

WO 2014/127478A1 describes a kit for use in inducing an immune response in a mammal, the kit includes: a first virus that expresses MAGE-A3, Human Papilloma Virus E6/E7 fusion protein, human Six-Transmembrane Epithelial Antigen of the Prostate protein, or Cancer Testis Antigen 1, or a variant thereof as an antigenic protein and that is formulated to generate an immunity to the protein or variant thereof in the mammal.

WO 2019/235915 A1 describes bispecific immunoglobulins of which one arm specifically binds to an MHC-MAGE derived peptide complex, and the other arm specifically recognizes a target associated with immune effector cells (xCD3). Listed are MAGE-derived Tc epitopes, including MAGE-A3 112-120. Presented molecules recognize HLA-A2/mMA peptide (YLEYEQVPG, SEQ ID NO: 45) and HLA-A2/MAGE-A3 peptide FLWGPRALV (SEQ ID NO: 65). TCR-like antibodies and CARs are disclosed.

WO 2021/173674A1 relates to antibodies and antigen-binding fragments thereof that bind to a MAGE-A3 peptide-MHC complex (pMHC) thereof. Also provided are chimeric antigen receptors and host cells comprising them. The compositions of the disclosure are useful in the diagnosis and treatment of cancer, including hematological malignancies, solid tumors, and epithelial cancers. In particular, provided are polypeptides that specifically bind to a pMHC complex displaying a MAGE-A3 peptide over an EPS8L2 pMHC complex displaying an EPS8L2 peptide, and methods of use thereof in treating cancer. In one aspect, the disclosure provides a polypeptide, comprising an antibody or antigen-binding fragment thereof that specifically binds to a peptide:MHC (pMHC) complex displaying a MAGE-A3₁₁₂₋₁₂₀ peptide comprising the sequence of KVAELVHFL (SEQ ID NO: 1).

While advances have been made in the development of molecular-targeting drugs for cancer therapy, there remains a need in the art to develop new anti-cancer agents that specifically target molecules highly specific to cancer cells but not to normal cells.

It is therefore an object of the present invention to provide these new anti-cancer agents, as well as compositions comprising these agents, as well as uses thereof. Other objects of the present invention will become apparent to the person of skill when studying the specification of the present invention.

In a first aspect thereof, the object of the present invention is solved by providing an antigen binding protein specifically binding to a MAGE-A antigenic peptide comprising or consisting of the amino acid sequence KVAELVHFL of SEQ ID NO: 1, wherein said antigenic peptide is preferably in a complex with a major histocompatibility complex (MHC) protein.

The antigen binding protein according to the present invention comprises a first polypeptide chain comprising a first variable domain comprising three complementary determining regions (CDRs) CDRH1, CDRH2 and CDRH3, wherein
i) the CDRH1 comprises or consists of the amino acid sequence DYTMH (SEQ ID NO: 96), or an amino acid sequence differing from SEQ ID NO: 96 by one or two amino acid substitutions,
   the CDRH2 comprises or consists of the amino acid sequence LISWDGGSTYYADSVKG (SEQ ID NO: 97), or an amino acid sequence differing from SEQ ID NO: 97 by one or two amino acid substitutions,
   the CDRH3 comprises or consists of the amino acid sequence DKGWLQLGN (SEQ ID NO: 98), or an amino acid sequence differing from SEQ ID NO: 98 by one or two amino acid substitutions, or
ii) the CDRH1 comprises or consists of the amino acid sequence SYAIS (SEQ ID NO: 78), or an amino acid sequence differing from SEQ ID NO: 78 by one or two amino acid substitutions,
   the CDRH2 comprises or consists of the amino acid sequence GIIPIFGTANYAQKFQG (SEQ ID NO: 79), or an amino acid sequence differing from SEQ ID NO: 79 by one or two amino acid substitutions,
   the CDRH3 comprises or consists of the amino acid sequence GGAPAVWSYFDY (SEQ ID NO: 80), or an amino acid sequence differing from SEQ ID NO: 80 by one or two amino acid substitutions, or
iii) the CDRH1 comprises or consists of the amino acid sequence SYGMH (SEQ ID NO: 2), or an amino acid sequence differing from SEQ ID NO: 2 by one or two amino acid substitutions,
   the CDRH2 comprises or consists of the amino acid sequence VISYDGSNKYYADSVKG (SEQ ID NO: 3), or an amino acid sequence differing from SEQ ID NO: 3 by one or two amino acid substitutions,
   the CDRH3 comprises or consists of the amino acid sequence DLVGGKTWDYGMDV (SEQ ID NO: 4), or an amino acid sequence differing from SEQ ID NO: 4 by one or two amino acid substitutions, or
iv) the CDRH1 comprises or consists of the amino acid sequence NYAMT (SEQ ID NO: 8), or an amino acid sequence differing from SEQ ID NO: 8 by one or two amino acid substitutions,
   the CDRH2 comprises or consists of the amino acid sequence AISGSRGSTYYADSVKG (SEQ ID NO: 9), or an amino acid sequence differing from SEQ ID NO: 9 by one or two amino acid substitutions,
   the CDRH3 comprises or consists of the amino acid sequence DNWNFPFYLDY (SEQ ID NO: 10), or an amino acid sequence differing from SEQ ID NO: 10 by one or two amino acid substitutions, or
v) the CDRH1 comprises or consists of the amino acid sequence SYAIS (SEQ ID NO: 14), or an amino acid sequence differing from SEQ ID NO: 14 by one or two amino acid substitutions,
   the CDRH2 comprises or consists of the amino acid sequence GIIPIFGAANYAQKFQG (SEQ ID NO: 15), or an amino acid sequence differing from SEQ ID NO: 15 by one or two amino acid substitutions,
   the CDRH3 comprises or consists of the amino acid sequence GGLESSYGMDV (SEQ ID NO: 16), or an amino acid sequence differing from SEQ ID NO: 16 by one or two amino acid substitutions, or
vi) the CDRH1 comprises or consists of the amino acid sequence SYAIS (SEQ ID NO: 66), or an amino acid sequence differing from SEQ ID NO: 66 by one or two amino acid substitutions,
   the CDRH2 comprises or consists of the amino acid sequence GIIPIFGTANYAQKFQG (SEQ ID NO: 67), or an amino acid sequence differing from SEQ ID NO: 67 by one or two amino acid substitutions,
   the CDRH3 comprises or consists of the amino acid sequence GIKGYAFDI (SEQ ID NO: 68), or an amino acid sequence differing from SEQ ID NO: 68 by one or two amino acid substitutions, or
vii) the CDRH1 comprises or consists of the amino acid sequence SYWMT (SEQ ID NO: 72), or an amino acid sequence differing from SEQ ID NO: 72 by one or two amino acid substitutions,
   the CDRH2 comprises or consists of the amino acid sequence NIQQDGSEKYYLDSVKG (SEQ ID NO: 73), or an amino acid sequence differing from SEQ ID NO: 73 by one or two amino acid substitutions,
   the CDRH3 comprises or consists of the amino acid sequence DWAYFDY (SEQ ID NO: 74), or an amino acid sequence differing from SEQ ID NO: 74 by one or two amino acid substitutions, or
viii) the CDRH1 comprises or consists of the amino acid sequence SYYMH (SEQ ID NO: 84), or an amino acid sequence differing from SEQ ID NO: 84 by one or two amino acid substitutions,
   the CDRH2 comprises or consists of the amino acid sequence IINPSGGSTSYAQKFQG (SEQ ID NO: 85), or an amino acid sequence differing from SEQ ID NO: 85 by one or two amino acid substitutions,
   the CDRH3 comprises or consists of the amino acid sequence DKSLGWFDP (SEQ ID NO: 86), or an amino acid sequence differing from SEQ ID NO: 86 by one or two amino acid substitutions, or
ix) the CDRH1 comprises or consists of the amino acid sequence SYAMS (SEQ ID NO: 90), or an amino acid sequence differing from SEQ ID NO: 90 by one or two amino acid substitutions,
   the CDRH2 comprises or consists of the amino acid sequence AISGSGGSTYYADSVKG (SEQ ID NO: 91), or an amino acid sequence differing from SEQ ID NO: 91 by one or two amino acid substitutions,
   the CDRH3 comprises or consists of the amino acid sequence ELGLRGAFDI (SEQ ID NO: 92), or an amino acid sequence differing from SEQ ID NO: 92 by one or two amino acid substitutions, or
x) the CDRH1 comprises or consists of the amino acid sequence SYAIS (SEQ ID NO: 116), or an amino acid sequence differing from SEQ ID NO: 116 by one or two amino acid substitutions,
   the CDRH2 comprises or consists of the amino acid sequence GIIPIFGTANYAQKFQG (SEQ ID NO: 117), or an amino acid sequence differing from SEQ ID NO: 117 by one or two amino acid substitutions,
   the CDRH3 comprises or consists of the amino acid sequence QTGYSSGWPSPDAFDI (SEQ ID NO: 118), or an amino acid sequence differing from SEQ ID NO: 118 by one or two amino acid substitutions, or
xi) the CDRH1 comprises or consists of the amino acid sequence SYAIS (SEQ ID NO: 124), or an amino acid sequence differing from SEQ ID NO: 124 by one or two amino acid substitutions,
   the CDRH2 comprises or consists of the amino acid sequence GIIPIFGTANYAQKFQG (SEQ ID NO: 125), or an amino acid sequence differing from SEQ ID NO: 125 by one or two amino acid substitutions,
   the CDRH3 comprises or consists of the amino acid sequence AVGIFGAFDI (SEQ ID NO: 126), or an amino acid sequence differing from SEQ ID NO: 126 by one or two amino acid substitutions, or
xii) the CDRH1 comprises or consists of the amino acid sequence SYAIS (SEQ ID NO: 132), or an amino acid sequence differing from SEQ ID NO: 132 by one or two amino acid substitutions,
   the CDRH2 comprises or consists of the amino acid sequence GIIPIFGTANYAQKFQG (SEQ ID NO: 133), or an amino acid sequence differing from SEQ ID NO: 133 by one or two amino acid substitutions,
   the CDRH3 comprises or consists of the amino acid sequence DSYDSSGYYYDWFDP (SEQ ID NO: 134), or an amino acid sequence differing from SEQ ID NO: 134 by one or two amino acid substitutions, or
xiii) the CDRH1 comprises or consists of the amino acid sequence NYAIS (SEQ ID NO: 140), or an amino acid sequence differing from SEQ ID NO: 140 by one or two amino acid substitutions,
   the CDRH2 comprises or consists of the amino acid sequence GIIPIFGTANYAQKFQG (SEQ ID NO: 141), or an amino acid sequence differing from SEQ ID NO: 141 by one or two amino acid substitutions,
   the CDRH3 comprises or consists of the amino acid sequence DYREGYSYGLYDAFDI (SEQ ID NO: 142), or an amino acid sequence differing from SEQ ID NO: 142 by one or two amino acid substitutions, or
xiv) the CDRH1 comprises or consists of the amino acid sequence SYAIS (SEQ ID NO: 148), or an amino acid sequence differing from SEQ ID NO: 148 by one or two amino acid substitutions,
   the CDRH2 comprises or consists of the amino acid sequence GIIPIFGTANYAQKFQG (SEQ ID NO: 149), or an amino acid sequence differing from SEQ ID NO: 149 by one or two amino acid substitutions,
   the CDRH3 comprises or consists of the amino acid sequence GGLESSYGMDV (SEQ ID NO: 150), or an amino acid sequence differing from SEQ ID NO: 150 by one or two amino acid substitutions.

Preferred is the antigen binding protein according to the present invention, further comprising a second polypeptide chain comprising a second variable domain comprising three CDRs CDRLl, CDRL2 and CDRL3, wherein
the CDRL2 comprises or consists of the amino acid sequence DISERPS (SEQ ID NO: 100), or an amino acid sequence differing from SEQ ID NO: 100 by one or two amino acid substitutions, and
the CDRL3 comprises or consists of the amino acid sequence SSYTATSVI (SEQ ID NO: 101), or an amino acid sequence differing from SEQ ID NO: 101 by one or two amino acid substitutions, or
b) the CDRLl comprises or consists of the amino acid sequence SGSSSNIGNNAVN (SEQ ID NO: 81), or an amino acid sequence differing from SEQ ID NO: 81 by one or two amino acid substitutions,
the CDRL2 comprises or consists of the amino acid sequence YDDLLPS (SEQ ID NO: 82), or an amino acid sequence differing from SEQ ID NO: 82 by one or two amino acid substitutions, and
the CDRL3 comprises or consists of the amino acid sequence AAWDDSLNGLV (SEQ ID NO: 83), or an amino acid sequence differing from SEQ ID NO: 83 by one or two amino acid substitutions, or
c) the CDRLl comprises or consists of the amino acid sequence RASQSINSYLN (SEQ ID NO: 5), or an amino acid sequence differing from SEQ ID NO: 5 by one or two amino acid substitutions,
the CDRL2 comprises or consists of the amino acid sequence AASSLQS (SEQ ID NO: 6), or an amino acid sequence differing from SEQ ID NO: 6 by one or two amino acid substitutions,
and the CDRL3 comprises or consists of the amino acid sequence QQSYSTPRT (SEQ ID NO: 7), or an amino acid sequence differing from SEQ ID NO: 7 by one or two amino acid substitutions, or
d) the CDRLl comprises or consists of the amino acid sequence RASQGISNWLA (SEQ ID NO: 11), or an amino acid sequence differing from SEQ ID NO: 11 by one or two amino acid substitutions,
the CDRL2 comprises or consists of the amino acid sequence AASNLQG (SEQ ID NO: 12), or an amino acid sequence differing from SEQ ID NO: 12 by one or two amino acid substitutions,
and the CDRL3 comprises or consists of the amino acid sequence QQGYSFPHT (SEQ ID NO: 13), or an amino acid sequence differing from SEQ ID NO: 13 by one or two amino acid substitutions, or
e) the CDRLl comprises or consists of the amino acid sequence SGSSSNIGNNAVN (SEQ ID NO: 17), or an amino acid sequence differing from SEQ ID NO: 17 by one or two amino acid substitutions,
the CDRL2 comprises or consists of the amino acid sequence YDDLLPS (SEQ ID NO: 18), or an amino acid sequence differing from SEQ ID NO: 18 by one or two amino acid substitutions,
and the CDRL3 comprises or consists of the amino acid sequence AAWDDSLNGLV (SEQ ID NO: 19), or an amino acid sequence differing from SEQ ID NO: 19 by one or two amino acid substitutions, or
f) the CDRL1 comprises or consists of the amino acid sequence SGSSSNIGAGYDVH (SEQ ID NO: 69), or an amino acid sequence differing from SEQ ID NO: 69 by one or two amino acid substitutions,
the CDRL2 comprises or consists of the amino acid sequence GNSNRPS (SEQ ID NO: 70), or an amino acid sequence differing from SEQ ID NO: 70 by one or two amino acid substitutions, and
the CDRL3 comprises or consists of the amino acid sequence QSYDSSLTGV (SEQ ID NO: 71), or an amino acid sequence differing from SEQ ID NO: 71 by one or two amino acid substitutions, or
g) the CDRLl comprises or consists of the amino acid sequence SGDALPKKYAY (SEQ ID NO: 75), or an amino acid sequence differing from SEQ ID NO: 75 by one or two amino acid substitutions,
the CDRL2 comprises or consists of the amino acid sequence GDSKRPS (SEQ ID NO: 76), or an amino acid sequence differing from SEQ ID NO: 76 by one or two amino acid substitutions, and
the CDRL3 comprises or consists of the amino acid sequence YSTDSSGNQGV (SEQ ID NO: 77), or an amino acid sequence differing from SEQ ID NO: 77 by one or two amino acid substitutions, or
h) the CDRLl comprises or consists of the amino acid sequence RASQSISSYLN (SEQ ID NO: 87), or an amino acid sequence differing from SEQ ID NO: 87 by one or two amino acid substitutions,
the CDRL2 comprises or consists of the amino acid sequence AASSLQS (SEQ ID NO: 88), or an amino acid sequence differing from SEQ ID NO: 88 by one or two amino acid substitutions,
and the CDRL3 comprises or consists of the amino acid sequence QQSYSTPFT (SEQ ID NO: 89), or an amino acid sequence differing from SEQ ID NO: 89 by one or two amino acid substitutions, or
i) the CDRLl comprises or consists of the amino acid sequence SGSRSNIGTNTVN (SEQ ID NO: 93), or an amino acid sequence differing from SEQ ID NO: 93 by one or two amino acid substitutions,
the CDRL2 comprises or consists of the amino acid sequence NNLQRPS (SEQ ID NO: 94), or an amino acid sequence differing from SEQ ID NO: 94 by one or two amino acid substitutions, and
the CDRL3 comprises or consists of the amino acid sequence ATWDDSLKGVV (SEQ ID NO: 95), or an amino acid sequence differing from SEQ ID NO: 95 by one or two amino acid substitutions,, or
j) the CDRLl comprises or consists of the amino acid sequence RASQGISSYLA (SEQ ID NO: 119), or an amino acid sequence differing from SEQ ID NO: 119 by one or two amino acid substitutions,
the CDRL2 comprises or consists of the amino acid sequence AASTLQS (SEQ ID NO: 120), or an amino acid sequence differing from SEQ ID NO: 120 by one or two amino acid substitutions, and
the CDRL3 comprises or consists of the amino acid sequence QQLNSYPPT (SEQ ID NO: 121), or an amino acid sequence differing from SEQ ID NO: 121 by one or two amino acid substitutions, or
k) the CDRLl comprises or consists of the amino acid sequence SGSNSNIGSNTVN (SEQ ID NO: 127), or an amino acid sequence differing from SEQ ID NO: 127 by one or two amino acid substitutions,
the CDRL2 comprises or consists of the amino acid sequence SNNQRPS (SEQ ID NO: 128), or an amino acid sequence differing from SEQ ID NO: 128 by one or two amino acid substitutions, and
the CDRL3 comprises or consists of the amino acid sequence AAWDDSLNGPYVV (SEQ ID NO: 129), or an amino acid sequence differing from SEQ ID NO: 129 by one or two amino acid substitutions, or
l) the CDRLl comprises or consists of the amino acid sequence TGTSSDVGGYDFVS (SEQ ID NO: 135), or an amino acid sequence differing from SEQ ID NO: 135 by one or two amino acid substitutions,
the CDRL2 comprises or consists of the amino acid sequence EVSTRHS (SEQ ID NO: 136), or an amino acid sequence differing from SEQ ID NO: 136 by one or two amino acid substitutions, and
the CDRL3 comprises or consists of the amino acid sequence SSFTSSPSVV (SEQ ID NO: 137), or an amino acid sequence differing from SEQ ID NO: 137 by one or two amino acid substitutions, or
m) the CDRLl comprises or consists of the amino acid sequence TGTSSDVGGYNYVS (SEQ ID NO: 143), or an amino acid sequence differing from SEQ ID NO: 143 by one or two amino acid substitutions,
the CDRL2 comprises or consists of the amino acid sequence DVSNRPS (SEQ ID NO: 144), or an amino acid sequence differing from SEQ ID NO: 144 by one or two amino acid substitutions, and
the CDRL3 comprises or consists of the amino acid sequence SSYRSSYTLV (SEQ ID NO: 145), or an amino acid sequence differing from SEQ ID NO: 145 by one or two amino acid substitutions, or
n) the CDRLl comprises or consists of the amino acid sequence SGSSSNIGNNAVN (SEQ ID NO: 151), or an amino acid sequence differing from SEQ ID NO: 151 by one or two amino acid substitutions,
the CDRL2 comprises or consists of the amino acid sequence YDDLLPS (SEQ ID NO: 152), or an amino acid sequence differing from SEQ ID NO: 152 by one or two amino acid substitutions, and
the CDRL3 comprises or consists of the amino acid sequence AAWDDSLSGYV (SEQ ID NO: 153), or an amino acid sequence differing from SEQ ID NO: 153 by one or two amino acid substitutions.

In one embodiment of the antigen binding protein according to the present invention, the CDRH1 comprises or consists of the amino acid sequence DYTMH (SEQ ID NO: 96), or an amino acid sequence differing from SEQ ID NO: 96 by at least one amino acid substitution, the CDRH2 comprises or consists of the amino acid sequence LISWDGGSTYYADSVKG (SEQ ID NO: 97), or an amino acid sequence differing from SEQ ID NO: 97 by at least one amino acid substitution, the CDRH3 comprises or consists of the amino acid sequence DKGWLQLGN (SEQ ID NO: 98), or an amino acid sequence differing from SEQ ID NO: 98 by at least one amino acid substitution, and the CDRLl comprises or consists of the amino acid sequence IGTSSDVGGYNSVS (SEQ ID NO: 99), or an amino acid sequence differing from SEQ ID NO: 99 by at least one amino acid substitution, the CDRL2 comprises or consists of the amino acid sequence DISERPS (SEQ ID NO: 100), or an amino acid sequence differing from SEQ ID NO: 100 by at least one amino acid substitution, and the CDRL3 comprises or consists of the amino acid sequence SSYTATSVI (SEQ ID NO: 101), or an amino acid sequence differing from SEQ ID NO: 101 by at least one amino acid substitution.

In another embodiment of the antigen binding protein according to the present invention, the CDRH1 comprises or consists of the amino acid sequence SYAIS (SEQ ID NO: 78), or an amino acid sequence differing from SEQ ID NO: 78 by at least one amino acid substitution, the CDRH2 comprises or consists of the amino acid sequence GIIPIFGTANYAQKFQG (SEQ ID NO: 79), or an amino acid sequence differing from SEQ ID NO: 79 by at least one amino acid substitution, the CDRH3 comprises or consists of the amino acid sequence GGAPAVWSYFDY (SEQ ID NO: 80), or an amino acid sequence differing from SEQ ID NO: 80 by at least one amino acid substitution, and the CDRLl comprises or consists of the amino acid sequence SGSSSNIGNNAVN (SEQ ID NO: 81), or an amino acid sequence differing from SEQ ID NO: 81 by at least one amino acid substitution, the CDRL2 comprises or consists of the amino acid sequence YDDLLPS (SEQ ID NO: 82), or an amino acid sequence differing from SEQ ID NO: 82 by at least one amino acid substitution, and the CDRL3 comprises or consists of the amino acid sequence AAWDDSLNGLV (SEQ ID NO: 83), or an amino acid sequence differing from SEQ ID NO: 83 by at least one amino acid substitution.

In another embodiment of the antigen binding protein according to the present invention, the CDRH1 comprises or consists of the amino acid sequence SYGMH (SEQ ID NO: 2), or an amino acid sequence differing from SEQ ID NO: 2 by at least one amino acid substitution, the CDRH2 comprises or consists of the amino acid sequence VISYDGSNKYYADSVKG (SEQ ID NO: 3), or an amino acid sequence differing from SEQ ID NO: 3 by at least one amino acid substitution, and the CDRH3 comprises or consists of the amino acid sequence DLVGGKTWDYGMDV (SEQ ID NO: 4), or an amino acid sequence differing from SEQ ID NO: 4 by at least one amino acid substitution, and the CDRLl comprises or consists of the amino acid sequence RASQSINSYLN (SEQ ID NO: 5), or an amino acid sequence differing from SEQ ID NO: 5 by at least one amino acid substitution, the CDRL2 comprises or consists of the amino acid sequence AASSLQS (SEQ ID NO: 6), or an amino acid sequence differing from SEQ ID NO: 6 by at least one amino acid substitution, and the CDRL3 comprises or consists of the amino acid sequence QQSYSTPRT (SEQ ID NO: 7), or an amino acid sequence differing from SEQ ID NO: 7 by at least one amino acid substitution. In another embodiment of the antigen binding protein according to the present invention, the CDRH1 comprises or consists of the amino acid sequence NYAMT (SEQ ID NO: 8), or an amino acid sequence differing from SEQ ID NO: 8 by at least one amino acid substitution, the CDRH2 comprises or consists of the amino acid sequence AISGSRGSTYYADSVKG (SEQ ID NO: 9), or an amino acid sequence differing from SEQ ID NO: 9 by at least one amino acid substitution, the CDRH3 comprises or consists of the amino acid sequence DNWNFPFYLDY (SEQ ID NO: 10), or an amino acid sequence differing from SEQ ID NO: 10 by at least one amino acid substitution, and the CDRLl comprises or consists of the amino acid sequence RASQGISNWLA (SEQ ID NO: 11), or an amino acid sequence differing from SEQ ID NO: 11 by at least one amino acid substitution, the CDRL2 comprises or consists of the amino acid sequence AASNLQG (SEQ ID NO: 12), or an amino acid sequence differing from SEQ ID NO: 12 by at least one amino acid substitution, and the CDRL3 comprises or consists of the amino acid sequence QQGYSFPHT (SEQ ID NO: 13), or an amino acid sequence differing from SEQ ID NO: 13 by at least one amino acid substitution. In another embodiment of the antigen binding protein according to the present invention, the CDRH1 comprises or consists of the amino acid sequence SYAIS (SEQ ID NO: 14), or an amino acid sequence differing from SEQ ID NO: 14 by at least one amino acid substitution, the CDRH2 comprises or consists of the amino acid sequence GIIPIFGAANYAQKFQG (SEQ ID NO: 15), or an amino acid sequence differing from SEQ ID NO: 15 by at least one amino acid substitution, the CDRH3 comprises or consists of the amino acid sequence GGLESSYGMDV (SEQ ID NO: 16), or an amino acid sequence differing from SEQ ID NO: 16 by at least one amino acid substitution, and the CDRLl comprises or consists of the amino acid sequence SGSSSNIGNNAVN (SEQ ID NO: 17), or an amino acid sequence differing from SEQ ID NO: 17 by at least one amino acid substitution, the CDRL2 comprises or consists of the amino acid sequence YDDLLPS (SEQ ID NO: 18), or an amino acid sequence differing from SEQ ID NO: 18 by at least one amino acid substitution, and the CDRL3 comprises or consists of the amino acid sequence AAWDDSLNGLV (SEQ ID NO: 19), or an amino acid sequence differing from SEQ ID NO: 19 by at least one amino acid substitution.

In another embodiment of the antigen binding protein according to the present invention, the CDRH1 comprises or consists of the amino acid sequence SYAIS (SEQ ID NO: 66), or an amino acid sequence differing from SEQ ID NO: 66 by at least one amino acid substitution, the CDRH2 comprises or consists of the amino acid sequence GIIPIFGTANYAQKFQG (SEQ ID NO: 67), or an amino acid sequence differing from SEQ ID NO: 67 by at least one amino acid substitution, the CDRH3 comprises or consists of the amino acid sequence GIKGYAFDI (SEQ ID NO: 68), or an amino acid sequence differing from SEQ ID NO: 68 by at least one amino acid substitution, and the CDRL1 comprises or consists of the amino acid sequence SGSSSNIGAGYDVH (SEQ ID NO: 69), or an amino acid sequence differing from SEQ ID NO: 69 by at least one amino acid substitution, the CDRL2 comprises or consists of the amino acid sequence GNSNRPS (SEQ ID NO: 70), or an amino acid sequence differing from SEQ ID NO: 70 by at least one amino acid substitution, and the CDRL3 comprises or consists of the amino acid sequence QSYDSSLTGV (SEQ ID NO: 71), or an amino acid sequence differing from SEQ ID NO: 71 by at least one amino acid substitution.

In another embodiment of the antigen binding protein according to the present invention, the CDRH1 comprises or consists of the amino acid sequence SYWMT (SEQ ID NO: 72), or an amino acid sequence differing from SEQ ID NO: 72 by at least one amino acid substitution, the CDRH2 comprises or consists of the amino acid sequence NIQQDGSEKYYLDSVKG (SEQ ID NO: 73), or an amino acid sequence differing from SEQ ID NO: 73 by at least one amino acid substitution, the CDRH3 comprises or consists of the amino acid sequence DWAYFDY (SEQ ID NO: 74), or an amino acid sequence differing from SEQ ID NO: 74 by at least one amino acid substitution, and the CDRLl comprises or consists of the amino acid sequence SGDALPKKYAY (SEQ ID NO: 75), or an amino acid sequence differing from SEQ ID NO: 75 by at least one amino acid substitution, the CDRL2 comprises or consists of the amino acid sequence GDSKRPS (SEQ ID NO: 76), or an amino acid sequence differing from SEQ ID NO: 76 by at least one amino acid substitution, and the CDRL3 comprises or consists of the amino acid sequence YSTDSSGNQGV (SEQ ID NO: 77), or an amino acid sequence differing from SEQ ID NO: 77 by at least one amino acid substitution.

In another embodiment of the antigen binding protein according to the present invention, the CDRH1 comprises or consists of the amino acid sequence SYYMH (SEQ ID NO: 84), or an amino acid sequence differing from SEQ ID NO: 84 by at least one amino acid substitution, the CDRH2 comprises or consists of the amino acid sequence IINPSGGSTSYAQKFQG (SEQ ID NO: 85), or an amino acid sequence differing from SEQ ID NO: 85 by at least one amino acid substitution, the CDRH3 comprises or consists of the amino acid sequence DKSLGWFDP (SEQ ID NO: 86), or an amino acid sequence differing from SEQ ID NO: 86 by at least one amino acid substitution, and the CDRLl comprises or consists of the amino acid sequence RASQSISSYLN (SEQ ID NO: 87), or an amino acid sequence differing from SEQ ID NO: 87 by at least one amino acid substitution, the CDRL2 comprises or consists of the amino acid sequence AASSLQS (SEQ ID NO: 88), or an amino acid sequence differing from SEQ ID NO: 88 by at least one amino acid substitution, and the CDRL3 comprises or consists of the amino acid sequence QQSYSTPFT (SEQ ID NO: 89), or an amino acid sequence differing from SEQ ID NO: 89 by at least one amino acid substitution.

In another embodiment of the antigen binding protein according to the present invention, the CDRH1 comprises or consists of the amino acid sequence SYAMS (SEQ ID NO: 90), or an amino acid sequence differing from SEQ ID NO: 90 by at least one amino acid substitution, the CDRH2 comprises or consists of the amino acid sequence AISGSGGSTYYADSVKG (SEQ ID NO: 91), or an amino acid sequence differing from SEQ ID NO: 91 by at least one amino acid substitution, the CDRH3 comprises or consists of the amino acid sequence ELGLRGAFDI (SEQ ID NO: 92), or an amino acid sequence differing from SEQ ID NO: 92 by at least one amino acid substitution, and the CDRLl comprises or consists of the amino acid sequence SGSRSNIGTNTVN (SEQ ID NO: 93), or an amino acid sequence differing from SEQ ID NO: 93 by at least one amino acid substitution, the CDRL2 comprises or consists of the amino acid sequence NNLQRPS (SEQ ID NO: 94), or an amino acid sequence differing from SEQ ID NO: 94 by at least one amino acid substitution, and the CDRL3 comprises or consists of the amino acid sequence ATWDDSLKGVV (SEQ ID NO: 95), or an amino acid sequence differing from SEQ ID NO: 95 by at least one amino acid substitution.

In another embodiment of the antigen binding protein according to the present invention, the CDRH1 comprises or consists of the amino acid sequence SYAIS (SEQ ID NO: 116), or an amino acid sequence differing from SEQ ID NO: 116 by at least one amino acid substitution, the CDRH2 comprises or consists of the amino acid sequence GIIPIFGTANYAQKFQG (SEQ ID NO: 117), or an amino acid sequence differing from SEQ ID NO: 117 by at least one amino acid substitution, the CDRH3 comprises or consists of the amino acid sequence QTGYSSGWPSPDAFDI (SEQ ID NO: 118), or an amino acid sequence differing from SEQ ID NO: 118 by at least one amino acid substitution, and the CDRLl comprises or consists of the amino acid sequence RASQGISSYLA (SEQ ID NO: 119), or an amino acid sequence differing from SEQ ID NO: 119 by at least one amino acid substitution, the CDRL2 comprises or consists of the amino acid sequence AASTLQS (SEQ ID NO: 120), or an amino acid sequence differing from SEQ ID NO: 120 by at least one amino acid substitution, and the CDRL3 comprises or consists of the amino acid sequence QQLNSYPPT (SEQ ID NO: 121), or an amino acid sequence differing from SEQ ID NO: 121 by at least one amino acid substitution.

In another embodiment of the antigen binding protein according to the present invention, the CDRH1 comprises or consists of the amino acid sequence SYAIS (SEQ ID NO: 124), or an amino acid sequence differing from SEQ ID NO: 124 by at least one amino acid substitution, the CDRH2 comprises or consists of the amino acid sequence GIIPIFGTANYAQKFQG (SEQ ID NO: 125), or an amino acid sequence differing from SEQ ID NO: 125 by at least one amino acid substitution, the CDRH3 comprises or consists of the amino acid sequence AVGIFGAFDI (SEQ ID NO: 126), or an amino acid sequence differing from SEQ ID NO: 126 by at least one amino acid substitution, and the CDRLl comprises or consists of the amino acid sequence SGSNSNIGSNTVN (SEQ ID NO: 127), or an amino acid sequence differing from SEQ ID NO: 127 by at least one amino acid substitution, the CDRL2 comprises or consists of the amino acid sequence SNNQRPS (SEQ ID NO: 128), or an amino acid sequence differing from SEQ ID NO: 128 by at least one amino acid substitution, and the CDRL3 comprises or consists of the amino acid sequence AAWDDSLNGPYVV (SEQ ID NO: 129), or an amino acid sequence differing from SEQ ID NO: 129 by at least one amino acid substitution.

In another embodiment of the antigen binding protein according to the present invention, the CDRH1 comprises or consists of the amino acid sequence SYAIS (SEQ ID NO: 132), or an amino acid sequence differing from SEQ ID NO: 132 by at least one amino acid substitution, the CDRH2 comprises or consists of the amino acid sequence GIIPIFGTANYAQKFQG (SEQ ID NO: 133), or an amino acid sequence differing from SEQ ID NO: 133 by at least one amino acid substitution, the CDRH3 comprises or consists of the amino acid sequence DSYDSSGYYYDWFDP (SEQ ID NO: 134), or an amino acid sequence differing from SEQ ID NO: 134 by at least one amino acid substitution, and the CDRLl comprises or consists of the amino acid sequence TGTSSDVGGYDFVS (SEQ ID NO: 135), or an amino acid sequence differing from SEQ ID NO: 135 by at least one amino acid substitution, the CDRL2 comprises or consists of the amino acid sequence EVSTRHS (SEQ ID NO: 136), or an amino acid sequence differing from SEQ ID NO: 136 by at least one amino acid substitution, and the CDRL3 comprises or consists of the amino acid sequence SSFTSSPSVV (SEQ ID NO: 137), or an amino acid sequence differing from SEQ ID NO: 137 by at least one amino acid substitution.

In another embodiment of the antigen binding protein according to the present invention, the CDRH1 comprises or consists of the amino acid sequence NYAIS (SEQ ID NO: 140), or an amino acid sequence differing from SEQ ID NO: 140 by at least one amino acid substitution, the CDRH2 comprises or consists of the amino acid sequence GIIPIFGTANYAQKFQG (SEQ ID NO: 141), or an amino acid sequence differing from SEQ ID NO: 141 by at least one amino acid substitution, the CDRH3 comprises or consists of the amino acid sequence DYREGYSYGLYDAFDI (SEQ ID NO: 142), or an amino acid sequence differing from SEQ ID NO: 142 by at least one amino acid substitution, and the CDRLl comprises or consists of the amino acid sequence TGTSSDVGGYNYVS (SEQ ID NO: 143), or an amino acid sequence differing from SEQ ID NO: 143 by at least one amino acid substitution, the CDRL2 comprises or consists of the amino acid sequence DVSNRPS (SEQ ID NO: 144), or an amino acid sequence differing from SEQ ID NO: 144 by at least one amino acid substitution, and the CDRL3 comprises or consists of the amino acid sequence SSYRSSYTLV (SEQ ID NO: 145), or an amino acid sequence differing from SEQ ID NO: 145 by at least one amino acid substitution.

In another embodiment of the antigen binding protein according to the present invention, the CDRH1 comprises or consists of the amino acid sequence SYAIS (SEQ ID NO: 148), or an amino acid sequence differing from SEQ ID NO: 148 by at least one amino acid substitution, the CDRH2 comprises or consists of the amino acid sequence GIIPIFGTANYAQKFQG (SEQ ID NO: 149), or an amino acid sequence differing from SEQ ID NO: 149 by at least one amino acid substitution, the CDRH3 comprises or consists of the amino acid sequence GGLESSYGMDV (SEQ ID NO: 150), or an amino acid sequence differing from SEQ ID NO: 150 by at least one amino acid substitution, and the CDRLl comprises or consists of the amino acid sequence SGSSSNIGNNAVN (SEQ ID NO: 151), or an amino acid sequence differing from SEQ ID NO: 151 by at least one amino acid substitution, the CDRL2 comprises or consists of the amino acid sequence YDDLLPS (SEQ ID NO: 152), or an amino acid sequence differing from SEQ ID NO: 152 by at least one amino acid substitution, and the CDRL3 comprises or consists of the amino acid sequence AAWDDSLSGYV (SEQ ID NO: 153), or an amino acid sequence differing from SEQ ID NO: 153 by at least one amino acid substitution. When mutated, preferred are amino acid sequences differing by one or two amino acid conservative substitutions.

The present invention thus provides novel antigen binding proteins that are specific for the tumor expressed antigen MAGE-A3 of SEQ ID NO: 1 in a complex with an MHC protein. It is desirable to design binder variants that bind with higher affinity to cancer antigens for use as antigen recognizing constructs in cancer therapy, e.g. as recognition module of a soluble approach, i.e. using bispecific molecules. However, increasing the affinity of binders may also increase the risk of side effects. Simply increasing the binder affinity for its target sequence is likely to also increase the affinity to similar non cancer-specific peptides and therefore increasing the risk of cross-reactivity and unwanted cytotoxic effects on healthy tissue.

Previously published results have shown lethal toxicities in two patients, who were infused with T cells engineered to express a TCR targeting MAGE-A3 cross-reacting with a peptide from the muscle protein Titin, even though no cross-reactivities had been predicted in the pre-clinical studies (Linette GP et al. Blood 2013;122:863-71 , Cameron BJ, et al. Sci. Transl. Med. 2013; 5: 197-103). These patients demonstrated that TCR-engineered T cells can have serious and unpredictable off-target and organ-specific toxicities.

Therefore, despite the advancements in peptide binder technology, there remains a need for additional cancer therapeutics, particularly those that efficiently target and kill cancer cells. In particular there is the need to develop new antigen binding proteins which are i) selective and specific for the tumor specific MAGE-A peptide of SEQ ID NO: 1 in a complex with an MHC protein having the desired biological activity, ii) have good metabolic, pharmacokinetic, solubility, stability and/or safety profiles, and iii) that can be manufactured in large scale.

Preferred is the antigen binding protein according to the present invention, comprising an amino acid sequence having at least 75%, preferably at least 90%, and more preferably at least 95% or at least 97%, 98% or 99/ sequence identity to a sequence selected from 
EVQLVESGGVVVQPGGSLRLSCAASGFTFDDYTMHWVRQAPGKGLEWVSLIS
WDGGSTYYADSVKGRFTISRDNSKNSLYLQMNSLRTEDTALYYCAKDKGWLQ
LGNWGQGTLVTVSS (SEQ ID NO: 112),
 and 
QSALTQPASVSGSPGQSITISCIGTSSDVGGYNSVSWYQQYPGRAPKLMIYDISE
RPSGVSTRFSGSKSGNTASLTISGLQAEDEADYYCSSYTATSVIFGGGTKVTVL
(SEQ ID NO: 103);
 
QVQLVQSGAEVKKPGSSVKVSCKASGGTFSSYAISWVRQAPGQGLEWMGGIIPI
FGTANYAQKFQGRVTITTDESMSTAYMELSSLRSEDTAVYYCARGGAPAVWS
YFDYWGQGTLVTVSS (SEQ ID NO: 106),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 107);
 
QVQLVQSGGGVVQPGRSLRLSCAASGFTFSSYGMHWVRQAPGKGLEWVAVIS
YDGSNKYYADSVKGRFTISRDNSKNTLYLQMNSLRAEDTAVYYCAKDLVGGK
TWDYGMDVWGQGTTVTVSS (SEQ ID NO: 20),
 and 
DIVMTQPPSSLSASVGDRVTITCRASQSINSYLNWYQQKPGKAPKLLIYAASSLQ
SGVPSRFSGSGSGTDFTLTISSLQPEDFATYYCQQSYSTPRTFGQGTKVEIK (SEQ
ID NO: 21);
 
QVQLVQSGGGLVQPGGSLRLSCAASGFTFNNYAMTWVRQAPGKGLEWVSAIS
GSRGSTYYADSVKGRFTISRDNSKNTLYLEMNSLRAEDTAVYFCAKDNWNFPF
YLDYWGQGTLVTVSS (SEQ ID NO: 22),
 and 
DIVMTQSPSSVSASVGDRVTITCRASQGISNWLAWYQQKPGKAPKLLIYAASNL
QGGVPSRFSGSGSGTDFTLTISSLQPEDFATYFCQQGYSFPHTFGQGTKLEIK
(SEQ ID NO: 23);
 
QVQLVQSGAEVKKPGSSVKVSCKASGGTFSSYAISWVRQAPGQGLEWMGGIIPI
FGAANYAQKFQGRVTITADESTSTAYMELSSLRSEDTAVYYCARGGLESSYGM
DVWGQGTTVTVSS (SEQ ID NO: 24),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 25);
 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARGIKGYAFDI
WGQGTMVTVSS (SEQ ID NO: 102),
 and 
QSVLTQPPSVSGAPGQRVTISCSGSSSNIGAGYDVHWYQQLPGRAPKLLIYGNS
NRPSGVPDRFSGSKSGTSASLAITGLQAEDEADYYCQSYDSSLTGVFGGGTKVT
VL (SEQ ID NO: 103);
 
EVQLLESGGGLVQPGGSLRLSCAASGFNFDSYWMTWVRQAPGKGLEWVANIQ
QDGSEKYYLDSVKGRFTISRDNTKNSLYLQMNSLRDEDTAVYYCARDWAYFD
YWGQGTLVTVSS (SEQ ID NO: 104),
 and 
SYELTQPPSVSVSPGQTARITCSGDALPKKYAYWYQQKSGQAPVLVIYGDSKRP
SGIPERFSGSSSGTMATLTVSGAQVEDEADYYCYSTDSSGNQGVFGGGTKLTVL
(SEQ ID NO: 105);
 
QVQLVQSGAEVKKPEASVKVSCKASGYTFTSYYMHWVRQAPGQGLEWMGIIN
PSGGSTSYAQKFQGRVTMTRDTSTSTVYMELSSLRSEDTAVYYCARDKSLGWF
DPWGQGTLVTVSS (SEQ ID NO: 108),
 and 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPFTFGPGTKVDIK (SEQ
ID NO: 109);
 
EVQLLESGGGLVQPGGSLRLSCAASGFTFSSYAMSWVRQAPGKGLEWVSAISG
SGGSTYYADSVKGRFTISRDNSKNTLYLQMNSLRAEDTAVYYCAKELGLRGAF
DIWGQGTMVTVSS (SEQ ID NO: 110),
 and 
QSVLTQPPSASGTPGQRVTFSCSGSRSNIGTNTVNWYQQLPGTAPKLLIYNNLQ
RPSGVPDRFSGSRSGTSASLAISGLQSEDEAEYYCATWDDSLKGVVFGGGTKVT
VL (SEQ ID NO: 111);
 
QVQLVQSGAEVKKPGSSVKVSCKAPGGTFSSYAISWVRQAPGQGLEWMGGIIPI
FGTANYAQKFQGRVTITADKSTSTAYMELSSLRSEDTAVYYCASQTGYSSGWP
SPDAFDIWGQGTMVTVSS (SEQ ID NO: 114),
 and 
DIQMTQSPSSLSASVGDRVTITCRASQGISSYLAWYQQKPGKVPKLLIYAASTLQ
SGVPSRFSGSGSGTDFTLTISSLQPEDFATYYCQQLNSYPPTFGPGTKVDIK (SEQ
ID NO: 115);
 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARAVGIFGAFDI
WGQGTMVTVSS (SEQ ID NO: 122),
 and 
QSVLTQPPSASGTPGQRVTISCSGSNSNIGSNTVNWYQQLPGTAPKLLIYSNNQR
PSGVPDRFSGSKSGTSASLAISGLQSEDEADYYCAAWDDSLNGPYVVFGGGTK
LTVL (SEQ ID NO: 123);
 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARDSYDSSGYY
YDWFDPWGQGTLVTVSS (SEQ ID NO: 130),
 and 
QSALTQPASVSGSPGQSITISCTGTSSDVGGYDFVSWYQQHPGKAPKLMIYEVS
TRHSGVSNRFSASKSGNTASLTISGLQAEDEADYYCSSFTSSPSVVFGGGTKVTV
L (SEQ ID NO: 131);
 
QIQLVQSGAEVKKPGSSVKVSCKASGGIFSNYAISWVRQAPGQGLEWMGGIIPI
FGTANYAQKFQGRVTITADESTSTAYMELSSLRSEDTAVYYCARDYREGYSYG
LYDAFDIWGQGTMVTVSS (SEQ ID NO: 138),
 and 
QSALTQPASVSGSPGQSITISCTGTSSDVGGYNYVSWYQQHPGKAPKLMIYDVS
NRPSGVSNRFSGSKSGNTASLTISGLQAEDEADYYCSSYRSSYTLVFGGGTKLT
VL (SEQ ID NO: 139);
 and 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARGGLESSYGM
DVWGQGTTVTVSS (SEQ ID NO: 146),
 and

QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLSGYVLGTGTKLT
VL (SEQ ID NO: 147).

In another embodiment, the above VH sequences and VL sequences can be interchanged, such as, for example, VH BCA0176 (VR-23) /VL BCA0177 (VR-24); VH BCA0176 (VR-23) /VL BCA0179 (VR-22); VH BCA0177 (VR-24)/VL BCA0176 (VR-23); VH BCA0177 (VR-24)/VL BCA0179 (VR-22); VH BCA0179 (VR-22) /VL BCA0176 (VR-23), or VH BCA0179 (VR-22)/VL BCA0177(VR-24). Similar interchanges can be made for the VH sequences and VL sequences of the clones VR-32 to VR-27, and VR-106 to VR-102, and for the CDRs of the sequences as mentioned above.

Further preferred is the antigen binding protein according to the present invention, wherein said antigen binding protein has an EC₅₀ value for the MAGE-A3/MHC complex presenting cells which is less than 200 nM, less than about 120 nM, such as less than 10 nM. or such as between 1 nM and 200 nM, or between 1 nM and 120 nM.

Particularly preferred is the antigen binding protein according to the present invention that exhibits a particularly low off-target binding, in particular to epitopes that are not derived from antigens that are expressed or associated with a cancerous cell and/or metastasis thereof, i.e. so-called "tumor associated antigens" (TAA). Thus, the antigen binding protein according to the present invention does not significantly bind to other antigenic peptides than the amino acid sequence KVAELVHFL of SEQ ID NO: 1, in particular as described hereinbelow, more particularly when said antigenic peptide(s) is/are in a complex with an MHC protein, preferably in complex with HLA-A*02. In the context of the present invention, relevant "off-target"-exceptions were found for the amino acid sequence KMAELVHFL (SEQ ID NO: 29), which is a TAA derived from MAGE A12, and SAAELVHFL (SEQ ID NO: 44), which is a TAA derived from EPS8L2.

Particularly preferred is the antigen binding protein according to the present invention, wherein the antigen binding protein is selected from the group consisting of an antibody or a binding fragment thereof, a bispecific, trispecific (bi-paratopic), multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, and a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain or nanobody, or wherein the antigen binding protein or a binding fragment thereof is selected from the group consisting of a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).

In a second aspect thereof, the object of the present invention is solved by providing a nucleic acid molecule encoding an antigen binding protein according to the present invention or components thereof, a vector comprising the nucleic acid molecule, such as, for example, an expression vector comprising and expressing the nucleic acid molecule, or a recombinant or genetically modified cell comprising and optionally expressing the nucleic acid molecule or vector. "Components" according to the present invention are individual amino acid sequences that are comprised antigen binding protein according to the present invention, such as individual VH and/or VL chains, fragments comprising the CDRs as described herein, and the like.

In a third aspect thereof, the object of the present invention is solved by providing a recombinant or genetically modified host cell according to the present invention, wherein the cell is a T cell, a hybridoma cell, or an antibody excreting cell.

In a fourth aspect thereof, the object of the present invention is solved by providing a method of producing an antigen binding protein according to the present invention, comprising a) culturing a host cell according to the present invention under conditions effective to express the antigen binding protein; and b) obtaining the expressed antigen binding protein from said host cell or the culture medium of said host cell.

In a fifth aspect thereof, the object of the present invention is solved by providing a method of producing a pharmaceutical composition, comprising formulating the antigen binding protein according to the present invention or the recombinant or genetically modified host cell according to the present invention with a suitable pharmaceutically acceptable carrier. In a sixth aspect thereof, the object of the present invention is solved by providing a pharmaceutical composition, produced according to the method of producing a pharmaceutical composition according to the present invention.

Preferred is the antigen binding protein according to the present invention, the recombinant or genetically modified host cell according to the present invention or the pharmaceutical composition according to the present invention for use as a medicament, preferably for use in the prevention or treatment of a cancer and/or a metastasis thereof.

Further preferred is the antigen binding protein according to the present invention, the recombinant or genetically modified host cell according to the present invention or the pharmaceutical composition according to the present invention for use in the prevention or treatment of a cancer and/or a metastasis thereof according to the present invention, wherein the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer.

In a sixth aspect thereof, the object of the present invention is solved by providing the antigen binding protein according to the present invention, the recombinant or genetically modified host cell according to the present invention or the pharmaceutical composition according to the present invention for use in treating an infectious disease or an autoimmune disease.

In a seventh aspect thereof, the object of the present invention is solved by providing a method for eliciting a CD8-positive T-cell response in a subject, comprising administering to said subject the pharmaceutical composition according to the present invention.

In an eighth aspect thereof, the object of the present invention is solved by providing a method of preventing or treating a cancer cell expressing MAGE-A3 and/or a metastasis thereof, the method comprising administering to a subject suffering or prospectively suffering from said cancer and/or a metastasis thereof an effective amount of the antigen binding protein according to the present invention, the recombinant or genetically modified host cell according to the present invention or the pharmaceutical according to the present invention, wherein preferably the method is combined with a further anti-senescence and/or cancer treatment, wherein preferably the further treatment is selected from the group consisting of chemotherapy, radiotherapy, a treatment with immune-stimulating substances, gene therapy, a treatment with antibodies and a treatment using suitable dendritic cells.

In a nineth aspect thereof, the object of the present invention is solved by providing a kit comprising at least one of the antigen binding proteins according to the present invention, the recombinant or genetically modified host cell according to the present invention or the pharmaceutical composition according to the present invention, and auxiliary agents and/or instructions for use thereof.

As described above, the present invention relates to an antigen binding protein which specifically binds to a MAGE-A, in particular MAGE-A3 antigenic peptide comprising or consisting of the amino acid sequence KVAELVHFL of SEQ ID NO: 1. In vivo and preferably, the antigenic peptide is bound and/or recognized when in a complex with a major histocompatibility complex (MHC) protein, see also below.

The antigen binding protein according to the present invention in a first aspect comprises a polypeptide chain comprising a first variable domain comprising three complementary determining regions (CDRs) CDRH1, CDRH2 and CDRH3, wherein
the CDRH1 comprises or consists of an amino acid sequence having at least 50%, 60%, 70%, 80%, 90%, 95%, 98%, 99%, or preferably 100% sequence identity to the amino acid sequence SYGMH (SEQ ID NO: 2), preferably an amino acid sequence differing from SEQ ID NO: 2 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence VISYDGSNKYYADSVKG (SEQ ID NO: 3), preferably an amino acid sequence differing from SEQ ID NO: 3 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DLVGGKTWDYGMDV (SEQ ID NO: 4), preferably an amino acid sequence differing from SEQ ID NO: 4 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence NYAMT (SEQ ID NO: 8), preferably an amino acid sequence differing from SEQ ID NO: 8 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence AISGSRGSTYYADSVKG (SEQ ID NO: 9), preferably an amino acid sequence differing from SEQ ID NO: 9 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DNWNFPFYLDY (SEQ ID NO: 10), preferably an amino acid sequence differing from SEQ ID NO: 10 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 14), preferably an amino acid sequence differing from SEQ ID NO: 14 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGAANYAQKFQG (SEQ ID NO: 15), preferably an amino acid sequence differing from SEQ ID NO: 15 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence GGLESSYGMDV (SEQ ID NO: 16), preferably an amino acid sequence differing from SEQ ID NO: 16 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 66), preferably an amino acid sequence differing from SEQ ID NO: 66 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 67), preferably an amino acid sequence differing from SEQ ID NO: 67 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence GIKGYAFDI (SEQ ID NO: 68), preferably an amino acid sequence differing from SEQ ID NO: 68 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYWMT (SEQ ID NO: 72), preferably an amino acid sequence differing from SEQ ID NO: 72 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence NIQQDGSEKYYLDSVKG (SEQ ID NO: 73), preferably an amino acid sequence differing from SEQ ID NO: 73 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DWAYFDY (SEQ ID NO: 74), preferably an amino acid sequence differing from SEQ ID NO: 74 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 78), preferably an amino acid sequence differing from SEQ ID NO: 78 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 79), preferably an amino acid sequence differing from SEQ ID NO: 79 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence GGAPAVWSYFDY (SEQ ID NO: 80), preferably an amino acid sequence differing from SEQ ID NO: 80 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYYMH (SEQ ID NO: 84), preferably an amino acid sequence differing from SEQ ID NO: 84 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence IINPSGGSTSYAQKFQG (SEQ ID NO: 85), preferably an amino acid sequence differing from SEQ ID NO: 85 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DKSLGWFDP (SEQ ID NO: 86), preferably an amino acid sequence differing from SEQ ID NO: 86 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAMS (SEQ ID NO: 90), preferably an amino acid sequence differing from SEQ ID NO: 90 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence AISGSGGSTYYADSVKG (SEQ ID NO: 91), preferably an amino acid sequence differing from SEQ ID NO: 91 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence ELGLRGAFDI (SEQ ID NO: 92), preferably an amino acid sequence differing from SEQ ID NO: 92 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence DYTMH (SEQ ID NO: 96), preferably an amino acid sequence differing from SEQ ID NO: 96 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence LISWDGGSTYYADSVKG (SEQ ID NO: 97), preferably an amino acid sequence differing from SEQ ID NO: 97 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DKGWLQLGN (SEQ ID NO: 98), preferably an amino acid sequence differing from SEQ ID NO: 98 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 116), preferably an amino acid sequence differing from SEQ ID NO: 116 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 117), preferably an amino acid sequence differing from SEQ ID NO: 117 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence QTGYSSGWPSPDAFDI (SEQ ID NO: 118), preferably an amino acid sequence differing from SEQ ID NO: 118 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 124), preferably an amino acid sequence differing from SEQ ID NO: 124 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 125), preferably an amino acid sequence differing from SEQ ID NO: 125 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence AVGIFGAFDI (SEQ ID NO: 126), preferably an amino acid sequence differing from SEQ ID NO: 126 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 132), preferably an amino acid sequence differing from SEQ ID NO: 132 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 133), preferably an amino acid sequence differing from SEQ ID NO: 133 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DSYDSSGYYYDWFDP (SEQ ID NO: 134), preferably an amino acid sequence differing from SEQ ID NO: 134 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence NYAIS (SEQ ID NO: 140), preferably an amino acid sequence differing from SEQ ID NO: 140 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 141), preferably an amino acid sequence differing from SEQ ID NO: 141 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence DYREGYSYGLYDAFDI (SEQ ID NO: 142), preferably an amino acid sequence differing from SEQ ID NO: 142 by one or two amino acid substitutions, or
the CDRH1 comprises or consists of an amino acid sequence having at least 50%, 60%, 70%, 80%, 90%, 95%, 98%, 99%, or preferably 100% sequence identity to the amino acid sequence SYAIS (SEQ ID NO: 148), preferably an amino acid sequence differing from SEQ ID NO: 148 by one or two amino acid substitutions,
the CDRH2 comprises or consists of an amino acid sequence having at least 50%, 60%, 70%, 80%, 90%, 95%, 98%, 99%, or preferably 100% sequence identity to the amino acid sequence GIIPIFGTANYAQKFQG (SEQ ID NO: 149), preferably an amino acid sequence differing from SEQ ID NO: 149 by one or two amino acid substitutions,
the CDRH3 comprises or consists of an amino acid sequence having at least 50%, 60%, 70%, 80%, 90%, 95%, 98%, 99%, or preferably 100% sequence identity to the amino acid sequence GGLESSYGMDV (SEQ ID NO: 150), preferably an amino acid sequence differing from SEQ ID NO: 150 by one or two amino acid substitutions. Preferred are conservative exchanges/substitutions.

Preferred is the antigen binding protein according to the present invention, further comprising a second polypeptide chain comprising a second variable domain comprising three CDRs CDRLl, CDRL2 and CDRL3, wherein
the CDRLl comprises or consists of an amino acid sequence having at least 50%, 60%, 70%, 80%, 90%, 95%, 98%, 99%, or preferably 100% sequence identity to the amino acid sequence RASQSINSYLN (SEQ ID NO: 5), preferably an amino acid sequence differing from SEQ ID NO: 5 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence AASSLQS (SEQ ID NO: 6), preferably an amino acid sequence differing from SEQ ID NO: 6 by one or two amino acid substitutions,
and the CDRL3 comprises or consists of an amino acid sequence having at least 50%, 60%, 70%, 80%, 90%, 95%, 98%, 99%, or preferably 100% sequence identity to the amino acid sequence QQSYSTPRT (SEQ ID NO: 7), preferably an amino acid sequence differing from SEQ ID NO: 7 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence RASQGISNWLA (SEQ ID NO: 11), preferably an amino acid sequence differing from SEQ ID NO: 11 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence AASNLQG (SEQ ID NO: 12), preferably an amino acid sequence differing from SEQ ID NO: 12 by one or two amino acid substitutions,
and the CDRL3 comprises or consists of an amino acid sequence having at least 50%, 60%, 70%, 80%, 90%, 95%, 98%, 99%, or preferably 100% sequence identity to the amino acid sequence QQGYSFPHT (SEQ ID NO: 13), preferably an amino acid sequence differing from SEQ ID NO: 13 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence SGSSSNIGNNAVN (SEQ ID NO: 17), preferably an amino acid sequence differing from SEQ ID NO: 17 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence YDDLLPS (SEQ ID NO: 18), preferably an amino acid sequence differing from SEQ ID NO: 18 by one or two amino acid substitutions,
and the CDRL3 comprises or consists of an amino acid sequence having at least 50%, 60%, 70%, 80%, 90%, 95%, 98%, 99%, or preferably 100% sequence identity to the amino acid sequence AAWDDSLNGLV (SEQ ID NO: 19), preferably an amino acid sequence differing from SEQ ID NO: 19 by one or two amino acid substitutions.
the CDRL1 comprises or consists of an amino acid sequence having at least 50%, 60%, 70%, 80%, 90%, 95%, 98%, 99%, or preferably 100% sequence identity to the amino acid sequence SGSSSNIGAGYDVH (SEQ ID NO: 69), preferably an amino acid sequence differing from SEQ ID NO: 69 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence GNSNRPS (SEQ ID NO: 70), preferably an amino acid sequence differing from SEQ ID NO: 70 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence QSYDSSLTGV (SEQ ID NO: 71), preferably an amino acid sequence differing from SEQ ID NO: 71 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence SGDALPKKYAY (SEQ ID NO: 75), preferably an amino acid sequence differing from SEQ ID NO: 75 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence GDSKRPS (SEQ ID NO: 76), preferably an amino acid sequence differing from SEQ ID NO: 76 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence YSTDSSGNQGV (SEQ ID NO: 77), preferably an amino acid sequence differing from SEQ ID NO: 77 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence SGSSSNIGNNAVN (SEQ ID NO: 81), preferably an amino acid sequence differing from SEQ ID NO: 81 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence YDDLLPS (SEQ ID NO: 82), preferably an amino acid sequence differing from SEQ ID NO: 82 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence AAWDDSLNGLV (SEQ ID NO: 83), preferably an amino acid sequence differing from SEQ ID NO: 83 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence RASQSISSYLN (SEQ ID NO: 87), preferably an amino acid sequence differing from SEQ ID NO: 87 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence AASSLQS (SEQ ID NO: 88), preferably an amino acid sequence differing from SEQ ID NO: 88 by one or two amino acid substitutions,
and the CDRL3 comprises or consists of an amino acid sequence having at least 50%, 60%, 70%, 80%, 90%, 95%, 98%, 99%, or preferably 100% sequence identity to the amino acid sequence QQSYSTPFT (SEQ ID NO: 89), preferably an amino acid sequence differing from SEQ ID NO: 89 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence SGSRSNIGTNTVN (SEQ ID NO: 93), preferably an amino acid sequence differing from SEQ ID NO: 93 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence NNLQRPS (SEQ ID NO: 94), preferably an amino acid sequence differing from SEQ ID NO: 94 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence ATWDDSLKGVV (SEQ ID NO: 95), preferably an amino acid sequence differing from SEQ ID NO: 95 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence IGTSSDVGGYNSVS (SEQ ID NO: 99), preferably an amino acid sequence differing from SEQ ID NO: 99 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence DISERPS (SEQ ID NO: 100), preferably an amino acid sequence differing from SEQ ID NO: 100 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence SSYTATSVI (SEQ ID NO: 101), preferably an amino acid sequence differing from SEQ ID NO: 101 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence RASQGISSYLA (SEQ ID NO: 119), preferably an amino acid sequence differing from SEQ ID NO: 119 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence AASTLQS (SEQ ID NO: 120), preferably an amino acid sequence differing from SEQ ID NO: 120 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence QQLNSYPPT (SEQ ID NO: 121), preferably an amino acid sequence differing from SEQ ID NO: 121 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence SGSNSNIGSNTVN (SEQ ID NO: 127), preferably an amino acid sequence differing from SEQ ID NO: 127 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence SNNQRPS (SEQ ID NO: 128), preferably an amino acid sequence differing from SEQ ID NO: 128 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence AAWDDSLNGPYVV (SEQ ID NO: 129), preferably an amino acid sequence differing from SEQ ID NO: 129 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence TGTSSDVGGYDFVS (SEQ ID NO: 135), preferably an amino acid sequence differing from SEQ ID NO: 135 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence EVSTRHS (SEQ ID NO: 136), preferably an amino acid sequence differing from SEQ ID NO: 136 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence SSFTSSPSVV (SEQ ID NO: 137), preferably an amino acid sequence differing from SEQ ID NO: 137 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence TGTSSDVGGYNYVS (SEQ ID NO: 143), preferably an amino acid sequence differing from SEQ ID NO: 143 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence DVSNRPS (SEQ ID NO: 144), preferably an amino acid sequence differing from SEQ ID NO: 144 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence SSYRSSYTLV (SEQ ID NO: 145), preferably an amino acid sequence differing from SEQ ID NO: 145 by one or two amino acid substitutions, or
the CDRLl comprises or consists of an amino acid sequence having at least 50%, 60%, 70%, 80%, 90%, 95%, 98%, 99%, or preferably 100% sequence identity to the amino acid sequence SGSSSNIGNNAVN (SEQ ID NO: 151), preferably an amino acid sequence differing from SEQ ID NO: 151 by one or two amino acid substitutions,
the CDRL2 comprises or consists of an amino acid sequence having at least 50%, 60%, 70%, 80%, 90%, 95%, 98%, 99%, or preferably 100% sequence identity to the amino acid sequence YDDLLPS (SEQ ID NO: 152), preferably an amino acid sequence differing from SEQ ID NO: 152 by one or two amino acid substitutions, and
the CDRL3 comprises or consists of an amino acid sequence having at least 50%, 60%, 70%, 80%, 90%, 95%, 98%, 99%, or preferably 100% sequence identity to the amino acid sequence AAWDDSLSGYV (SEQ ID NO: 153), preferably an amino acid sequence differing from SEQ ID NO: 153 by one or two amino acid substitutions. Preferred are conservative exchanges/substitutions.

A sequence "at least 85% identical to a reference sequence" is a sequence having over its entire length, 85%, or more, in particular 90%, 91%, 92%, 93%, 94%, 95%, 96%, 97%, 98% or 99% sequence identity with the entire length of a reference sequence.

Sequence identity may be determined by a protein sequence alignment using the BLASTP 2.9.0 algorithm (Stephen F et al. (1997) Nucleic Acids Res. 25:3389-3402). In the context of the present application, the "percentage of identity" is calculated using a global pairwise alignment (i.e. the two sequences are compared over their entire length). Methods for comparing the identity of two or more sequences are well known in the art. The "needle" program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the World Wide Web site and is further described in the following publication (EMBOSS: The European Molecular Biology Open Software Suite (2000) Rice, P. Longden, I. and Bleasby, A. Trends in Genetics 16, (6) pp. 276 - 277). The percentage of identity between two polypeptides, in accordance with the invention, is calculated using the EMBOSS: needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95%, 96%, 97%, 98% or 99% identical" to a reference sequence may comprise amino acid mutations such as deletions, insertions and/or substitutions compared to the reference sequence. In case of substitutions, the protein consisting of an amino acid sequence at least 80%, 85%, 90%, 95%, 96%, 97%, 98% or 99% identical to a reference sequence may correspond to a homologous sequence derived from another species than the reference sequence.

"Amino acid substitutions" may be conservative or non-conservative. Preferably, substitutions are conservative substitutions, in which one amino acid is substituted for another amino acid with similar structural and/or chemical properties.

In an embodiment, conservative substitutions may include those, which are described by Dayhoff in "The Atlas of Protein Sequence and Structure. Vol. 5", Natl. Biomedical Research, the contents of which are incorporated by reference in their entirety. For example, in an aspect, amino acids, which belong to one of the following groups, can be exchanged for one another, thus, constituting a conservative exchange or substitution: Group 1: alanine (A), proline (P), glycine (G), asparagine (N), serine (S), threonine (T); Group 2: cysteine (C), serine (S), tyrosine (Y), threonine (T); Group 3: valine (V), isoleucine (I), leucine (L), methionine (M), alanine (A), phenylalanine (F); Group 4: lysine (K), arginine (R), histidine (H); Group 5: phenylalanine (F), tyrosine (Y), tryptophan (W), histidine (H); and Group 6: aspartic acid (D), glutamic acid (E). In an aspect, a conservative amino acid substitution may be selected from the following of T A, G A, A I, T V, A M, T I, A V, T G, and/or T S. In a further embodiment, a conservative amino acid substitution may include the substitution of an amino acid by another amino acid of the same class, for example, (1) nonpolar: Ala, Val, Leu, Ile, Pro, Met, Phe, Trp; (2) uncharged polar: Gly, Ser, Thr, Cys, Tyr, Asn, Gln; (3) acidic: Asp, Glu; and (4) basic: Lys, Arg, His. Other conservative amino acid substitutions may also be made as follows: (1) aromatic: Phe, Tyr, His; (2) proton donor: Asn, Gin, Lys, Arg, His, Trp; and (3) proton acceptor: Glu, Asp, Thr, Ser, Tyr, Asn, Gin (see, for example, U.S. Patent No. 10,106,805, the contents of which are incorporated by reference in their entirety).

The antigen binding protein according to the present invention in a preferred aspect comprises a) a first polypeptide chain comprising an amino acid sequence having at least 75%, preferably at least 90%, and more preferably at least 95% or at least 97%, 98% or 99/ sequence identity to a sequence selected from 
EVQLVESGGVVVQPGGSLRLSCAASGFTFDDYTMHWVRQAPGKGLEWVSLIS
WDGGSTYYADSVKGRFTISRDNSKNSLYLQMNSLRTEDTALYYCAKDKGWLQ
LGNWGQGTLVTVSS (SEQ ID NO: 112),
 and 
QVQLVQSGAEVKKPGSSVKVSCKASGGTFSSYAISWVRQAPGQGLEWMGGIIPI
FGTANYAQKFQGRVTITTDESMSTAYMELSSLRSEDTAVYYCARGGAPAVWS
YFDYWGQGTLVTVSS (SEQ ID NO: 106),
 and 
QVQLVQSGGGVVQPGRSLRLSCAASGFTFSSYGMHWVRQAPGKGLEWVAVIS
YDGSNKYYADSVKGRFTISRDNSKNTLYLQMNSLRAEDTAVYYCAKDLVGGK
TWDYGMDVWGQGTTVTVSS (SEQ ID NO: 20),
 and 
QVQLVQSGGGLVQPGGSLRLSCAASGFTFNNYAMTWVRQAPGKGLEWVSAIS
GSRGSTYYADSVKGRFTISRDNSKNTLYLEMNSLRAEDTAVYFCAKDNWNFPF
YLDYWGQGTLVTVSS (SEQ ID NO: 22),
 and 
QVQLVQSGAEVKKPGSSVKVSCKASGGTFSSYAISWVRQAPGQGLEWMGGIIPI
FGAANYAQKFQGRVTITADESTSTAYMELSSLRSEDTAVYYCARGGLESSYGM
DVWGQGTTVTVSS (SEQ ID NO: 24),
 and 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARGIKGYAFDI
WGQGTMVTVSS (SEQ ID NO: 102),
 and 
EVQLLESGGGLVQPGGSLRLSCAASGFNFDSYWMTWVRQAPGKGLEWVANIQ
QDGSEKYYLDSVKGRFTISRDNTKNSLYLQMNSLRDEDTAVYYCARDWAYFD
YWGQGTLVTVSS (SEQ ID NO: 104),
 and 
QVQLVQSGAEVKKPEASVKVSCKASGYTFTSYYMHWVRQAPGQGLEWMGIIN
PSGGSTSYAQKFQGRVTMTRDTSTSTVYMELSSLRSEDTAVYYCARDKSLGWF
DPWGQGTLVTVSS (SEQ ID NO: 108),
 and 
EVQLLESGGGLVQPGGSLRLSCAASGFTFSSYAMSWVRQAPGKGLEWVSAISG
SGGSTYYADSVKGRFTISRDNSKNTLYLQMNSLRAEDTAVYYCAKELGLRGAF
DIWGQGTMVTVSS (SEQ ID NO: 110),
 and 
QVQLVQSGAEVKKPGSSVKVSCKAPGGTFSSYAISWVRQAPGQGLEWMGGIIPI
FGTANYAQKFQGRVTITADKSTSTAYMELSSLRSEDTAVYYCASQTGYSSGWP
SPDAFDIWGQGTMVTVSS (SEQ ID NO: 114),
 and 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARAVGIFGAFDI
WGQGTMVTVSS (SEQ ID NO: 122),
 and 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARDSYDSSGYY
YDWFDPWGQGTLVTVSS (SEQ ID NO: 130),
 and 
QIQLVQSGAEVKKPGSSVKVSCKASGGIFSNYAISWVRQAPGQGLEWMGGIIPI
FGTANYAQKFQGRVTITADESTSTAYMELSSLRSEDTAVYYCARDYREGYSYG
LYDAFDIWGQGTMVTVSS (SEQ ID NO: 138),
 and 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARGGLESSYGM
DVWGQGTTVTVSS (SEQ ID NO: 146),
 and b) a second polypeptide chain comprising a second variable domain comprising an amino acid sequence having at least 75%, preferably at least 90%, and more preferably at least 95% or at least 97%, 98% or 99/ sequence identity to a sequence selected from 
QSALTQPASVSGSPGQSITISCIGTSSDVGGYNSVSWYQQYPGRAPKLMIYDISE
RPSGVSTRFSGSKSGNTASLTISGLQAEDEADYYCSSYTATSVIFGGGTKVTVL
(SEQ ID NO: 103),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 107),
 and 
DIVMTQPPSSLSASVGDRVTITCRASQSINSYLNWYQQKPGKAPKLLIYAASSLQ
SGVPSRFSGSGSGTDFTLTISSLQPEDFATYYCQQSYSTPRTFGQGTKVEIK (SEQ
ID NO: 21),
 
DIVMTQSPSSVSASVGDRVTITCRASQGISNWLAWYQQKPGKAPKLLIYAASNL
QGGVPSRFSGSGSGTDFTLTISSLQPEDFATYFCQQGYSFPHTFGQGTKLEIK(SE
Q ID NO: 23),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 25),
 and 
QSVLTQPPSVSGAPGQRVTISCSGSSSNIGAGYDVHWYQQLPGRAPKLLIYGNS
NRPSGVPDRFSGSKSGTSASLAITGLQAEDEADYYCQSYDSSLTGVFGGGTKVT
VL (SEQ ID NO: 103),
 and 
SYELTQPPSVSVSPGQTARITCSGDALPKKYAYWYQQKSGQAPVLVIYGDSKRP
SGIPERFSGSSSGTMATLTVSGAQVEDEADYYCYSTDSSGNQGVFGGGTKLTVL
(SEQ ID NO: 105),
 and 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPFTFGPGTKVDIK (SEQ
ID NO: 109),
 and 
QSVLTQPPSASGTPGQRVTFSCSGSRSNIGTNTVNWYQQLPGTAPKLLIYNNLQ
RPSGVPDRFSGSRSGTSASLAISGLQSEDEAEYYCATWDDSLKGVVFGGGTKVT
VL (SEQ ID NO: 111),
 and 
DIQMTQSPSSLSASVGDRVTITCRASQGISSYLAWYQQKPGKVPKLLIYAASTLQ
SGVPSRFSGSGSGTDFTLTISSLQPEDFATYYCQQLNSYPPTFGPGTKVDIK (SEQ
ID NO: 115),
 and 
QSVLTQPPSASGTPGQRVTISCSGSNSNIGSNTVNWYQQLPGTAPKLLIYSNNQR
PSGVPDRFSGSKSGTSASLAISGLQSEDEADYYCAAWDDSLNGPYVVFGGGTK
LTVL (SEQ ID NO: 123),
 and 
QSALTQPASVSGSPGQSITISCTGTSSDVGGYDFVSWYQQHPGKAPKLMIYEVS
TRHSGVSNRFSASKSGNTASLTISGLQAEDEADYYCSSFTSSPSVVFGGGTKVTV
L (SEQ ID NO: 131),
 and 
QSALTQPASVSGSPGQSITISCTGTSSDVGGYNYVSWYQQHPGKAPKLMIYDVS
NRPSGVSNRFSGSKSGNTASLTISGLQAEDEADYYCSSYRSSYTLVFGGGTKLT
VL (SEQ ID NO: 139),
 and

QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLSGYVLGTGTKLT
VL (SEQ ID NO: 147).

The term "antigen binding protein" herein refers to polypeptides or binding proteins that are able to bind to at least one antigen, preferably in the context of MHC, as disclosed herein.

The term "antigen" as used herein refers to a molecule or a portion of a molecule or complex that is capable of being bound by at least one antigen binding site, wherein said one antigen binding site is, for example, present in a conventional antibody, a conventional TCR and/or in the antigen binding proteins of the present invention (see also below).

According to one aspect of the present invention, the antigen binding protein preferably must specifically or substantially bind to a MAGE-A, in particular MAGE-A3, antigenic peptide comprising or consisting of the amino acid sequence KVAELVHFL of SEQ ID NO: 1, wherein said antigenic peptide is in a complex with a major histocompatibility complex (MHC) protein.

As is shown herein, the antigenic peptide epitope as recognized by the preferred embodiments according to the present invention, MAGE-A3 Peptide (112-120, KVAELVHFL (SEQ ID NO: 1)), seems to display a specificity with a general specific reactivity toward the 4-6 C-terminal amino acids of the peptide, and sometimes the N-terminal amino acid, as described in the examples,

Preferred is therefore the antigen binding protein according to the present invention, wherein said antigen binding protein specifically binds to an epitope comprising or consisting of at least three amino acid positions of the MAGE-A antigenic peptide of SEQ ID NO: 1, preferably, at least three amino acid positions selected from the group of amino acid positions consisting of the amino acid positions 1 to 6 and 8 or 9 or 6, 8 and 9, with a selection including position 6 being most preferred being most preferred, of the amino acid sequence of SEQ ID NO: 1. In another aspect, preferred is an antigen binding protein specifically binding to the amino acid sequence KXXEXVXFL (SEQ ID NO: 26), where X can be any amino acid, with the amino acid sequence KVAELVHFL (SEQ ID NO: 1) being preferred.

In the context of the present invention, the term "human MAGE A3" or "MAGE-A3" shall mean the human MAGE-A3 polypeptide, or an immunogenically active peptide fragment comprising a consecutive sequence of at least 8, preferably 9, amino acids of the amino acid sequence KXXEXVXFL (SEQ ID NO: 26), where X can be any amino acid, with the amino acid sequence KVAELVHFL (SEQ ID NO: 1) being preferred. The term shall also include glycosylated and non-glycosylated proteins and fragments, and respective amino acid sequences having at least 75% sequence identity, more preferably at least 80% sequence identity, even more preferably at least 90% sequence identity, and more preferably at least 95% sequence identity or at least 98 or 99% sequence identity to the human or murine MAGE-A3 amino acid sequence.

The amino acid sequence of human MAGE-A3 can be found in the Uniprot database (status 02/25) at P43357

MPLEQRSQHCKPEEGLEARGEALGLVGAQAPATEEQEAASSSSTLVEVTLGEVP
AAESPDPPQSPQGASSLPTTMNYPLWSQSYEDSSNQEEEGPSTFPDLESEFQAAL
SRKVAELVHFLLLKYRAREPVTKAEMLGSVVGNWQYFFPVIFSKASSSLQLVF
GIELMEVDPIGHLYIFATCLGLSYDGLLGDNQIMPKAGLLIIVLAIIAREGDCAPE
EKIWEELSVLEVFEGREDSILGDPKKLLTQHFVQENYLEYRQVPGSDPACYEFL
WGPRALVETSYVKVLHHMVKISGGPHISYPPLHEWVLREGEE (SEQ ID NO: 27)

The amino acid sequence of mouse MAGE-A3 can be found in the Uniprot database (status 02/25) at Q6T340

MADSHNTQYCNLEESAQAQQELDNDQETMETSEEEEDTTTSNKVYGSAIPSPP
QSPQRAYSPCVALASIPDSPSEEASIKGSEGLEDPLHLLHNAQNTKVYDLVDFLV
LNYQMKAFTTKAEMLENIGREYEEYYPLIFSEASECLKMVFGLDMVEVDSSVH
TYMLVTALGITYDGMMTDVQGMPKTGILIAVLSVIFMKGNYVSEEIIWEMLNNI
GLCGGRDPYIHKDPRKLISEEFVQEGYLEYRQVPNSDPPSYGFLWGPRAFAETS
KMKVLQFFASINKTHPRAYPEKYAEALQDEIDRTKTWILNRCSNSSDLHTF
(SEQ ID NO: 28)

In addition, the MAGE-A antigen binding proteins as described herein may be generated using the murine MAGE-A protein or fragments thereof, as described below.

In a preferred embodiment thereof, the antigen binding protein according to the present invention specifically binds to the amino acid sequence KVAELVHFL of the antigenic peptide of SEQ ID NO: 1, in particular when the antigenic peptide is in a complex with a human leukocyte antigen (HLA) protein, preferably HLA-A*02, in particular HLA-A*02:01. In the following, this is also termed "complex" or "antigenic complex". In other words, the antigen binding site of the antigen binding protein according to the present invention specifically binds to a target antigenic (TA) peptide/MHC complex.

The term "functional variant" as used herein refers to an antigen binding protein having substantial or significant sequence identity or similarity to a parent antigen binding protein, such as those antigen binding proteins containing conservative amino acid substitutions as above, wherein said functional variant retains the biological activity of the parental antigen binding protein.

"Affinity" is defined, in theory, by the equilibrium binding between the antigen binding protein and the antigen, in the context of the present invention by the equilibrium binding between the antigen binding protein and its antigen TAA/MHC. Affinity may be expressed for example in half-maximal effective concentration (EC₅₀) (sometimes also referred to as half-maximal binding concentration (EC₅₀)).

"Half maximal effective concentration" also called "EC₅₀" typically refers to the concentration of a molecule which induces a response halfway between the baseline and maximum after a specified exposure time. EC₅₀ and affinity are inversely related, the lower the EC₅₀ value the higher the affinity of the molecule. In one example, the "EC₅₀" refers to the concentration of the antigen binding protein of the invention which induces a response halfway between the baseline and maximum after a specified exposure time, more particularly, refers to the concentration of the antigen binding protein of the invention which induces a response halfway between the baseline and maximum after a specified exposure time. EC₅₀ values can be experimentally assessed by a variety of known methods, using for example an IFN-gamma release assay or an LDH release assay. In the context of the present invention the EC₅₀ value is preferably determined by LDH release assay and thus, refers to induced cytotoxicity.

Further preferred is an antigen binding protein according to the present invention, wherein said antigen binding protein has an EC₅₀ value for the MAGE-A3/MHC complex presenting cells which is less than 200 nM, less than about 120 nM, such as less than 10 nM. or such as between 1 nM and 200 nM, or between 1 nM and 120 nM.

The "TAA/MHC presenting cell" or "TAA presenting cell" herein refers to a cell that presents on its surface the TAA antigenic peptide, such as the particular TAA antigenic peptide comprising or consisting of the amino acid sequence of SEQ ID NO: 1 or a mutated variant thereof as described herein in complex with an MHC protein, wherein the copy number of said TAA peptide/MHC complex that is present on the cell surface can typically be determined with methods known to the skilled in the art. The TAA/MHC presenting cell is typically a cancer cell.

**In** one embodiment, the TAA/MHC presenting cell, has a TAA/MHC copy number, respectively, of more than 50, more than 100, more than 150, more than 200, more than 300, more than 600, more than 800, more than 1000, more than 1500, more than 2000, preferably a TAA/MHC copy number of 50 to 5000.

"Copy number" herein refers to the number of TAA antigenic peptide/MHC complex that are present on the cell surface of a cell, such as a TAA/MHC presenting cell, such as a cancer cell, or a normal healthy cell.

Such copy numbers depend, for example, on the specific TA and on the cell type and can be detected with methods that are known to the skilled in the art, such as FACS analysis, Mass spectrometry (MS) and RNA-Sequencing, preferably Mass spectrometry (MS) and RNA- Sequencing.

"Healthy cells" may also be referred to as "normal cells" and herein refer to cells that are no cancer cells, preferably healthy cells herein refer to cells of the tissue surrounding the TA presenting cells. Healthy cells are preferably selected from the group consisting of astrocytes, GABA neurons, cardiomyocytes, cardiac microvascular endothelial cells, chondrocytes, coronary artery endothelial cells, dermal microvascular endothelial cells, mesenchymal stem cells, nasal epithelial cells, peripheral blood mononuclear cells, pulmonary artery smooth muscle cells, preferably GABA neurons, cardiomyocytes, Cardiac Microvascular endothelial cells, chondrocytes, coronary artery endothelial cells, nasal epithelial cells, peripheral blood mononuclear cells, or pulmonary artery smooth muscle cells.

The antigen binding proteins of the present invention have a high safety profile. "Safety profile" herein refers to the capacity to distinguish tumor cells from normal healthy tissue cells or from similar peptide presenting cells. In the art, the safety profile is described using a safety window. The "safety window" or "therapeutic window" herein refers to a factor that compares the half maximal concentration of a compound that is required for inducing 100% cytotoxicity in a tumor cell line in comparison to the half maximal concentration of a compound that is required for inducing 100% cytotoxicity normal tissue cells. If, for an antigen binding protein of interest, the EC₅₀ determined for a tumor cell line is 1 nM and the EC₅₀ value determined for, for instance, primary cells, is 1000 nM, then the safety window is 1000 since the EC50 for the tumor cell line is 1000 times smaller than the EC₅₀ for the primary cells.

"Similar peptides" in the context of the present invention may also be referred to as "off-targets" and relates to peptides comprising typically 8 to 16 amino acids in length. The similar peptides in the context of the present invention are typically MHC presented. Furthermore, similar peptides in the context of the present invention comprises or consists of an amino acid sequence that is similar to the amino acid sequence of the TA antigenic peptide, in one preferred example in the context of the present invention, peptides that, in comparison to the epitope of the TAA antigenic peptide, comprise an epitope wherein at least one amino acid, such as at least 1, 2, 3, 4 or 5, preferably 1, 2 or 3, more preferably 1, of said epitope in comparison to the epitope of the TAA antigenic peptide is substituted/mutated. Due to this sequence similarity, similar peptides might be bound by a antigen binding protein of the invention, in this scenario, if, for example, the similar peptide is presented by a MHC protein and, thus bound by an antigen binding protein, the ability of a given antigen binding protein, to bind to a similar peptide will not lead to the desired effector cell response but may lead to adverse reactions. Such adverse reactions may be "off-tumor" side effects, such as cross reactivity of a specific antigen binder, such as a TCRm or TCR that cross-reacts with a peptide in normal tissues as reported in Lowdell et al, Cytotherapy, published on December 4, 2018, page 7.

Similar peptides in the context of the present invention may be selected from a database of, for instance, normal tissue-presented HLA-class I bound peptides, such as HLA-A*02 bound peptides in case of MAGE-A, based on, for instance, high sequence similarity (similarity BLAST search) to the TAA antigenic peptide, such as the MAGE A3 peptide herein. Due to these adverse reactions, the antigen binding proteins of the present invention are thus engineered to avoid cytotoxicity towards cells of normal tissues presenting similar peptides.

"Does not significantly bind" in the context of similar peptides and in the context of the antigen binding proteins herein is characterized by lower binding signals and/or higher K_{D} values. For example, in the context of the present invention the antigen binding protein has a binding response for the at least one similar peptides/MHC complex that is less than 50%, less than 45%, less than 40%, less than 30%, less than 20%, less than 20%, less than 10%, less than 5%, less than 4%, less than 3%, less than 3 % of the binding response of the same antigen binding protein to MAGE-A antigenic peptide/MHC complex in the same experimental setting and at the same antigen binding protein concentration and/or, for example, in the context of the present invention the antigen binding protein binds to the at least one similar peptide/MHC complex with an affinity that is decreased in comparison to the affinity for the specific antigen, i.e. the MAGE-A antigenic peptide/MHC complex as described herein and wherein the respective K_{D} for the respective similar peptide is increased by the factor of 5, 7, 10, 15, 20, 30, 40, 50, 100, preferably 20 to 100, more preferably 30 to 100, such as 40 to 100, typically 40 to 50.

It was found in the context of the present invention, that the antigen binding proteins of the invention, in particular the binders VR-23, VR-24, or VR-22, and VR-27 to VR-32, and VR-102 to VR-106 as disclosed do not bind to some and in particular many other antigenic peptides when said antigenic peptides are in a complex with a MHC protein, preferably in complex with HLA-A*02, when tested in comparison with known antigen binders (e.g. VR-33). Furthermore, many of the VR-23, VR-24, or VR-22, and VR-27 to VR-32, and VR-102 to VR-106 as disclosed show improved binding kinetics, when tested in comparison with known antigen binders (e.g. VR-33). See also examples, below.

In one embodiment, the antigen binding proteins of the invention have a comparable or improved stability, optionally, in comparison to a reference protein. In the context of the present invention, a comparable or improved stability refers for example to a comparable or increased physical stability when exposed to thermal stress. The newly developed antigen binding proteins of the invention can thus comparable or better withstand stress conditions, especially thermal stress than the reference antigen binding protein, wherein said antigen binding protein is preferably in the same format.

The term "stability" in the context of the present invention refers to physical stability and can be evaluated qualitatively and/or quantitatively using various analytical techniques that are described in the art and are reviewed in for example Peptide and Protein Drug Delivery, 247- 301, Vincent Lee Ed., Marcel Dekker, Inc., New York, N.Y., Pubs. (1991) and Jones, A. Adv. Drug Delivery Rev. 10: 29-90 (1993). In the context of the present invention, those methods refer in particular to the evaluation of aggregate formation (for example using size exclusion chromatography, by measuring turbidity, and/or by visual inspection. In order to measure stability, a sample which comprises the antigen binding protein of the invention may be tested in a stability study, wherein a sample is exposed for a selected time period to a stress condition followed by quantitative and optionally qualitative analysis of the chemical and physical stability using an adequate analytical technique.

In one embodiment, the antigen binding proteins of the present invention are physical stable, for instance, when exposed to stress condition for a certain period of time, such as when exposed for, for instance, 14 days, to a temperature of 40°C. "Physical stability" refers substantially, in the context of the present invention, to an antigen binding protein having no signs of aggregation, precipitation and/or denaturation. Methods to assess the physical stability are for example size exclusion chromatography (SEC), dynamic light scattering (DLS), light obscuration (LO) and color and clarity.

In the context of the present invention, the antigen binding protein according to the present invention may be of any suitable format, preferred is the antigen binding protein is an antibody or an antigen binding fragment thereof, a bispecific, trispecific (bi-paratopic), multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain or nanobody, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR). Preferred is a TCR mimic (TCRm) antibody or a functional antigen binding fragment thereof, in particular as a bispecific format with the second binder binding to CD3 or CD16.

Herein, the term "antibody" denotes antibodies and fragments thereof, as well as single domain antibodies and fragments thereof, in particular a variable heavy chain of a single domain antibody, and chimeric, humanized, bispecific or multispecific antibodies. A "conventional antibody" as herein referred to is an antibody that has the same domains as an antibody isolated from nature and comprises antibody derived CDRs and Framework regions. In analogy, a "conventional TCR" as herein referred to is a TCR that comprises the same domains as a native TCR and TCR derived CDRs and Framework regions.

The term "humanized antibody" refers to an antibody which is completely or partially of non-human origin, and which has been modified to replace certain amino acids, in particular in the framework regions of the heavy and light chains of the non-human monoclonal antibody, in order to avoid or minimize an immune response in humans. The constant domains of a humanized antibody are mainly human CH and CL domains.

Numerous methods for humanization of an antibody sequence are known in the art; see e.g. the review by Almagro & Fransson (2008) Front Biosci. 13: 1619-1633. One commonly used method is CDR grafting, or antibody reshaping, which involves grafting of the CDR sequences of a donor antibody, generally a mouse antibody, into the framework scaffold of a human antibody of different specificity. Since CDR grafting may reduce the binding specificity and affinity, and, thus, the biological activity of a CDR grafted non-human antibody, back mutations may be introduced at selected positions of the CDR grafted antibody in order to retain the binding specificity and affinity of the parent antibody. Identification of positions for possible back mutations can be performed using information available in the literature and in antibody databases. For chimeric antibodies, humanization typically involves modification of the framework regions of the variable region sequences.

Amino acid residues that are part of a CDR will typically not be altered in connection with humanization, although in certain cases it may be desirable to alter individual CDR amino acid residues, for example to remove a glycosylation site, a deamidation site, an isomerization site, or an undesired cysteine residue. N-linked glycosylation occurs by attachment of an oligosaccharide chain to an asparagine residue in the tripeptide sequence Asn-X-Ser or Asn- X-Thr, where X may be any amino acid except Pro. Removal of an N-glycosylation site may be achieved by mutating either the Asn or the Ser/Thr residue to a different residue, in particular by way of conservative substitution. Deamidation of asparagine and glutamine residues can occur depending on factors such as pH and surface exposure. Asparagine residues are particularly susceptible to deamidation, primarily when present in the sequence Asn-Gly, and to a lesser extent in other dipeptide sequences such as Asn-Ala. When such a deamidation site, in particular Asn-Gly, is present in a CDR sequence, it may therefore be desirable to remove the site, typically by conservative substitution to remove one of the implicated residues. Substitution in a CDR sequence to remove one of the implicated residues is also intended to be encompassed by the present invention. Thus, another type of amino acid modification of the antigen binding protein of the invention may be useful for altering the original glycosylation pattern of the antigen binding protein, i.e. by deleting one or more carbohydrate moieties found in the antigen binding protein, and/or adding one or more glycosylation sites that are not present in the antigen binding protein. The presence of either of the tripeptide sequences asparagine-X-serine, and asparagine-X- threonine, where X is any amino acid except proline, creates a potential glycosylation site. Addition or deletion of glycosylation sites to the antigen binding protein is conveniently accomplished by altering the amino acid sequence such that it contains one or more of the above-described tripeptide sequences (for N-linked glycosylation sites).

Preferred is an antigen binding protein according to the present invention, comprising one or more amino acid substitutions which remove a glycosylation site, wherein preferably the substitution is an N to Q substitution.

"Fragments of antibodies" in the context of the present invention comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of an antibody may also be a single domain antibody, such as a heavy chain antibody or VHH.

The term "Fab" denotes an antibody fragment having a molecular weight of about 50,000 Dalton and antigen binding activity, in which about a half of the N-terminal side of H chain and the entire L chain, among fragments obtained by treating IgG with a protease, e.g. papain, are bound together through a disulfide bond.

The antibodies can also be engineered, as described, for example, in Sa Wang, et al. (Precision engineering of antibodies: A review of modification and design in the Fab region, International Journal of Biological Macromolecules, Volume 275, Part 2, 2024, 133730, ISSN 0141-8130, https://doi.org/10.1016/j.ijbiomac.2024.133730).

In a particular preferred embodiment, the antigen binding protein according to the present invention is at least bispecific. Preferably, the antigen binding protein according to the present invention in addition to the antigen as described herein binds to an antigen selected from the group consisting of CD3, CD16, CD28, OX40, GITR, CD137, CD27, CD40L, HVEM or human glucocorticoid-induced TNFR-related protein (GITR). An overview over bispecific antibodies may be found in Brinkmann U, Kontermann RE (in: the making of bispecific antibodies. MAbs. 2017 Feb/Mar;9(2):182-212. doi: 10.1080/19420862.2016.1268307. PMID: 28071970; PMCID: PMC5297537) or Klein, C., Brinkmann, U., Reichert, J.M. et al. (in: The present and future of bispecific antibodies for cancer therapy. Nat Rev Drug Discov 23, 301-319 (2024). https://doi.org/10.1038/s41573-024-00896-6).

The term "linker" as used herein refers to one or more amino acid residues inserted between two domains to provide sufficient mobility for the domains, for example in single chain constructs, between the first variable domain and the second variable domain of the antigen binding proteins of the invention and, optionally, between the variable domains of light and heavy chain variable domains, to fold correctly to form the antigen binding site.

Preferred is an antigen binding protein according to the present invention, wherein two or more of the amino acid sequences are linked via linkers and/or IgG hinge sequences, wherein preferably the linker or linkers have an amino acid sequence selected from the group consisting of GGGGS (SEQ ID NO: 30), GGGSG (SEQ ID NO: 31), GGSGG (SEQ ID NO: 32), GSGGG (SEQ ID NO: 33), GSGGGP (SEQ ID NO: 34), GGEPS (SEQ ID NO: 35), GGEGGGP (SEQ ID NO: 36), GGEGGGSEGGGS (SEQ ID NO: 37), GGGSGGGG (SEQ ID NO: 38), GGGGSGGGGSGGGGSGGGGSGGGS (SEQ ID NO: 39), GGGGSGGGGSGGGGSGGGGS (SEQ ID NO: 40), EAAAK (SEQ ID NO: 41), and EAAAKEAAAKEAAAK (SEQ ID NO: 42).

By "purified" and "isolated" it is meant, when referring to a polypeptide (i.e. the antigen binding protein of the invention) or a nucleotide sequence, that the indicated molecule is present in the substantial absence of other biological macromolecules of the same type. The term "purified" as used herein in particular means at least 75%, 85%, 95%, or 98% by weight, of biological macromolecules of the same type are present.

An "isolated" nucleic acid molecule that encodes a particular polypeptide refers to a nucleic acid molecule that is substantially free of other nucleic acid molecules that do not encode the subject polypeptide; however, the molecule may include some additional bases or moieties, which do not deleteriously affect the basic characteristics of the composition.

A "domain" may be any region of a protein, generally defined on the basis of sequence homologies and often related to a specific structural or functional entity. A "recombinant" molecule is one that has been prepared, expressed, created, or isolated by recombinant means.

In another aspect of the invention, the antigen binding protein according to the present invention further comprises a half-life extending domain, such as, for example, an Fc fragment or domain. In one embodiment, the Fc-domain is a human IgG Fc domain, preferably derived from human IgG1, IgG2, IgG3 or IgG4, preferably IgG1 or IgG2, more preferably IgG1.

It may be also desirable to modify the antigen binding protein of the present invention with respect to effector function, e.g. so as to enhance or reduce antigen-dependent cell-mediated cytotoxicity (ADCC) and/or complement dependent cytotoxicity (CDC) of the antigen binding protein. This may be achieved by introducing one or more amino acid substitutions in an F_{c} region of the antigen binding protein, herein also called F_{c} -variants in the context with the antigen binding proteins of the present invention. Alternatively or additionally, cysteine residue(s) may be introduced in the F_{c} region, thereby allowing inter-chain disulfide bond formation in this region. The homodimeric antigen binding protein thus generated may have improved or reduced internalization capability and/or increased complement-mediated cell killing and/or antibody-dependent cellular cytotoxicity (ADCC) (Caron PC. et al. 1992; and Shopes B. 1992).

Preferred is therefore an antigen binding protein according to the present invention, wherein the half-life extending domain comprises one or more amino acid substitutions which facilitate dimerization of the amino acid sequences forming the Fc fragment, such as the C_{H}2 domains and/or C_{H}3 domains, in particular S228P (numbered according to the EU numbering scheme, see Kabat EA, Te Wu T, Foeller C, Perry HM, Gottesman KS. Sequences of Proteins of Immunological Interest. (1991). U.S. Department of Health and Human Services, Public Health Service, National Institutes of Health).

The "Knob-into-Hole" or also called "Knob-in-Hole" technology usually refers to amino acid substitutions T366S, L368A and Y407V (Hole) and T366W (Knob) both in the CH3-CH3 interface to promote heteromultimer formation. Those knob-into-hole mutations can be further stabilized by the introduction of additional cysteine amino acid substitutions Y349C and S354C. The "Knob-into-Hole" technology together with the stabilizing cysteine amino acid substitutions has been described, for example, in WO 1996/027011A1.

Preferably, one of the domains comprises one or more amino acid substitutions selected from the group consisting of S228P, F234A, L234A, L235A, R409K, M252Y, S254T, T256E, Y349C, T366S, L368A, and Y407V (part of these mutations can form a "hole", numbered according to the EU numbering scheme); and S228P, F234A, L234A, L235A, R409K, M252Y, S254T, T256E, S354C, and T366W (part of these mutations can form a "knob", numbered according to the EU numbering scheme).

Elimination of the binding of immunoglobulin Fc to Fc gamma receptors is highly desirable for the avoidance of unwanted inflammatory responses to therapeutic antibodies and fusion proteins (see, for example, Hale G, et al. Systematic analysis of Fc mutations designed to reduce binding to Fc-gamma receptors. MAbs. 2024 Jan-Dec;16(1):2402701. doi: 10.1080/19420862.2024.2402701. Epub 2024 Sep 15).

Preferred is therefore an antigen binding protein according to the present invention, wherein the half-life extending domain comprises one or more amino acid substitutions which prevent or reduce binding to FcγR, such as, for example, an N297G amino acid substitution (numbered according to the EU numbering scheme).

Preferred is therefore an antigen binding protein according to the present invention, wherein the half-life extending domain comprises one or more amino acid substitutions which promote binding to FcRn, for example an M252Y, S254T and/or T256E amino acid substitution (numbered according to the EU numbering scheme). (see, for example, Ward ES, Ober RJ. Targeting FcRn to Generate Antibody-Based Therapeutics. Trends Pharmacol Sci. 2018 Oct;39(10):892-904. doi: 10.1016/j.tips.2018.07.007. Epub 2018 Aug 22. PMID: 30143244; PMCID: PMC6169532).

In another aspect of the invention, the antigen binding protein according to the present invention further comprises an effector molecule, wherein the effector molecule is a therapeutic agent selected from the group consisting of a drug, a toxin, a radioisotope, a protein, a peptide, and a nucleic acid or wherein the effector molecule is a label.

In some embodiments, the antigen binding proteins of the present invention are covalently attached, directly or via a cleavable or non-cleavable linker, to at least one growth inhibitory agent. An antigen binding protein to which such the at least one growth inhibitory agent is attached may also be referred to as a conjugate. The preparation of such conjugates, for example immunoconjugates, is described in the application WO2004/091668 or Hudecz, F., Methods Mol. Biol. 298: 209-223 (2005) and Kirin et al., Inorg Chem. 44(15): 5405-5415 (2005), and may by the skilled in the art be transferred to the preparation of antigen binding proteins of the present invention to which such a at least one growth inhibitory agent is attached.

"Linker" in the context of the attachment of at least one growth inhibitory agent, means a chemical moiety comprising a covalent bond or a chain of atoms that covalently attaches a polypeptide to a drug moiety. The conjugates may be prepared by *in vitro* methods. In order to link a drug or prodrug to the antibody, a linking group is used. Suitable linking groups are well known in the art and include disulfide groups, thioether groups, acid labile groups, photolabile groups, peptidase labile groups and esterase labile groups. Conjugation of an antigen binding protein of the invention with cytotoxic agents or growth inhibitory agents may be made using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1- pyrrolidinyl ester (nitro-SPDB), 4-(Pyridin-2-yldisulfanyl)-2-sulfo-butyric acid (sulfo-SPDB), N- succinimidyl (2-pyridyldithio) propionate (SPDP), succinimidyl (N-maleimidomethyl) cyclohexane-1 -carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1 ,5-difluoro-2,4- dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" and thus facilitating release of the cytotoxic agent or growth inhibitory agent in the cell. For example, an acid-labile linker, a peptidase-sensitive linker, an esterase labile linker, a photolabile linker or a disulfide-containing linker (See e.g. U.S. Patent No. 5,208,020) may be used. The linker may be also a "non-cleavable linker" (for example SMCC linker) that might lead to better tolerance in some cases.

Alternatively, a fusion protein comprising the antigen binding protein of the invention and a cytotoxic or growth inhibitory polypeptide may be made by recombinant techniques or peptide synthesis. The length of DNA may comprise respective regions encoding the two portions of the conjugate either adjacent one another or separated by a region encoding a linker peptide which does not destroy the desired properties of the conjugate.

A further object of the invention relates to an isolated nucleic acid sequence comprising or consisting of a sequence encoding for an antigen binding protein according to the present invention or functional fragments or components thereof such as light and/or heavy chains, or CDR sequences of the binding protein. Typically, the nucleic acid is a DNA or RNA molecule or mixtures thereof, which may be included in any suitable vector, such as a plasmid, cosmid, episome, artificial chromosome, phage or a viral vector.

The invention further relates to a vector comprising the nucleic acid molecule of the invention, such as, for example, an expression vector comprising and expressing the nucleic acid molecule. The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

Thus, a further object of the invention relates to a vector comprising a nucleic acid of the invention. Such vectors may comprise regulatory elements, such as a promoter, enhancer, terminator and the like, to cause or direct expression of said polypeptide upon administration to a subject. Examples of promoters and enhancers used in the expression vector for animal cell include early promoter and enhancer of SV40 (Mizukami T. etal. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Any suitable expression vector for animal cells can be used, as long as a gene encoding the human antibody C region can be inserted and expressed. Examples of suitable vectors include PAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like. Other examples of plasmids include replicating plasmids comprising an origin of replication, or integrative plasmids, such as for instance pUC, pcDNA, pBR, and the like.

Other examples of viral vectors include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, and WO 94/19478.

The term "viral vector" herein refers to a nucleic acid vector construct that includes at least one element of viral origin and has the capacity to be packaged into a viral vector particle, and encodes at least an exogenous nucleic acid. The vector and/or particle can be utilized for the purpose of transferring any nucleic acids into cells either in vitro or in vivo. Numerous forms of viral vectors are known in the art. The term "virion" is used to refer to a single infective viral particle. "Viral vector", "viral vector particle" and "viral particle" also refer to a complete virus particle with its DNA or RNA core and protein coat as it exists outside the cell. For example, a viral vector may be selected from adenoviruses, poxviruses, alphaviruses, arenaviruses, flaviruses, rhabdoviruses, retroviruses, lentiviruses, herpesviruses, paramyxoviruses, or picornaviruses.

The term viruses may refer to natural occurring viruses as well as artificial viruses. Viruses in accordance with some embodiments of the present invention may be either an enveloped or non-enveloped virus. Parvoviruses (such as AAVs) are examples of non-enveloped viruses. In a preferred embodiment, the viruses may be enveloped viruses. In preferred embodiments, the viruses may be retroviruses and in particular lentiviruses. Viral envelope proteins that can promote viral infection of eukaryotic cells may include HIV-1 derived lentiviral vectors (LVs) pseudotyped with envelope glycoproteins (GPs) from the vesicular stomatitis virus (VSV-G), the modified feline endogenous retrovirus (RD114TR), and the modified gibbon ape leukemia virus (GALVTR). These envelope proteins can efficiently promote entry of other viruses, such as parvoviruses, including adeno-associated viruses (AAV), thereby demonstrating their broad efficiency. For example, other viral envelop proteins may be used including Moloney murine leukemia virus (MLV) 4070 env (such as described in Merten et al., J. Virol. 79:834-840, 2005; the content of which is incorporated herein by reference), RD114 env, chimeric envelope protein RD114pro or RDpro (which is an RD114-HIV chimera that was constructed by replacing the R peptide cleavage sequence of RD114 with the HIV-1 matrix/capsid (MA/CA) cleavage sequence, such as described in Bell et al. Experimental Biology and Medicine 2010; 235: 1269-1276; the content of which is incorporated herein by reference), baculovirus GP64 env (such as described in Wang et al. J. Virol. 81:10869-10878, 2007; the content of which is incorporated herein by reference), or GALV env (such as described in Merten et al., J. Virol. 79:834-840, 2005; the content of which is incorporated herein by reference), or derivatives thereof.

Another object of the present invention relates to a recombinant or genetically modified cell, such as a host cell, comprising and optionally expressing the nucleic acid molecule or vector according to the invention. The host cell has been transformed, transduced or transfected with a nucleic acid and/or a vector according to the invention. Preferred is a recombinant or genetically modified host cell according to the present invention, wherein the cell is a T cell, a hybridoma cell, or an antibody or antigen binding protein excreting cell.

The term "transformation" refers to a naturally occurring process of gene transfer into a host cell which involves absorption of the genetic material, such as nucleic acids, for instance, DNA or RNA, by a cell through cell membrane, so that the host cell will express the introduced gene or sequence to produce a desired substance, typically a protein or enzyme coded by the introduced gene or sequence. There are two types of transformation, designated as natural transformation or artificial or induced transformation. The artificial or induced method of transformation is done under laboratory conditions. A host cell that receives and expresses foreign nucleic acids, such as DNA or RNA, by the process of a transformation has been "transformed".

The term "transfection" refers to a mode of gene transfer involving creation of pores on the cell membrane of the host cell enabling the host cell to receive the foreign genetic material. Typically, transfection refers to a transformation of eukaryotic cells, such as insect or mammalian cells. Chemical mediated transfection involves use of, for instance, calcium phosphate or cationic polymers or liposomes. Non-chemical mediated transfection methods are typically electroporation, sonoporation, impalefection, optical transfection or hydro dynamic delivery. Particle based transfection uses gene gun technique where a nanoparticle is used to transfer the nucleic acid to host cell or by another method called magnetofection. Nucleofection and use of heat shock are the other evolved methods for successful transfection. A host cell that receives foreign nucleic acids via a transfection method has been "transfected".

The term "transduction" generally relates to the transfer of foreign nucleic acids, such as DNA or RNA, into a cell by a virus or viral vector. A host cell that receives and expresses foreign nucleic acids, such as DNA or RNA, by a virus or viral vector has been "transduced".

The nucleic acids of the invention may be used to produce a recombinant antigen binding protein of the invention in a suitable expression system.

Another object of the present invention therefore relates to a method for producing an antigen binding protein according to the present invention or functional fragments or components thereof such as light and/or heavy chains, or CDR sequences of the binding protein. The method comprises a) culturing a host cell according to the present invention under conditions effective to express the antigen binding protein according to the present invention; and b) obtaining the expressed antigen binding protein according to the present invention from said host cell or the culture medium of said host cell.

The term "expression system" means a host cell and compatible vector under suitable conditions, e.g. for the expression of a protein coded for by foreign DNA carried by the vector and introduced to the host cell.

Common expression systems include *E. coli* host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include *E. coli* or *Saccharomyces* yeasts, mammalian cell lines (e.g., Vero cells, CHO cells, 3T3 cells, COS cells, HEK cells, etc.) as well as primary or established mammalian cell cultures (e.g., produced from lymphoblasts, fibroblasts, embryonic cells, epithelial cells, nervous cells, adipocytes, etc.). Examples include CHO cells in which a dihydrofolate reductase gene (hereinafter referred to as "DHFR gene") is defective (Urlaub G et al; 1980), or rat YE32/3HL.P2.G11.16Ag.20 cell (ATCC CRL1662, hereinafter referred to as "YB2/0 cell"), and the like. In some embodiments, the YB2/0 cell may be preferred, since ADCC activity of chimeric or humanized antibodies is enhanced when expressed in this cell.

The invention also pertains to a host cell comprising an antigen binding protein in accordance with the invention. Specifically, the host cell of the invention comprises a nucleic acid, or a vector as described herein above. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing an antigen binding protein, such as an antibody, TCRm, TCR, polypeptide, or protein, the host cell is preferably a mammalian cell.

According to the above, in one embodiment, the invention refers to a host cell comprising the antigen binding protein of the invention which is defined herein above, or the nucleic acid, or the vector of the invention, wherein said host cell preferably is a) mesenchymal stem cell or b) a cell for recombinant expression, such as a Chinese Hamster Ovary (CHO) cell. In particular, for expression of some of the antigen binding proteins of the invention the expression vector may be either of a type in which a gene encoding the first polypeptide, such as an antibody heavy chain or an alpha chain, and a gene encoding a second polypeptide, such as an antibody light chain or a beta chain, exists on separate vectors or of a type in which both genes exist on the same vector (tandem type). In respect of easiness of construction of antigen binding protein expression vector, easiness of introduction into animal cells, and balance between the expression levels of antibody H and L chains in animal cells, humanized antibody expression vector of the tandem type is preferred (Shitara K et al. J Immunol Methods. 1994 Jan. 3; 167(1 -2):271-8). Examples of tandem type humanized antibody expression vector include pKANTEX93 (WO 97/10354), pEE18 and the like.

Standard techniques for production of polypeptides, such as antibodies or fragments thereof and TCRs or fragments thereof, are known in the art and these techniques can be used by the person skilled in the art to produce the antigen binding proteins of the present invention. For instance, they can be synthesized using well-known solid phase method, in particular using a commercially available peptide synthesis apparatus (such as that made by Applied Biosystems, Foster City, California) and following the manufacturer's instructions. Alternatively, antigen binding proteins of the invention, such as antibodies or fragments thereof and TCRs or fragments thereof, can be synthesized by recombinant DNA techniques as is well-known in the art. For example, fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques. In one embodiment such recombinant host cells can be used for the production of at least one antigen binding protein of the invention. See also above.

In one example, i.e. in case of, for example, bispecific molecules, DNA-sequences coding for various combinations of VH and VL and variable alpha (Valpha) and variable beta (Vbeta), as well as sequences coding for linkers may be obtained by, for instance, gene synthesis. Resulting DNA-sequences may be cloned in frame into expression vectors coding for hinge region, CH2 and CH3 domain and may be further engineered. Engineering may be performed to incorporate knob-into-hole mutations into CH3-domains with and without additional interchain disulfide bond stabilization; to remove an N-glycosylation site in CH3 (e.g. N297Q mutation); to introduce F_{c}-silencing mutations or to introduce additional disulfide bond stabilization into VL and VH, respectively, according to the methods described by Reiter et al. (Stabilization of the Fv Fragments in Recombinant Immunotoxins by Disulfide Bonds Engineered into Conserved Framework Regions. Biochemistry, 1994, 33, 5451 - 5459). Antigen binding proteins of the invention are suitably separated from the culture medium by immunoglobulin purification procedures such as, for example, protein A- Sepharose, hydroxyl apatite chromatography, gel electrophoresis, dialysis, or affinity chromatography.

The invention further refers to a pharmaceutical composition comprising the antigen binding protein of the invention, the nucleic acids of the invention, the vector of the invention, or the host cell of the invention and a pharmaceutically acceptable carrier.

The invention also relates to an antigen binding protein according to the invention, for use as a medicament. The invention also relates to a pharmaceutical composition of the invention for use as a medicament. The invention also relates to the use of an antigen binding protein according to the invention and/or to a pharmaceutical composition of the invention, in the manufacture of a medicament.

The terms "pharmaceutical composition" or "therapeutic composition" as used herein refer to a compound or composition capable of inducing a desired therapeutic effect when properly administered to a subject. In some embodiments, the subject may also be referred to as patient.

Such therapeutic or pharmaceutical compositions may comprise a therapeutically effective amount of an antigen binding protein of the invention or an antigen binding protein further comprising a therapeutic agent, in admixture with a pharmaceutically or physiologically acceptable formulation agent selected for suitability with the mode of administration.

The antigen binding protein of the present invention will usually be supplied as part of a sterile, pharmaceutical composition which will normally include a pharmaceutically acceptable carrier.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

A "pharmaceutically acceptable carrier" may also be referred to as "pharmaceutically acceptable diluent" or "pharmaceutically acceptable vehicles" and may include solvents, bulking agents, stabilizing agents, dispersion media, coatings, antibacterial and antifungal agents, isotonic and absorption delaying agents, and the like which are physiologically compatible. Accordingly, in one embodiment the carrier is an aqueous carrier.

In another aspect, the aqueous carrier is capable of imparting improved properties when combined with an antigen binding protein described herein, for example, improved solubility, efficacy, and/or improved immunotherapy.

In another aspect, the present invention relates to a method for producing a pharmaceutical composition comprising formulating the antigen binding protein according to the present invention or the recombinant or genetically modified host cell according to the present invention with a suitable pharmaceutically acceptable carrier.

An antigen-binding protein of the invention can be formulated into a composition in a neutral or salt form using pharmaceutically acceptable salts.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

In another aspect, the present invention relates to a pharmaceutical composition, produced according to the method according to the present invention.

Such therapeutic or pharmaceutical compositions may comprise a therapeutically effective amount of the antigen binding protein of the invention and/or further comprise a therapeutic agent, in admixture with a pharmaceutically or physiologically acceptable formulation agent selected for suitability with the mode of administration.

In another aspect, the aqueous carrier is capable of imparting improved properties when combined with an antigen binding protein described herein, for example, improved solubility, efficacy, and/or improved immunotherapy.

The form of the pharmaceutical compositions, the route of administration, the dosage and the regimen naturally depend upon the condition to be treated, the severity of the illness, the age, weight, and gender of the patient, the desired duration of the treatment etc. This pharmaceutical composition may be in any suitable form, (depending upon the desired method of administering it to a patient). It may be provided in unit dosage form, will generally be provided in a sealed container and may be provided as part of a kit. Such a kit would normally (although not necessarily) include instructions for use. It may include a plurality of said unit dosage forms.

Empirical considerations, such as the biological half-life, generally will contribute to the determination of the dosage. Frequency of administration may be determined and adjusted over the course of therapy and is based on reducing the number of cancer cells, maintaining the reduction of cancer cells, reducing the proliferation of cancer cells, or killing the cancer cells. Alternatively, sustained continuous release formulations of the antigen binding protein may be appropriate. Various formulations and devices for achieving sustained release are known in the art.

In one embodiment, dosages for the antigen binding proteins may be determined empirically in individuals who have been given one or more administration(s). Individuals are given incremental dosages of the antigen binding protein. To assess efficacy of the antigen binding protein, a marker of the cancer cell state can be followed. These include direct measurements of cancer cell proliferation and cell death by FACS, other imaging techniques; an improvement in health as assessed by such measurements, or an increase in quality of life as measured by accepted tests or prolongation of survival.

It will be apparent to one of skill in the art that the dosage will vary depending on the individual, the stage of the disease, and the past and concurrent treatments being used.

In particular, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. To prepare pharmaceutical compositions, an effective amount of the antigen binding protein of the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Under ordinary conditions of storage and use, preparations contain a preservative to prevent the growth of microorganisms.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

The antigen binding proteins of the present invention can bind to diseased cells and reduce the growth of and/or kill the diseased cells presenting the TAA peptide/MHC complex, in particular the complex with the MAGE-A3 peptide of SEQ ID NO. 1 on their cell surface. **In** one preferred embodiment, the antigen binding proteins of the present invention can bind to tumor cells and reduce the growth of and/or kill the tumor cells presenting the TAA peptide/MHC complex on their cell surface.

It is understood that the antigen binding protein is administered at a concentration that promotes binding under physiological (e.g., *in vivo*) conditions. Accordingly, in one embodiment, the antigen binding proteins of the invention can be used for immunotherapy directed against tumor cells of different tissues, as described below. In another embodiment, the antigen binding proteins of the invention can bind to and reduce the growth of and/or kill tumor cells.

In another aspect, the present invention relates to the antigen binding protein according to the present invention, the recombinant or genetically modified host cell according to the present invention or the pharmaceutical composition according to the present invention for use as a medicament, preferably for use in the prevention or treatment of a cancer and/or a metastasis thereof.

Preferred is the antigen binding protein according to the present invention, the recombinant or genetically modified host cell according to the present invention or the pharmaceutical composition according to the present invention for use in the prevention or treatment of a cancer and/or a metastasis thereof according to the present invention, wherein the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer.

In yet another aspect, the present invention relates to a method of preventing or treating a cancer cell expressing MAGE-A3 and/or a metastasis thereof, the method comprising administering to a subject suffering or prospectively suffering from said cancer and/or a metastasis thereof an effective amount of the antigen binding protein according to the present invention, the recombinant or genetically modified host cell according to the present invention or the pharmaceutical composition according to the present invention, wherein preferably the method is combined with a further anti-senescence and/or cancer treatment, wherein preferably the further treatment is selected from the group consisting of chemotherapy, radiotherapy, a treatment with immune-stimulating substances, gene therapy, a treatment with antibodies and a treatment using suitable dendritic cells.

Therefore, the invention relates to a method of treating or preventing a proliferative disease or disorder comprising administering to a subject in need thereof a therapeutically effective amount of the antigen binding protein, the nucleic acid or vector, the host cell or the pharmaceutical composition according to the invention as defined herein above. In a particular embodiment, the invention relates to a method of treating a subject who has a disease comprising administering to said subject the antigen binding protein of the invention.

Preferred is the method of preventing or treating a cancer cell and/or a metastasis thereof according to the present invention, wherein the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer.

The term "subject" or "individual" are used interchangeably and may be, for example, a human or a non-human mammal, preferably, a human.

In the context of the invention, the term "treating" or "treatment", refers to a therapeutic use (i.e. on a subject having a given disease) and means reversing, alleviating, inhibiting the progress of one or more symptoms of such disorder or condition. Therefore, treatment does not only refer to a treatment that leads to a complete cure of the disease, but also to treatments that slow down the progression of the disease and/or prolong the survival of the subject.

By "preventing" is meant a prophylactic use (i.e. on a subject susceptible of developing a given disease).

"Proliferative diseases", such as cancer, involve the unregulated and/or inappropriate proliferation of cells. In one embodiment, the proliferative disorder or disease is a tumor disease characterized by the expression of MAGE-A3 and/or MAGE-A12, in a cancer or tumor cell of said tumor disease, and the presentation of the MAGE-A3 peptide as described herein.

The antigen binding proteins of the present invention can be used by itself or can be added to a therapy regimen using other anti-cancer agents typically used in treating a cancer patient. Accordingly, in some embodiments, the antigen binding protein of the invention can be administered concurrently with, before, or after a variety of drugs and treatments widely employed in cancer treatment such as, for example, chemotherapeutic agents, non-chemotherapeutic, anti-neoplastic agents, and/or radiation, preferably chemotherapeutic agents.

By a "therapeutically effective amount" of the antigen binding protein or pharmaceutical composition thereof is meant a sufficient amount of the antigen binding protein to treat said proliferative disease, at a reasonable benefit/risk ratio applicable to any medical treatment. It will be understood, however, that the total daily or monthly usage of the antigen binding proteins, the nucleic acid or vector, the host cell or the pharmaceutical composition of the present invention will be decided by the attending physician within the scope of sound medical judgment. The specific therapeutically effective dose level for any particular patient will depend upon a variety of factors including the disorder or disease being treated and the severity of the disorder; activity of the specific antigen binding protein employed; the specific composition employed, the age, body weight, general health, sex and diet of the patient; the time of administration, route of administration, and rate of excretion of the specific polypeptide employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved.

In one embodiment, efficacy of the treatment with the antigen binding protein of the invention is assayed in vivo, for instance in a mouse model of cancer and by measuring, for example, changes in tumor volume between treated and control groups.

Pharmaceutical compositions, vectors, nucleic acids and cells of the invention may be provided in substantially pure form, for example, wherein at least 80%, at least 85%, at least 90%, at least 91%, at least 92%, at least 93%, at least 94%, at least 95%, at least 96%, at least 97%, at least 98%, at least 99% or 100% by weight of the antigen binding protein of the same type are present.

The antigen binding protein of the invention, the nucleic acid of the invention or the vector of the invention, the host cell of the invention or the pharmaceutical composition of the invention can be administered by any feasible method.

Another aspect of the present inventio relates to the antigen binding protein according to the present invention, the recombinant or genetically modified host cell according to the present invention or the pharmaceutical composition according to the present invention for use in treating an infectious disease or an autoimmune disease.

In one embodiment, a "disease" or "disorder" is any condition that would benefit from treatment with the antigen binding protein of the invention. In one embodiment, this includes chronic and acute disorders or diseases including those pathological conditions which predisposes the subject to the disorder in question. In particular, the disease referred to in the context of the present invention may be a proliferative disease as above or a disease caused by a virus or a bacteria.

A disease caused by a virus or bacteria may also be referred to as a viral or bacterial infection. In the context of the present invention, the virus causing the disease may be selected from the group constituted of for example, human immunodeficiency viruses (HIV), Humane Cytomegalovirus (HCMV), cytomegalovirus (CMV), human papillomavirus (HPV), Hepatitis B virus (HBV), Hepatitis C virus (HCV), Epstein-Barr virus (EBV), Influenza virus, preferably human immunodeficiency viruses (HIV).

It will be understood by the skilled in the art, that when the antigen binding protein targets a viral antigenic peptide, for instance, of HIV, the antigen binding protein is for use in the treatment of HIV. Accordingly, an antigen binding protein targeting the viral or bacterial antigenic peptide (TAA) is thus for use in the treatment of virus or bacteria from which said antigenic viral or bacterial antigenic peptide, was derived.

In a further embodiment, the invention refers to a method for eliciting a CD8-positive T-cell response in a subject, comprising administering to said subject the pharmaceutical composition according to the present invention.

In an aspect, binding of the antigen binding protein to a target cell elicits an immune response, such a response may refer to the proliferation and activation of effector functions, in vitro or in vivo. For MHC class I restricted cytotoxic T cells, for example, effector functions may be lysis of peptide-pulsed, peptide-precursor pulsed or naturally peptide-presenting target cells, secretion of cytokines, preferably Interferon-gamma, TNF-alpha, or IL-2 induced by peptide, secretion of effector molecules, for example, granzymes or perforins induced by peptide, or degranulation. Accordingly, the antigen binding proteins of the present invention may be used to treat a wide variety of conditions including, for example, various forms of cancer. The bispecific binding proteins of the present invention may be used for therapeutic purposes in humans and/or non human mammalian animals, in particular in humans.

Another aspect of the present invention relates to a kit comprising at least one of the antigen binding protein according to the present invention, the recombinant or genetically modified host cell according to the present invention or the pharmaceutical composition according to the present invention, and auxiliary agents and/or instructions for use thereof.

In one embodiment, the kit comprises a) at least one antigen binding protein of the invention as defined herein, b) optionally packaging material, and c) optionally a label or packaging insert contained within said packaging material indicting that said antigen binding protein is effective for treating a disease, preferably cancer or for use for the treatment of a disease, preferably cancer.

In a related embodiment, the at least one antigen binding protein of the invention is contained in a single and/or multi-chambered pre-filled syringe/s (e.g., liquid syringes and lyosyringes). In one embodiment, the invention encompasses kits for producing a single-dose administration unit. Accordingly, in one embodiment, the at least one antigen binding protein of the invention as mentioned in a) of the kit of the invention is a dried antigen binding protein of the invention contained in a first container. The kit then further contains a second container having an aqueous formulation.

Accordingly, in another embodiment, the kit comprises a) a first container comprising at least one dried, such as freeze-dried, antigen binding protein of the invention as defined herein, b) a second container comprising an aqueous formulation; c) optionally packaging material, and d) optionally a label or packaging insert contained within said packaging material indicting that said antigen binding protein is effective for treating a disease, preferably cancer, or for use for the treatment of a disease, preferably cancer.

The aqueous formulation is typically an aqueous solution comprising pharmaceutically-acceptable carriers as described herein above. In a related embodiment, the "first container" and the "second" container refer to the chambers of a multi-chambered pre-filled syringes (e.g., lyosyringes).

Another aspect of the present invention relates to a kit comprising; a) the pharmaceutical composition for use according to the present invention or the pharmaceutical composition according to the present invention, and b) a composition comprising at least one second active ingredient. Preferred is the kit according to the present invention, wherein the second active ingredient is an anti-cancer agent, such as an anti-cancer agent selected from a chemotherapeutic agent, for example selected from: Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine, and Vinorelbine.

Preferred is the kit according to the present invention, where the provided compositions are to be administered simultaneously or sequentially.

The cancer is as defined herein above in the context of the invention.

Finally, another aspect of the present invention relates to the use of a kit according to the present invention for producing an antigen binding protein according to the present invention, for producing a pharmaceutical composition according to the present invention, for eliciting a CD8-positive T-cell response in a subject according to the present invention, or for method of preventing or treating a cancer cell and/or a metastasis thereof according to the present invention.

"About" herein refers to a deviation of +/- 10% from the given value, unless indicated otherwise.

"At least one" herein refers to one or more of the specified objects such as 1, 2, 3, 4, 5 or 6 or more of the specified objects. For example, at least one binding site herein refers to 1, 2, 3, 4, 5 or 6 or more binding sites.

The present invention relates to the following items:
Item 1. An antigen binding protein which specifically binds to a MAGE-A antigenic peptide comprising or consisting of the amino acid sequence KVAELVHFL of SEQ ID NO: 1,
   wherein said antigenic peptide is in a complex with a major histocompatibility complex (MHC) protein, the antigen binding protein comprising a polypeptide chain comprising a first variable domain comprising three complementary determining regions (CDRs) CDRH1, CDRH2 and CDRH3, wherein
   the CDRH1 comprises or consists of the amino acid sequence SYGMH (SEQ ID NO: 2), or an amino acid sequence differing from SEQ ID NO: 2 by one or two amino acid substitutions,
   the CDRH2 comprises or consists of the amino acid sequence VISYDGSNKYYADSVKG (SEQ ID NO: 3), or an amino acid sequence differing from SEQ ID NO: 3 by one or two amino acid substitutions,
   the CDRH3 comprises or consists of the amino acid sequence DLVGGKTWDYGMDV (SEQ ID NO: 4), or an amino acid sequence differing from SEQ ID NO: 4 by one or two amino acid substitutions, or
   the CDRH1 comprises or consists of the amino acid sequence NYAMT (SEQ ID NO: 8), or an amino acid sequence differing from SEQ ID NO: 8 by one or two amino acid substitutions,
   the CDRH2 comprises or consists of the amino acid sequence AISGSRGSTYYADSVKG (SEQ ID NO: 9), or an amino acid sequence differing from SEQ ID NO: 9 by one or two amino acid substitutions,
   the CDRH3 comprises or consists of the amino acid sequence DNWNFPFYLDY (SEQ ID NO: 10), or an amino acid sequence differing from SEQ ID NO: 10 by one or two amino acid substitutions, or
   the CDRH1 comprises or consists of the amino acid sequence SYAIS (SEQ ID NO: 14), or an amino acid sequence differing from SEQ ID NO: 14 by one or two amino acid substitutions,
   the CDRH2 comprises or consists of the amino acid sequence GIIPIFGAANYAQKFQG (SEQ ID NO: 15), or an amino acid sequence differing from SEQ ID NO: 15 by one or two amino acid substitutions,
   the CDRH3 comprises or consists of the amino acid sequence GGLESSYGMDV (SEQ ID NO: 16), or an amino acid sequence differing from SEQ ID NO: 16 by one or two amino acid substitutions, or
   the CDRH1 comprises or consists of the amino acid sequence SYAIS (SEQ ID NO: 66), or an amino acid sequence differing from SEQ ID NO: 66 by one or two amino acid substitutions,
   the CDRH2 comprises or consists of the amino acid sequence GIIPIFGTANYAQKFQG (SEQ ID NO: 67), or an amino acid sequence differing from SEQ ID NO: 67 by one or two amino acid substitutions,
   the CDRH3 comprises or consists of the amino acid sequence GIKGYAFDI (SEQ ID NO: 68), or an amino acid sequence differing from SEQ ID NO: 68 by one or two amino acid substitutions, or
   the CDRH1 comprises or consists of the amino acid sequence SYWMT (SEQ ID NO: 72), or an amino acid sequence differing from SEQ ID NO: 72 by one or two amino acid substitutions,
   the CDRH2 comprises or consists of the amino acid sequence NIQQDGSEKYYLDSVKG (SEQ ID NO: 73), or an amino acid sequence differing from SEQ ID NO: 73 by one or two amino acid substitutions,
   the CDRH3 comprises or consists of the amino acid sequence DWAYFDY (SEQ ID NO: 74), or an amino acid sequence differing from SEQ ID NO: 74 by one or two amino acid substitutions, or
   the CDRH1 comprises or consists of the amino acid sequence SYAIS (SEQ ID NO: 78), or an amino acid sequence differing from SEQ ID NO: 78 by one or two amino acid substitutions,
   the CDRH2 comprises or consists of the amino acid sequence GIIPIFGTANYAQKFQG (SEQ ID NO: 79), or an amino acid sequence differing from SEQ ID NO: 79 by one or two amino acid substitutions,
   the CDRH3 comprises or consists of the amino acid sequence GGAPAVWSYFDY (SEQ ID NO: 80), or an amino acid sequence differing from SEQ ID NO: 80 by one or two amino acid substitutions, or
   the CDRH1 comprises or consists of the amino acid sequence SYYMH (SEQ ID NO: 84), or an amino acid sequence differing from SEQ ID NO: 84 by one or two amino acid substitutions,
   the CDRH2 comprises or consists of the amino acid sequence IINPSGGSTSYAQKFQG (SEQ ID NO: 85), or an amino acid sequence differing from SEQ ID NO: 85 by one or two amino acid substitutions,
   the CDRH3 comprises or consists of the amino acid sequence DKSLGWFDP (SEQ ID NO: 86), or an amino acid sequence differing from SEQ ID NO: 86 by one or two amino acid substitutions, or
   the CDRH1 comprises or consists of the amino acid sequence SYAMS (SEQ ID NO: 90), or an amino acid sequence differing from SEQ ID NO: 90 by one or two amino acid substitutions,
   the CDRH2 comprises or consists of the amino acid sequence AISGSGGSTYYADSVKG (SEQ ID NO: 91), or an amino acid sequence differing from SEQ ID NO: 91 by one or two amino acid substitutions,
   the CDRH3 comprises or consists of the amino acid sequence ELGLRGAFDI (SEQ ID NO: 92), or an amino acid sequence differing from SEQ ID NO: 92 by one or two amino acid substitutions, or
   the CDRH1 comprises or consists of the amino acid sequence DYTMH (SEQ ID NO: 96), or an amino acid sequence differing from SEQ ID NO: 96 by one or two amino acid substitutions,
   the CDRH2 comprises or consists of the amino acid sequence LISWDGGSTYYADSVKG (SEQ ID NO: 97), or an amino acid sequence differing from SEQ ID NO: 97 by one or two amino acid substitutions,
   the CDRH3 comprises or consists of the amino acid sequence DKGWLQLGN (SEQ ID NO: 98), or an amino acid sequence differing from SEQ ID NO: 98 by one or two amino acid substitutions, or
   the CDRH1 comprises or consists of the amino acid sequence SYAIS (SEQ ID NO: 116), or an amino acid sequence differing from SEQ ID NO: 116 by one or two amino acid substitutions,
   the CDRH2 comprises or consists of the amino acid sequence GIIPIFGTANYAQKFQG (SEQ ID NO: 117), or an amino acid sequence differing from SEQ ID NO: 117 by one or two amino acid substitutions,
   the CDRH3 comprises or consists of the amino acid sequence QTGYSSGWPSPDAFDI (SEQ ID NO: 118), or an amino acid sequence differing from SEQ ID NO: 118 by one or two amino acid substitutions, or
   the CDRH1 comprises or consists of the amino acid sequence SYAIS (SEQ ID NO: 124), or an amino acid sequence differing from SEQ ID NO: 124 by one or two amino acid substitutions,
   the CDRH2 comprises or consists of the amino acid sequence GIIPIFGTANYAQKFQG (SEQ ID NO: 125), or an amino acid sequence differing from SEQ ID NO: 125 by one or two amino acid substitutions,
   the CDRH3 comprises or consists of the amino acid sequence AVGIFGAFDI (SEQ ID NO: 126), or an amino acid sequence differing from SEQ ID NO: 126 by one or two amino acid substitutions, or
   the CDRH1 comprises or consists of the amino acid sequence SYAIS (SEQ ID NO: 132), or an amino acid sequence differing from SEQ ID NO: 132 by one or two amino acid substitutions,
   the CDRH2 comprises or consists of the amino acid sequence GIIPIFGTANYAQKFQG (SEQ ID NO: 133), or an amino acid sequence differing from SEQ ID NO: 133 by one or two amino acid substitutions,
   the CDRH3 comprises or consists of the amino acid sequence DSYDSSGYYYDWFDP (SEQ ID NO: 134), or an amino acid sequence differing from SEQ ID NO: 134 by one or two amino acid substitutions, or
   the CDRH1 comprises or consists of the amino acid sequence NYAIS (SEQ ID NO: 140), or an amino acid sequence differing from SEQ ID NO: 140 by one or two amino acid substitutions,
   the CDRH2 comprises or consists of the amino acid sequence GIIPIFGTANYAQKFQG (SEQ ID NO: 141), or an amino acid sequence differing from SEQ ID NO: 141 by one or two amino acid substitutions,
   the CDRH3 comprises or consists of the amino acid sequence DYREGYSYGLYDAFDI (SEQ ID NO: 142), or an amino acid sequence differing from SEQ ID NO: 142 by one or two amino acid substitutions, or
   the CDRH1 comprises or consists of the amino acid sequence SYAIS (SEQ ID NO: 148), or an amino acid sequence differing from SEQ ID NO: 148 by one or two amino acid substitutions,
   the CDRH2 comprises or consists of the amino acid sequence GIIPIFGTANYAQKFQG (SEQ ID NO: 149), or an amino acid sequence differing from SEQ ID NO: 149 by one or two amino acid substitutions,
   the CDRH3 comprises or consists of the amino acid sequence GGLESSYGMDV (SEQ ID NO: 150), or an amino acid sequence differing from SEQ ID NO: 150 by one or two amino acid substitutions.
Item 2. The antigen binding protein according to Item 1, further comprising a second polypeptide chain comprising a second variable domain comprising three CDRs CDRLl, CDRL2 and CDRL3, wherein
   the CDRLl comprises or consists of the amino acid sequence RASQSINSYLN (SEQ ID NO: 5), or an amino acid sequence differing from SEQ ID NO: 5 by one or two amino acid substitutions,
   the CDRL2 comprises or consists of the amino acid sequence AASSLQS (SEQ ID NO: 6), or an amino acid sequence differing from SEQ ID NO: 6 by one or two amino acid substitutions,
   and the CDRL3 comprises or consists of the amino acid sequence QQSYSTPRT (SEQ ID NO: 7), or an amino acid sequence differing from SEQ ID NO: 7 by one or two amino acid substitutions, or
   the CDRLl comprises or consists of the amino acid sequence RASQGISNWLA (SEQ ID NO: 11), or an amino acid sequence differing from SEQ ID NO: 11 by one or two amino acid substitutions,
   the CDRL2 comprises or consists of the amino acid sequence AASNLQG (SEQ ID NO: 12), or an amino acid sequence differing from SEQ ID NO: 12 by one or two amino acid substitutions,
   and the CDRL3 comprises or consists of the amino acid sequence QQGYSFPHT (SEQ ID NO: 13), or an amino acid sequence differing from SEQ ID NO: 13 by one or two amino acid substitutions, or
   the CDRLl comprises or consists of the amino acid sequence SGSSSNIGNNAVN (SEQ ID NO: 17), or an amino acid sequence differing from SEQ ID NO: 17 by one or two amino acid substitutions,
   the CDRL2 comprises or consists of the amino acid sequence YDDLLPS (SEQ ID NO: 18), or an amino acid sequence differing from SEQ ID NO: 18 by one or two amino acid substitutions,
   and the CDRL3 comprises or consists of the amino acid sequence AAWDDSLNGLV (SEQ ID NO: 19), or an amino acid sequence differing from SEQ ID NO: 19 by one or two amino acid substitutions, or
   the CDRL1 comprises or consists of the amino acid sequence SGSSSNIGAGYDVH (SEQ ID NO: 69), or an amino acid sequence differing from SEQ ID NO: 69 by one or two amino acid substitutions,
   the CDRL2 comprises or consists of the amino acid sequence GNSNRPS (SEQ ID NO: 70), or an amino acid sequence differing from SEQ ID NO: 70 by one or two amino acid substitutions, and
   the CDRL3 comprises or consists of the amino acid sequence QSYDSSLTGV (SEQ ID NO: 71), or an amino acid sequence differing from SEQ ID NO: 71 by one or two amino acid substitutions, or
   the CDRLl comprises or consists of the amino acid sequence SGDALPKKYAY (SEQ ID NO: 75), or an amino acid sequence differing from SEQ ID NO: 75 by one or two amino acid substitutions,
   the CDRL2 comprises or consists of the amino acid sequence GDSKRPS (SEQ ID NO: 76), or an amino acid sequence differing from SEQ ID NO: 76 by one or two amino acid substitutions, and
   the CDRL3 comprises or consists of the amino acid sequence YSTDSSGNQGV (SEQ ID NO: 77), or an amino acid sequence differing from SEQ ID NO: 77 by one or two amino acid substitutions, or
   the CDRLl comprises or consists of the amino acid sequence SGSSSNIGNNAVN (SEQ ID NO: 81), or an amino acid sequence differing from SEQ ID NO: 81 by one or two amino acid substitutions,
   the CDRL2 comprises or consists of the amino acid sequence YDDLLPS (SEQ ID NO: 82), or an amino acid sequence differing from SEQ ID NO: 82 by one or two amino acid substitutions, and
   the CDRL3 comprises or consists of the amino acid sequence AAWDDSLNGLV (SEQ ID NO: 83), or an amino acid sequence differing from SEQ ID NO: 83 by one or two amino acid substitutions, or
   the CDRLl comprises or consists of the amino acid sequence RASQSISSYLN (SEQ ID NO: 87), or an amino acid sequence differing from SEQ ID NO: 87 by one or two amino acid substitutions,
   the CDRL2 comprises or consists of the amino acid sequence AASSLQS (SEQ ID NO: 88), or an amino acid sequence differing from SEQ ID NO: 88 by one or two amino acid substitutions,
   and the CDRL3 comprises or consists of the amino acid sequence QQSYSTPFT (SEQ ID NO: 89), or an amino acid sequence differing from SEQ ID NO: 89 by one or two amino acid substitutions, or
   the CDRLl comprises or consists of the amino acid sequence SGSRSNIGTNTVN (SEQ ID NO: 93), or an amino acid sequence differing from SEQ ID NO: 93 by one or two amino acid substitutions,
   the CDRL2 comprises or consists of the amino acid sequence NNLQRPS (SEQ ID NO: 94), or an amino acid sequence differing from SEQ ID NO: 94 by one or two amino acid substitutions, and
   the CDRL3 comprises or consists of the amino acid sequence ATWDDSLKGVV (SEQ ID NO: 95), or an amino acid sequence differing from SEQ ID NO: 95 by one or two amino acid substitutions,, or
   the CDRLl comprises or consists of the amino acid sequence IGTSSDVGGYNSVS (SEQ ID NO: 99), or an amino acid sequence differing from SEQ ID NO: 99 by one or two amino acid substitutions,
   the CDRL2 comprises or consists of the amino acid sequence DISERPS (SEQ ID NO: 100), or an amino acid sequence differing from SEQ ID NO: 100 by one or two amino acid substitutions, and
   the CDRL3 comprises or consists of the amino acid sequence SSYTATSVI (SEQ ID NO: 101), or an amino acid sequence differing from SEQ ID NO: 101 by one or two amino acid substitutions, or
   the CDRLl comprises or consists of the amino acid sequence RASQGISSYLA (SEQ ID NO: 119), or an amino acid sequence differing from SEQ ID NO: 119 by one or two amino acid substitutions,
   the CDRL2 comprises or consists of the amino acid sequence AASTLQS (SEQ ID NO: 120), or an amino acid sequence differing from SEQ ID NO: 120 by one or two amino acid substitutions, and
   the CDRL3 comprises or consists of the amino acid sequence QQLNSYPPT (SEQ ID NO: 121), or an amino acid sequence differing from SEQ ID NO: 121 by one or two amino acid substitutions, or
   the CDRLl comprises or consists of the amino acid sequence SGSNSNIGSNTVN (SEQ ID NO: 127), or an amino acid sequence differing from SEQ ID NO: 127 by one or two amino acid substitutions,
   the CDRL2 comprises or consists of the amino acid sequence SNNQRPS (SEQ ID NO: 128), or an amino acid sequence differing from SEQ ID NO: 128 by one or two amino acid substitutions, and
   the CDRL3 comprises or consists of the amino acid sequence AAWDDSLNGPYVV (SEQ ID NO: 129), or an amino acid sequence differing from SEQ ID NO: 129 by one or two amino acid substitutions, or
   the CDRLl comprises or consists of the amino acid sequence TGTSSDVGGYDFVS (SEQ ID NO: 135), or an amino acid sequence differing from SEQ ID NO: 135 by one or two amino acid substitutions,
   the CDRL2 comprises or consists of the amino acid sequence EVSTRHS (SEQ ID NO: 136), or an amino acid sequence differing from SEQ ID NO: 136 by one or two amino acid substitutions, and
   the CDRL3 comprises or consists of the amino acid sequence SSFTSSPSVV (SEQ ID NO: 137), or an amino acid sequence differing from SEQ ID NO: 137 by one or two amino acid substitutions, or
   the CDRLl comprises or consists of the amino acid sequence TGTSSDVGGYNYVS (SEQ ID NO: 143), or an amino acid sequence differing from SEQ ID NO: 143 by one or two amino acid substitutions,
   the CDRL2 comprises or consists of the amino acid sequence DVSNRPS (SEQ ID NO: 144), or an amino acid sequence differing from SEQ ID NO: 144 by one or two amino acid substitutions, and
   the CDRL3 comprises or consists of the amino acid sequence SSYRSSYTLV (SEQ ID NO: 145), or an amino acid sequence differing from SEQ ID NO: 145 by one or two amino acid substitutions, or
   the CDRLl comprises or consists of the amino acid sequence SGSSSNIGNNAVN (SEQ ID NO: 151), or an amino acid sequence differing from SEQ ID NO: 151 by one or two amino acid substitutions,
   the CDRL2 comprises or consists of the amino acid sequence YDDLLPS (SEQ ID NO: 152), or an amino acid sequence differing from SEQ ID NO: 152 by one or two amino acid substitutions, and
   the CDRL3 comprises or consists of the amino acid sequence AAWDDSLSGYV (SEQ ID NO: 153), or an amino acid sequence differing from SEQ ID NO: 153 by one or two amino acid substitutions.
Item 3. The antigen binding protein according to Item 1 or 2, comprising an amino acid sequence having at least 75%, preferably at least 90%, and more preferably at least 95% or at least 97%, 98% or 99/ sequence identity to a sequence selected from 
QVQLVQSGGGVVQPGRSLRLSCAASGFTFSSYGMHWVRQAPGKGLEWVAVIS
YDGSNKYYADSVKGRFTISRDNSKNTLYLQMNSLRAEDTAVYYCAKDLVGGK
TWDYGMDVWGQGTTVTVSS (SEQ ID NO: 20),
 and 
DIVMTQPPSSLSASVGDRVTITCRASQSINSYLNWYQQKPGKAPKLLIYAASSLQ
SGVPSRFSGSGSGTDFTLTISSLQPEDFATYYCQQSYSTPRTFGQGTKVEIK (SEQ
ID NO: 21),
 
QVQLVQSGGGLVQPGGSLRLSCAASGFTFNNYAMTWVRQAPGKGLEWVSAIS
GSRGSTYYADSVKGRFTISRDNSKNTLYLEMNSLRAEDTAVYFCAKDNWNFPF
YLDYWGQGTLVTVSS (SEQ ID NO: 22),
 and 
DIVMTQSPSSVSASVGDRVTITCRASQGISNWLAWYQQKPGKAPKLLIYAASNL
QGGVPSRFSGSGSGTDFTLTISSLQPEDFATYFCQQGYSFPHTFGQGTKLEIK(SE
Q ID NO: 23),
 and 
QVQLVQSGAEVKKPGSSVKVSCKASGGTFSSYAISWVRQAPGQGLEWMGGIIPI
FGAANYAQKFQGRVTITADESTSTAYMELSSLRSEDTAVYYCARGGLESSYGM
DVWGQGTTVTVSS (SEQ ID NO: 24),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 25),
 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARGIKGYAFDI
WGQGTMVTVSS (SEQ ID NO: 102),
 and 
QSVLTQPPSVSGAPGQRVTISCSGSSSNIGAGYDVHWYQQLPGRAPKLLIYGNS
NRPSGVPDRFSGSKSGTSASLAITGLQAEDEADYYCQSYDSSLTGVFGGGTKVT
VL (SEQ ID NO: 103),
 
EVQLLESGGGLVQPGGSLRLSCAASGFNFDSYWMTWVRQAPGKGLEWVANIQ
QDGSEKYYLDSVKGRFTISRDNTKNSLYLQMNSLRDEDTAVYYCARDWAYFD
YWGQGTLVTVSS (SEQ ID NO: 104),
 and 
SYELTQPPSVSVSPGQTARITCSGDALPKKYAYWYQQKSGQAPVLVIYGDSKRP
SGIPERFSGSSSGTMATLTVSGAQVEDEADYYCYSTDSSGNQGVFGGGTKLTVL
(SEQ ID NO: 105),
 
QVQLVQSGAEVKKPGSSVKVSCKASGGTFSSYAISWVRQAPGQGLEWMGGIIPI
FGTANYAQKFQGRVTITTDESMSTAYMELSSLRSEDTAVYYCARGGAPAVWS
YFDYWGQGTLVTVSS (SEQ ID NO: 106),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 107),
 
QVQLVQSGAEVKKPEASVKVSCKASGYTFTSYYMHWVRQAPGQGLEWMGIIN
PSGGSTSYAQKFQGRVTMTRDTSTSTVYMELSSLRSEDTAVYYCARDKSLGWF
DPWGQGTLVTVSS (SEQ ID NO: 108),
 and 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPFTFGPGTKVDIK (SEQ
ID NO: 109),
 
EVQLLESGGGLVQPGGSLRLSCAASGFTFSSYAMSWVRQAPGKGLEWVSAISG
SGGSTYYADSVKGRFTISRDNSKNTLYLQMNSLRAEDTAVYYCAKELGLRGAF
DIWGQGTMVTVSS (SEQ ID NO: 110),
 and 
QSVLTQPPSASGTPGQRVTFSCSGSRSNIGTNTVNWYQQLPGTAPKLLIYNNLQ
RPSGVPDRFSGSRSGTSASLAISGLQSEDEAEYYCATWDDSLKGVVFGGGTKVT
VL (SEQ ID NO: 111),
 
EVQLVESGGVVVQPGGSLRLSCAASGFTFDDYTMHWVRQAPGKGLEWVSLIS
WDGGSTYYADSVKGRFTISRDNSKNSLYLQMNSLRTEDTALYYCAKDKGWLQ
LGNWGQGTLVTVSS (SEQ ID NO: 112),
 and 
QSALTQPASVSGSPGQSITISCIGTSSDVGGYNSVSWYQQYPGRAPKLMIYDISE
RPSGVSTRFSGSKSGNTASLTISGLQAEDEADYYCSSYTATSVIFGGGTKVTVL
(SEQ ID NO: 103),
 
QVQLVQSGAEVKKPGSSVKVSCKAPGGTFSSYAISWVRQAPGQGLEWMGGIIPI
FGTANYAQKFQGRVTITADKSTSTAYMELSSLRSEDTAVYYCASQTGYSSGWP
SPDAFDIWGQGTMVTVSS (SEQ ID NO: 114),
 and 
DIQMTQSPSSLSASVGDRVTITCRASQGISSYLAWYQQKPGKVPKLLIYAASTLQ
SGVPSRFSGSGSGTDFTLTISSLQPEDFATYYCQQLNSYPPTFGPGTKVDIK (SEQ
ID NO: 115),
 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARAVGIFGAFDI
WGQGTMVTVSS (SEQ ID NO: 122),
 and 
QSVLTQPPSASGTPGQRVTISCSGSNSNIGSNTVNWYQQLPGTAPKLLIYSNNQR
PSGVPDRFSGSKSGTSASLAISGLQSEDEADYYCAAWDDSLNGPYVVFGGGTK
LTVL (SEQ ID NO: 123),
 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARDSYDSSGYY
YDWFDPWGQGTLVTVSS (SEQ ID NO: 130),
 and 
QSALTQPASVSGSPGQSITISCTGTSSDVGGYDFVSWYQQHPGKAPKLMIYEVS
TRHSGVSNRFSASKSGNTASLTISGLQAEDEADYYCSSFTSSPSVVFGGGTKVTV
L (SEQ ID NO: 131),
 and 
QIQLVQSGAEVKKPGSSVKVSCKASGGIFSNYAISWVRQAPGQGLEWMGGIIPI
FGTANYAQKFQGRVTITADESTSTAYMELSSLRSEDTAVYYCARDYREGYSYG
LYDAFDIWGQGTMVTVSS (SEQ ID NO: 138),
 and 
QSALTQPASVSGSPGQSITISCTGTSSDVGGYNYVSWYQQHPGKAPKLMIYDVS
NRPSGVSNRFSGSKSGNTASLTISGLQAEDEADYYCSSYRSSYTLVFGGGTKLT
VL (SEQ ID NO: 139),
 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARGGLESSYGM
DVWGQGTTVTVSS (SEQ ID NO: 146),
 and 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLSGYVLGTGTKLT
VL (SEQ ID NO: 147).
Item 4. The antigen binding protein according to any one of Items 1 to 3, wherein said antigen binding protein specifically binds to an epitope comprising or consisting of at least three amino acid positions of the MAGE-A3 antigenic peptide of SEQ ID NO: 1, preferably, at least three amino acid positions selected from the group of amino acid positions consisting of positions 1 to 6 and 8 or 9 or 6, 8 and 9, with a selection including position 6 being most preferred, of the amino acid sequence of SEQ ID NO: 1.
Item 5. The antigen binding protein according to any one of Items 1 to 4, wherein said antigen binding protein specifically binds to the amino acid sequence KVAELVHFL of the antigenic peptide of SEQ ID NO: 1 and wherein said antigenic peptide is in a complex with a human leukocyte antigen (HLA) protein.
Item 6. The antigen binding protein according to Item 5, wherein said antigenic peptide is in a complex with a human leukocyte antigen (HLA) protein HLA-A*02, in particular HLA-A*02:01.
Item 7. An antigen binding protein according to any one of Items 1 to 6, wherein said antigen binding protein has an EC₅₀ value for the MAGE-A3/MHC complex presenting cells which is less than 200 nM, less than about 120 nM, such as less than 10 nM. or such as between 1 nM and 200 nM, or between 1 nM and 120 nM.
Item 8. The antigen binding protein according to any one of Items 1 to 7, wherein the antigen binding protein furthermore binds to the antigenic peptides KMAELVHFL (SEQ ID NO: 20) and/or SAAELVHFL (SEQ ID NO: 44) when said antigenic peptides are in a complex with a MHC protein, preferably in complex with HLA-A*02.
Item 9. The antigen binding protein according to any one of Items 1 to 8, wherein the antigen binding protein is an antibody or a binding fragment thereof, a bispecific, trispecific (bi-paratopic), multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain or nanobody, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).
Item 10. The antigen binding protein according to any one of Items 1 to 9, wherein the antigen binding protein is at least bispecific and furthermore binds to an antigen selected from the group consisting of CD3, CD28, OX40, GITR, CD137, CD27, CD40L, HVEM or human glucocorticoid-induced TNFR-related protein (GITR).
Item 11. The antigen binding protein according to any one of Items 1 to 10, wherein two or more of the amino acid sequences are linked via linkers and/or IgG hinge sequences, wherein preferably the linker or linkers have an amino acid sequence selected from the group consisting of GGGGS (SEQ ID NO: 30), GGGSG (SEQ ID NO: 31), GGSGG (SEQ ID NO: 32), GSGGG (SEQ ID NO: 33), GSGGGP (SEQ ID NO: 34), GGEPS (SEQ ID NO: 35), GGEGGGP (SEQ ID NO: 36), GGEGGGSEGGGS (SEQ ID NO: 37), GGGSGGGG (SEQ ID NO: 38), GGGGSGGGGSGGGGSGGGGSGGGS (SEQ ID NO: 39), GGGGSGGGGSGGGGSGGGGS (SEQ ID NO: 40), EAAAK (SEQ ID NO: 41), and EAAAKEAAAKEAAAK (SEQ ID NO: 42).
Item 12. The antigen binding protein according to any one of Items 1 to 11, further comprising a half-life extending domain, such as, for example, an Fc fragment.
Item 13. The antigen binding protein according to Item 12, comprising one or more amino acid substitutions which remove a glycosylation site, wherein preferably the substitution is an N to Q substitution.
Item 14. The antigen binding protein according to Item 12 or 13, wherein the half-life extending domain comprises one or more amino acid substitutions which facilitate dimerization of the amino acid sequences forming the Fc fragment, such as the C_{H}2 domains and/or C_{H}3 domains, in particular S228P (numbered according to the EU numbering scheme).
Item 15. The antigen binding protein according to Item 14, wherein one of the domains comprises one or more amino acid substitutions selected from the group consisting of S228P, F234A, L234A, L235A, R409K, M252Y, S254T, T256E, Y349C, T366S, L368A, Y407V, S354C, and T366W (numbered according to the EU numbering scheme).
Item 16. The antigen binding protein according to any one of Items 12 to 15, wherein the half-life extending domain comprises one or more amino acid substitutions which prevent or reduce binding to FcγR, such as, for example, an N297G amino acid substitution (numbered according to the EU numbering scheme).
Item 17. The antigen binding protein according to any one of Items 12 to 16, wherein the half-life extending domain comprises one or more amino acid substitutions which promote binding to FcRn, for example an M252Y, S254T and/or T256E amino acid substitution (numbered according to the EU numbering scheme).
Item 18. The antigen binding protein according to any one of Items 1 to 17, further comprising an effector molecule, wherein the effector molecule is a therapeutic agent selected from the group consisting of a drug, a toxin, a radioisotope, a protein, a peptide, and a nucleic acid or wherein the effector molecule is a label.
Item 19. A nucleic acid molecule encoding an antigen binding protein according to any one of Items 1 to 18 or at least one component thereof, a vector comprising the nucleic acid molecule, such as, for example, an expression vector comprising and expressing the nucleic acid molecule, or a recombinant or genetically modified cell comprising and optionally expressing the nucleic acid molecule or vector.
Item 20. A recombinant or genetically modified host cell according to Item 19, wherein the cell is a T cell, a hybridoma cell, or an antibody excreting cell.
Item 21. A method of producing an antigen binding protein according to any one of Items 1 to 18, comprising a) culturing a host cell according to Item 19 or 20 under conditions effective to express the antigen binding protein; and b) obtaining the expressed antigen binding protein from said host cell or the culture medium of said host cell.
Item 22. A method of producing a pharmaceutical composition comprising formulating the antigen binding protein according to any one of Items 1 to 18 or the recombinant or genetically modified host cell according to Item 19 or 20 with a suitable pharmaceutically acceptable carrier.
Item 23. A pharmaceutical composition, produced according to Item 22.
Item 24. The antigen binding protein according to any one of Items 1 to 18, the recombinant or genetically modified host cell according to Item 19 or 20 or the pharmaceutical composition according to Item 23 for use as a medicament, preferably for use in the prevention or treatment of a cancer and/or a metastasis thereof.
Item 25. The antigen binding protein according to any one of Items 1 to 18, the recombinant or genetically modified host cell according to Item 19 or 20 or the pharmaceutical composition according to Item 23 for use in the prevention or treatment of a cancer and/or a metastasis thereof according to Item 24, wherein the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer.
Item 26. A method for eliciting a CD8-positive T-cell response in a subject, comprising administering to said subject the pharmaceutical composition according to Item 23.
Item 27. A method of preventing or treating a cancer cell expressing MAGE-A3 and/or a metastasis thereof, the method comprising administering to a subject suffering or prospectively suffering from said cancer and/or a metastasis thereof an effective amount of the antigen binding protein according to any one of Items 1 to 18, the recombinant or genetically modified host cell according to Item 19 or 20 or the pharmaceutical composition according to Item 23, wherein preferably the method is combined with a further anti-senescence and/or cancer treatment, wherein preferably the further treatment is selected from the group consisting of chemotherapy, radiotherapy, a treatment with immune-stimulating substances, gene therapy, a treatment with antibodies and a treatment using suitable dendritic cells.
Item 28. The method of preventing or treating a cancer cell and/or a metastasis thereof according to Item 27, wherein the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer..
Item 29. A kit comprising at least one of the antigen binding proteins according to any one of Items 1 to 18, the recombinant or genetically modified host cell according to Item 19 or 20 or the pharmaceutical composition according to Item 23, and auxiliary agents and/or instructions for use thereof.
Item 30. Use of a kit according to Item 29 for producing an antigen binding protein according to any one of Items 1 to 18, for producing a pharmaceutical composition according to Item 23, for eliciting a CD8-positive T-cell response in a subject according to Item 26, or for method of preventing or treating a cancer cell and/or a metastasis thereof according to Item 27 or 28.

The invention will now be described in more detail with reference to the following figures, sequence listing, and examples. All literature and patent documents cited herein are hereby incorporated by reference in their entirety. While the invention has been illustrated and described in detail in the foregoing description, the examples are to be considered illustrative or exemplary and not restrictive.

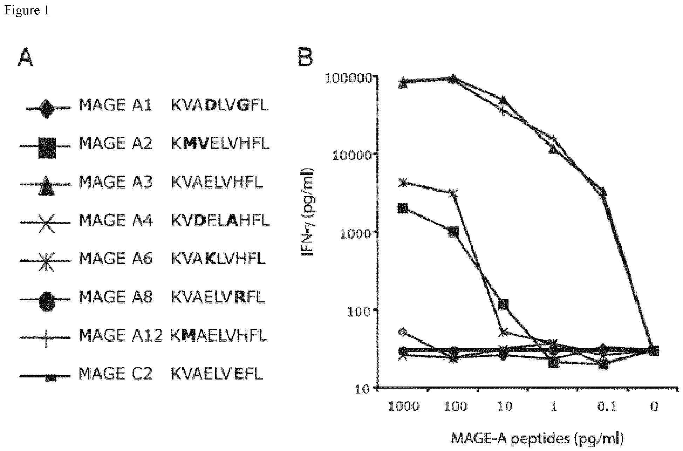

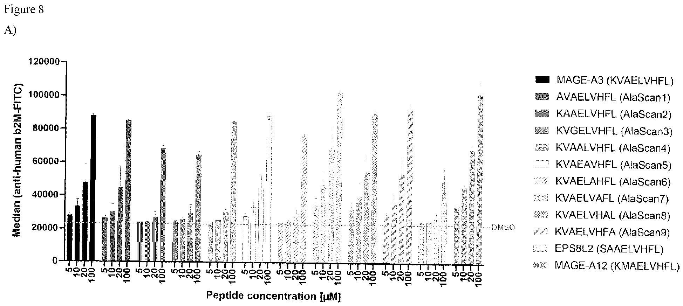

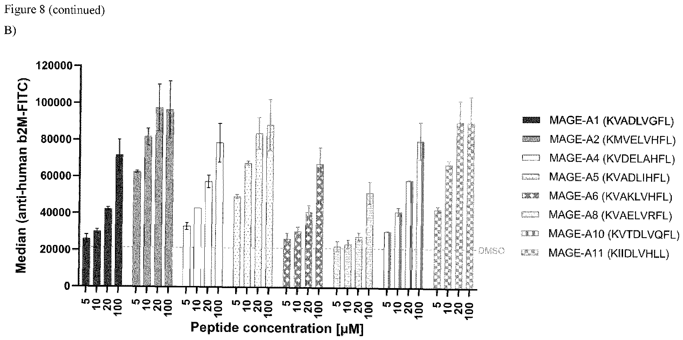

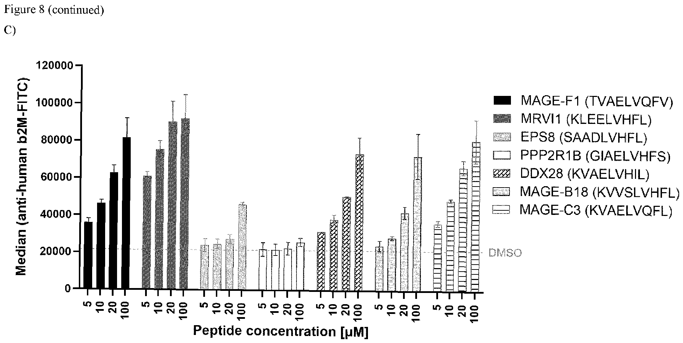

Figure 1 shows the analysis of TCR-T targeting HLA-A*0201/MAGE-A3₁₁₂₋₁₂₀ (Fig.8 from Chinnasamy N *et al.,* 2011. PMID: 21149604). An increased functional avidity of TCR-T by A118T in CDR3 of TCR α-chain was shown, and a recognition of other members of the MAGE-A family by TCR-T (A118T) was identified. T2 cells pulsed with indicated peptides were incubated with TCR-T (A118T). The amount of IFNg secreted in the cell culture supernatant was measured by ELISA. It can be seen that HLA-A*0201 loaded with MAGE-A3 and MAGE-A12-derived peptides are efficiently recognized by the TCR-T (A118T). However, HLA-A*0201 loaded with MAGE-A6 and MAGE-A2-derived peptides are also recognized with lower avidity by TCR-T (A118T). KVADLVGFL (SEQ ID NO: 161), KMVELVHFL (SEQ ID NO: 56), KVAELVHFL (SEQ ID NO: 1), KVDELAHFL (SEQ ID NO: 57), KVAKLVHFL (SEQ ID NO: 59), KVAELVRFL (SEQ ID NO: 60), KMAELVHFL (SEQ ID NO: 29), and KVAELVEFL (SEQ ID NO: 162).

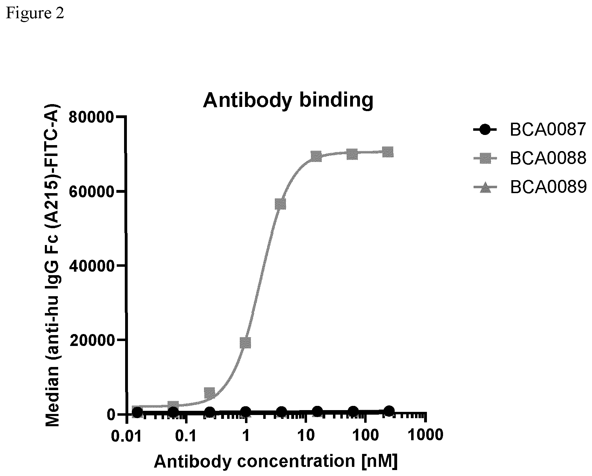

Figure 2 shows the antibody binding of the reference constructs as tested, VR-33 (BCA0088) was chosen as a comparative construct/control for the further assays and tests.

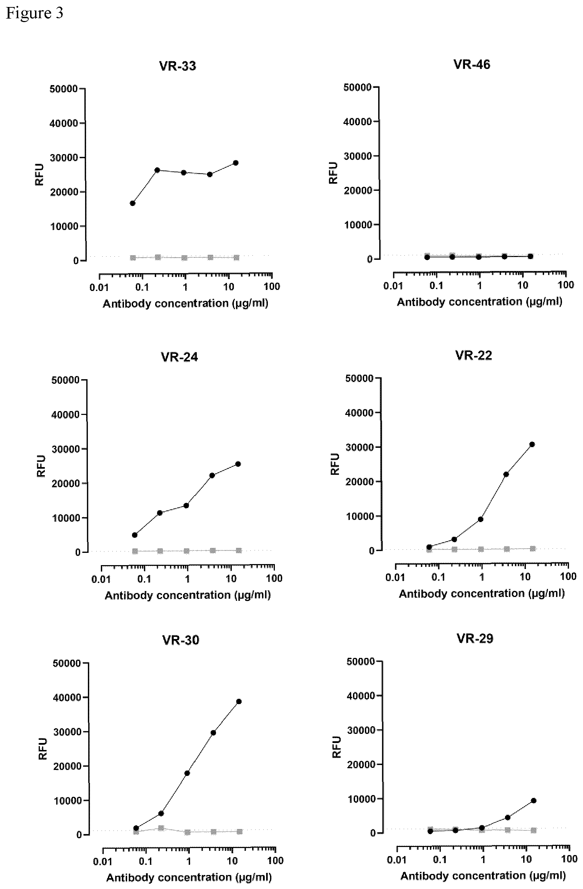

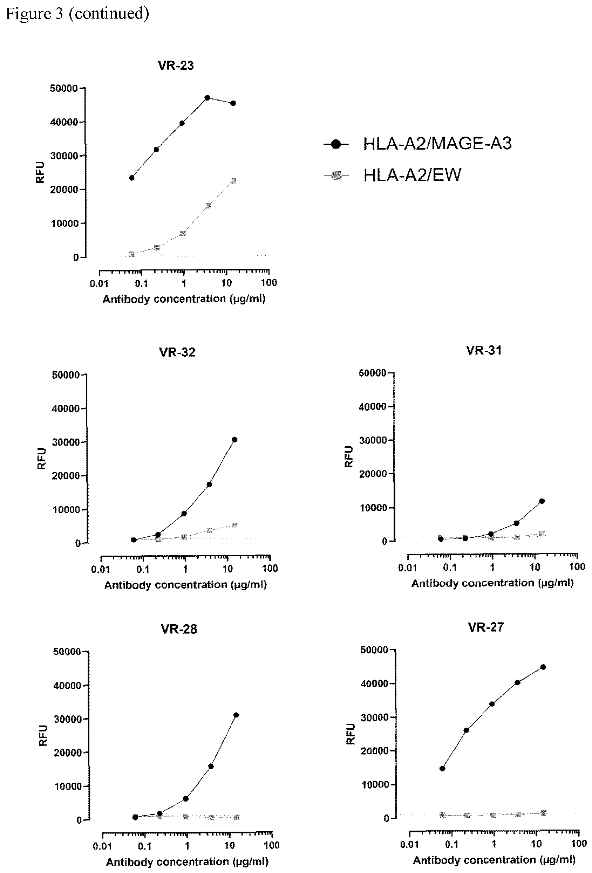

Figure 3 shows the binding analysis of selected phage display clones against recombinant pHLA complexes after re-formatting to IgG1 (Fc silenced). Selected scFv clones derived from phage display (VR-23, VR-24, VR-22, VR-32, VR-31, VR-30, VR-29, VR-28 and VR-27) were re-formatted to IgG1 (Fc silenced) and tested for binding to the recombinant target pHLA complex (HLA-A*0201/MAGE-A3) and control pHLA complex (HLA-A*0201/EW) by ELISA. Streptavidin-coated 96-well MTP plates were blocked with 50% SuperBlock/PBS for 1h at RT followed by the addition of 1µg/ml biotinylated HLA-A*0201 complexed with MAGE-A3₁₁₂₋₁₂₀ and EW peptide, respectively. pHLA-A2 complexes were incubated for 1h at RT. After washing, serial diluted antibodies were incubated for 1h at RT. Binding of antibodies to the pHLA complexes was detected using an anti-human IgG H&L conjugated with HRP. Subsequently, substrate was added (QuantaRed Enhanced Chemiluminescence HRP substrate Kit) and fluorescence was measured (Tecan Reader). RFU = relative fluorescence unit. The dotted gray line in the graphs corresponds to the background signal. VR-33 = reference molecule (WO2021/173674), VR-46 = isotype control.

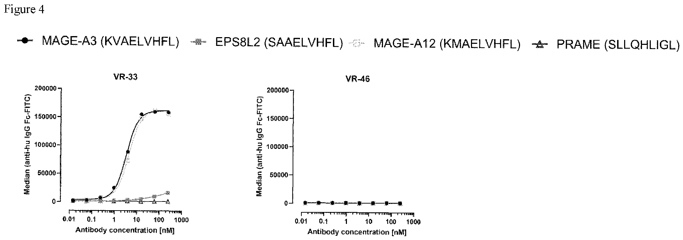

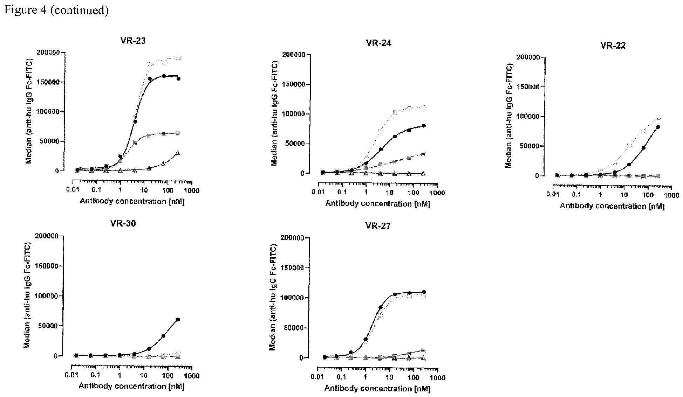

Figure 4 shows the binding of selected pHLA/MAGE-A3 antibodies to peptide-loaded T2 cells. T2 (HLA-A2+) cells were loaded with 100µM peptides derived from MAGE-A3 (KVAELVHFL) (SEQ ID NO: 1), MAGE-A12 (KMAELVHFL) (SEQ ID NO: 29), EPS8L2 (SAAELVHFL) (SEQ ID NO: 44) and PRAME (SLLQHLIGL) (SEQ ID NO: 43), respectively for 16h at 37°C. Antibodies were titrated from 250nM to 0.02nM and incubated with peptide-pulsed T2 cells for 1h on ice. Antibody binding was detected using anti-human IgG Fc-FITC. VR-33 = reference molecule/control (WO2021/173674), VR46 = isotype control. VR-23, VR-24, VR-22, VR-30 and VR-27 = HLA-A*0201/MAGE-A3₁₁₂₋₁₂₀ binders according to the present invention.

The EC₅₀ values were as follows:

| | **MAGE-A3 (KVAELVHF L)** | **EPS8L2 (SAAE LVHFL)** | **MAGE-A12 (KMAELVHF L)** | **PRAME (SLLQ HLIGL)** |
|---|---|---|---|---|
| **VR-33** | 3.419 | 330.7 | 4.393 | No binding |
| **VR-23** | 3.629 | 1.991 | 4.099 | 810 |
| **VR-24** | 4.18 | 15.76 | 2.454 | No binding |
| **VR-22** | 91.17 | No binding | 20.17 | No binding |
| **VR-30** | 98.78 | No binding | 469 | No binding |
| **VR-27** | 1.864 | 204.2 | 2.28 | No binding |

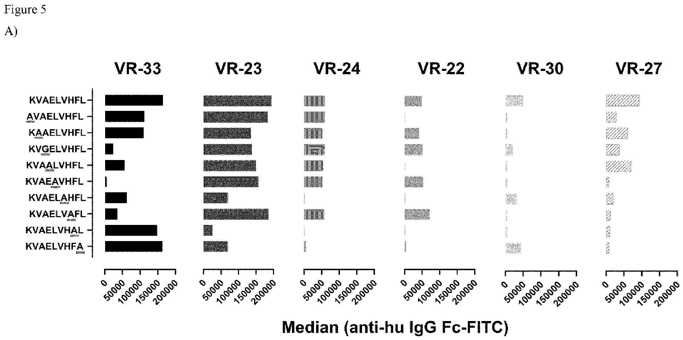

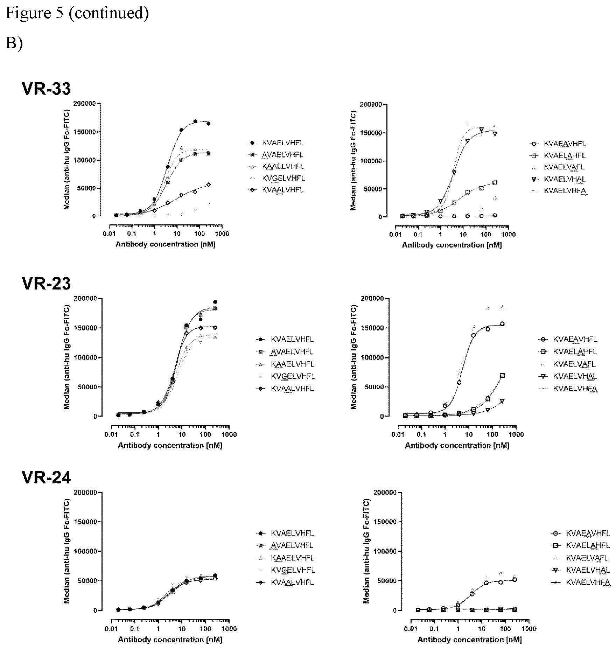

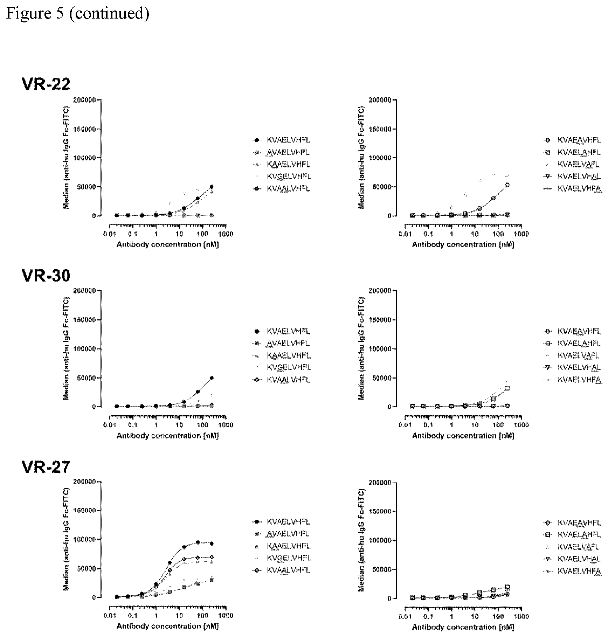

Figure 5 shows the critical amino acid positions within the peptide target sequence for pHLA/antibody binding. T2 (HLA-A2+) cells were loaded with 100µM peptides (MAGE-A3 (KVAELVHFL) (SEQ ID NO: 1)) target and a positional scanning library derived from the target peptide) for 16h at 37°C. For the positional scanning peptide library, amino acid positions changed to alanine are underlined in red. As the target sequence harbors an alanine at position 3, this site has been replaced by a glycine. Selected antibodies were titrated from 250nM to 0.02nM and incubated with peptide-pulsed T2 cells for 1h on ice. Antibody binding was detected using anti-human IgG Fc-FITC. (A) Overview. The data presented were derived from the binding analysis shown in (B). Shown are the binding signals obtained with 250nM of the respective antibody. (B) Antibody binding analysis. Binding curves for the HLA-A*0201/MAGE-A3₁₁₂₋₁₂₀ building blocks and reference antibody VR-33 (WO2021/173674) against T2 cells loaded with the target peptide and the positional scanning peptide library, respectively. VR-33 = reference molecule (WO2021/173674); VR-23, VR-24, VR-22, VR-30 and VR-27 = HLA-A*0201/MAGE-A3₁₁₂₋₁₂₀ building blocks derived from phage display. PEP1349: KVAELVHFL (SEQ ID NO: 1); PEP1350: AVAELVHFL (SEQ ID NO: 46); PEP1351: KAAELVHFL (SEQ ID NO: 47); PEP1352: KVGELVHFL (SEQ ID NO: 48); PEP1353: KVAALVHFL (SEQ ID NO: 49); PEP1354: KVAEAVHFL (SEQ ID NO: 50); PEP1355: KVAELAHFL (SEQ ID NO: 51); PEP1356: KVAELVAFL (SEQ ID NO: 52); PEP1357: KVAELVHAL (SEQ ID NO: 53); and PEP1358: KVAELVHFA (SEQ ID NO: 54).

The EC₅₀ values as determined were as follows:

| | **KVAELVH FL** | **AVAELVH FL** | **KAAELVH FL** | **KVGELVH FL** | **KVAALVH FL** |
|---|---|---|---|---|---|
| **VR-33** | 3.621 | 3.525 | 2.891 | 292.5 | 8.362 |
| **VR-23** | 6 | 6.072 | 5.273 | 6.114 | 5.104 |
| **VR-24** | 3.084 | 3.688 | 2.177 | 1.886 | 2.88 |
| **VR-22** | 79.22 | No binding | 108.6 | 5.56 | No binding |
| **VR-30** | 118.2 | No binding | No binding | 214.7 | No binding |
| **VR-27** | 2.596 | 15.51 | 2.387 | 4.609 | 2.32 |

| | **KVAEAVH FL** | **KVAELAH FL** | **KVAELVA FL** | **KVAELVH AL** | **KVAELVH FA** |
|---|---|---|---|---|---|
| **VR-33** | No binding | 6.246 | 1374 | 3.688 | 3.656 |
| **VR-23** | 5.327 | 562.8 | 5.311 | 2721 | 265.6 |
| **VR-24** | 3.507 | 728.6 | 3.042 | No binding | No binding |
| **VR-22** | 103.7 | No binding | 3.736 | No binding | No binding |
| **VR-30** | No binding | 606.6 | No binding | No binding | 255 |
| **VR-27** | No binding | 32.81 | 475 | 826.8 | 495.2 |

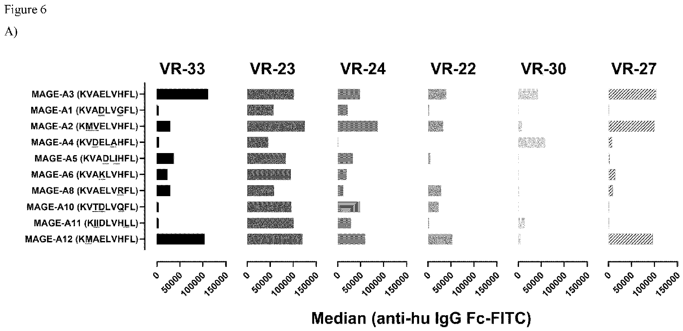

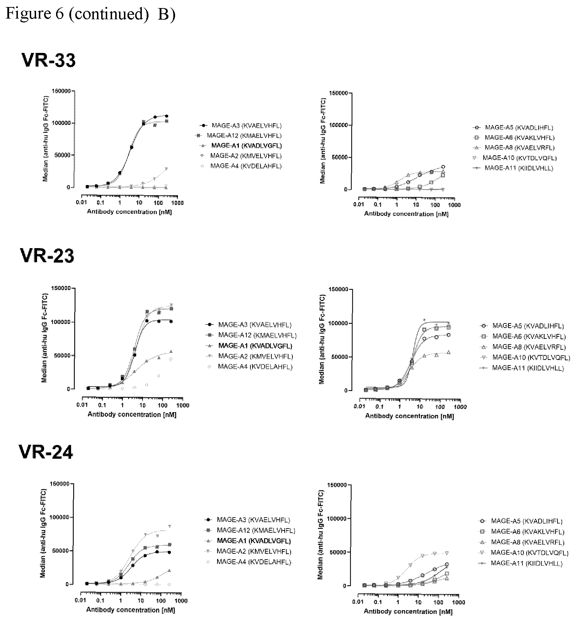

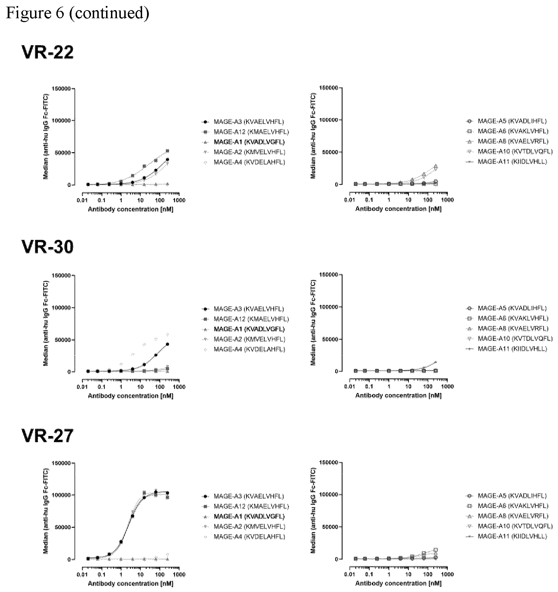

Figure 6 shows the selectivity of HLA-A*0201/MAGE-A3₁₁₂₋₁₂₀ building blocks against closely related peptides derived from MAGE-A-family members. T2 (HLA-A2+) cells were loaded with 100µM peptides derived from MAGE-A3 (KVAELVHFL) (SEQ ID NO: 1), MAGE-A1 (KVADLVGFL) (SEQ ID NO: 161), MAGE-A2 (KMVELVHFL) (SEQ ID NO: 56), MAGE-A4 (KVDELAHFL) (SEQ ID NO: 57), MAGE-A5 (KVADLIHFL) (SEQ ID NO: 58), MAGE-A6 (KVAKLVHFL) (SEQ ID NO: 59), MAGE-A8 (KVAELVRFL) (SEQ ID NO: 60), MAGE-A10 (KVTDLVQFL) (SEQ ID NO: 61), MAGE-A11 (KIIDLVHLL) (SEQ ID NO: 62) and MAGE-A12 (KMAELVHFL) (SEQ ID NO: 29), respectively for 16h at 37°C. Selected antibodies were titrated from 250nM to 0.02nM and incubated with peptide-pulsed T2 cells for 1h on ice. Antibody binding was detected using anti-human IgG Fc-FITC. Differences in the sequence between the MAGE-A-family members compared to the target sequence are underlined in red. (A) Overview. The data presented were derived from the binding analysis shown in (B). Shown are the binding signals obtained with 250nM of the respective antibody. (B) Antibody binding analysis. Binding curves for the HLA-A*0201/MAGE-A3₁₁₂₋₁₂₀ building blocks and reference antibody VR-33 (WO2021/173674) against T2 cells loaded with the target peptide and the corresponding epitope in MAGE-A family members, respectively. VR-33 = reference molecule (WO2021/173674); VR-23, VR-24, VR-22, VR-30 and VR-27 = HLA-A*0201/MAGE-A3₁₁₂₋₁₂₀ binders according to the present invention derived from phage display.

The EC₅₀ values were as follows:

| **MAGE-A1 (KVADLVGFL )** | **MAGE-A2 (KMVELVHF L)** | **MAGE-A4 (KVDELAHFL)** | **MAGE-A5 (KVADLIHFL )** |
|---|---|---|---|
| No binding | 681.9 | 230.4 | 11.87 |
| 4.742 | 4.821 | 244.7 | 3.54 |
| 155.6 | 2.731 | No binding | 24.63 |
| 866.2 | 190.3 | No binding | No binding |
| No binding | 702.9 | 5.168 | No binding |
| No binding | 2.186 | 214.2 | 189.4 |

| **MAGE-A6 (KVAKLVHF L)** | **MAGE-A8 (KVAELVRFL )** | **MAGE-A10 (KVTDLVQF L)** | **MAGE-A11 (KIIDLVHLL )** |
|---|---|---|---|
| 134.7 | 1.36 | No binding | No binding |
| 4.495 | 2.876 | 4.031 | 4.214 |
| 139.3 | 88.16 | 2.592 | 111.7 |
| No binding | 157.3 | 307.5 | No binding |
| No binding | No binding | No binding | 628.5 |
| 72.91 | 48.19 | No binding | No binding |

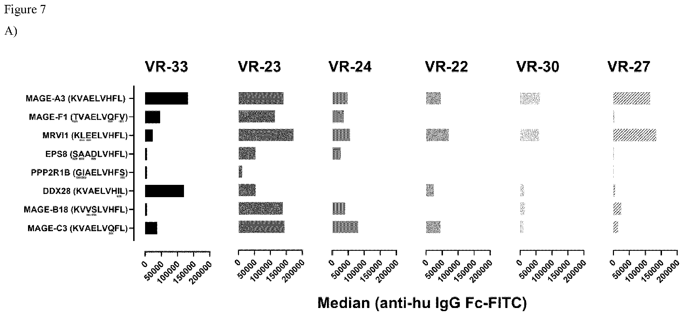

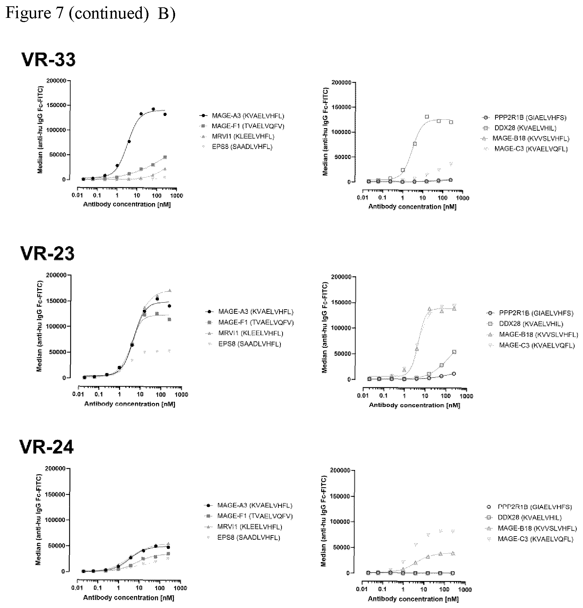

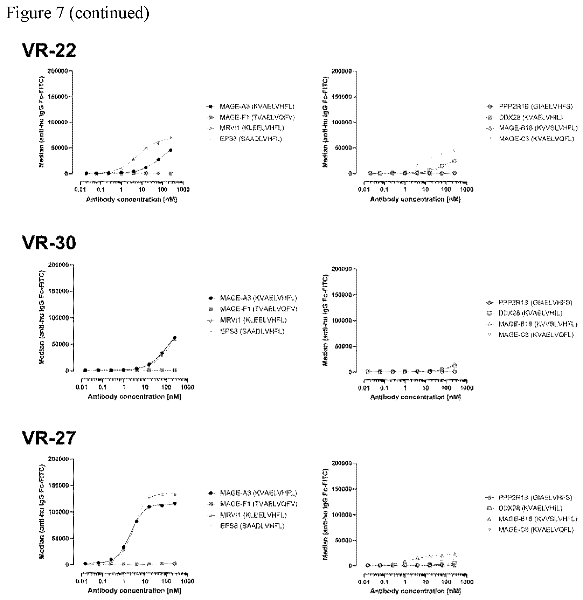

Figure 7 shows the selectivity of HLA-A*0201/MAGE-A3₁₁₂₋₁₂₀ building blocks against sequence related peptides derived from non-MAGE-A-family member. T2 (HLA-A2+) cells were loaded with 100µM peptides derived from MAGE-A3 (KVAELVHFL, SEQ ID NO: 1), MAGE-F1 (TVAELVQFV, SEQ ID NO: 154), MRVI1 (KLEELVHFL, SEQ ID NO: 155), EPS8 (SAADLVHFL, SEQ ID NO: 156), PPP2R1B (GIAELVHFS, SEQ ID NO: 157), DDX28 (KVAELVHIL, SEQ ID NO: 158), MAGE-B18 (KVVSLVHFL, SEQ ID NO: 159) and MAGE-C3 (KVAELVQFL, SEQ ID NO: 160), respectively for 16h at 37°C. Selected antibodies were titrated from 250nM to 0.02nM and incubated with peptide-pulsed T2 cells for 1h on ice. Antibody binding was detected using anti-human IgG Fc-FITC. Differences in the sequence between the non-MAGE-A-family members compared to the target sequence are underlined in red. (A) Overview. The data presented were derived from the binding analysis shown in (B). Shown are the binding signals obtained with 250nM of the respective antibody. (B) Antibody binding analysis. Binding curves for the HLA-A*0201/MAGE-A3₁₁₂₋₁₂₀ building blocks and reference antibody VR-33 (WO2021/173674) against T2 cells loaded with the target peptide and the corresponding epitope in non-MAGE-A family members, respectively. VR-33 = reference molecule (WO2021/173674); VR-23, VR-24, VR-22, VR-30 and VR-27 = HLA-A*0201/MAGE-A3₁₁₂₋₁₂₀ binders according to the present invention.

The EC₅₀ values as determined were as follows:

| | **MAGE-A3 (KVAELVHF L)** | **MAGE-F1 (TVAELVQF V)** | **MRVI1 (KLEEL VHFL)** | **EPS8 (SAADLV HFL)** |
|---|---|---|---|---|
| **VR -33** | 3.226 | 210.4 | 169.9 | 665.1 |
| **VR -23** | 4.694 | 3.577 | 5.341 | 2.579 |
| **VR -24** | 3.572 | 7.626 | 3.62 | 27.68 |
| **VR -22** | 86.95 | No binding | 6.229 | No binding |
| **VR -30** | 104.7 | No binding | 151.8 | No binding |
| **VR -27** | 1.985 | No binding | 2.818 | No binding |

| | **PPP2R1B (GIAE LVHFS)** | **DDX28 (KVAEL VHIL)** | **MAGE-B18 (KVVSLVHF L)** | **MAGE-C3 (KVAELVQF L**) |
|---|---|---|---|---|
| **VR -33** | No binding | 2.853 | No binding | 108.2 |
| **VR -23** | 129.9 | 134.9 | 4.459 | 4.317 |
| **VR -24** | No binding | No binding | 3.843 | 2.24 |
| **VR -22** | No binding | 85.88 | No binding | 8.907 |
| **VR -30** | No binding | 548.1 | 467.5 | 366.8 |
| **VR -27** | No binding | 1187 | 1.72 | 181.8 |

Figure 8 shows peptide loading efficiencies. The efficiency of peptide loading to HLA-A2 on T2 cells was determined using an anti-human b2M-FITC antibody. For this, T2 cells were incubated with respective peptides at different concentrations for 16h at 37°C and subsequently stained with anti-human b2M-FITC. (A) MAGE-A3 target peptide, positional alanine scanning library and potential off-targets. (B) Related peptides from the MAGE-A family. (C) Related peptides from non-MAGE-A family members.

### Description of the Sequences

| **SEQ ID No:** | **Name** | **Amino acid sequence** |
|---|---|---|
| 1 | MAGE-A3 | KVAELVHFL |
| 2 | | SYGMH |
| 3 | | VISYDGSNKYYADSVKG |
| 4 | | DLVGGKTWDYGMDV |
| 5 | | RASQSINSYLN |
| 6 | | AASSLQS |
| 7 | | QQSYSTPRT |
| 8 | | NYAMT |
| 9 | | AISGSRGSTYYADSVKG |
| 10 | | DNWNFPFYLDY |
| 11 | | RASQGISNWLA |
| 12 | | AASNLQG |
| 13 | | QQGYSFPHT |
| 14 | | SYAIS |
| 15 | | GIIPIFGAANYAQKFQG |
| 16 | | GGLESSYGMDV |
| 17 | | SGSSSNIGNNAVN |
| 18 | | YDDLLPS |
| 19 | | AAWDDSLNGLV |
| 20 | BCA017 6 - (VR-23) -VH | QVQLVQSGGGVVQPGRSLRLSCAASGFTFSSYGMHWVRQ APGKGLEWVAVISYDGSNKYYADSVKGRFTISRDNSKNTL YLQMNSLRAEDTAVYYCAKDLVGGKTWDYGMDVWGQG TTVTVSS |
| 21 | BCA017 6 - (VR-23) - VL | DIVMTQPPSSLSASVGDRVTITCRASQSINSYLNWYQQKPG KAPKLLIYAASSLQSGVPSRFSGSGSGTDFTLTISSLQPEDFA TYYCQQSYSTPRTFGQGTKVEIK |
| 22 | BCA017 7 - VH (VR-24) | QVQLVQSGGGLVQPGGSLRLSCAASGFTFNNYAMTWVRQ APGKGLEWVSAISGSRGSTYYADSVKGRFTISRDNSKNTLY LEMNSLRAEDTAVYFCAKDNWNFPFYLDYWGQGTLVTVS S |
| 23 | BCA017 7 - VL (VR-24) | DIVMTQSPSSVSASVGDRVTITCRASQGISNWLAWYQQKP GKAPKLLIYAASNLQGGVPSRFSGSGSGTDFTLTISSLQPED FATYFCQQGYSFPHTFGQGTKLEIK |
| 24 | BCA017 9 - VH (VR-22) | QVQLVQSGAEVKKPGSSVKVSCKASGGTFSSYAISWVRQA PGQGLEWMGGIIPIFGAANYAQKFQGRVTITADESTSTAYM ELSSLRSEDTAVYYCARGGLESSYGMDVWGQGTTVTVSS |
| 25 | BCA017 9 - VL (VR-22) | QSVLTQPPSVSEAPRQRVTISCSGSSSNIGNNAVNWYQQLP GKAPKLLIYYDDLLPSGVSDRFSGSKSGTSASLAISGLQSED EADYYCAAWDDSLNGLVFGGGTKLTVL |
| 26 | artificial | KXXEXVXFL, where X can be any amino acid |
| 27 | MAGE-A3 (human) | MPLEQRSQHCKPEEGLEARGEALGLVGAQAPATEEQEAAS SSSTLVEVTLGEVPAAESPDPPQSPQGASSLPTTMNYPLWS QSYEDSSNQEEEGPSTFPDLESEFQAALSRKVAELVHFLLL KYRAREPVTKAEMLGSVVGNWQYFFPVIFSKASSSLQLVF GIELMEVDPIGHLYIFATCLGLSYDGLLGDNQIMPKAGLLII VLAIIAREGDCAPEEKIWEELSVLEVFEGREDSILGDPKKLL TQHFVQENYLEYRQVPGSDPACYEFLWGPRALVETSYVKV LHHMVKISGGPHISYPPLHEWVLREGEE |
| 28 | MAGE-A3 (mouse) | MADSHNTQYCNLEESAQAQQELDNDQETMETSEEEEDTTT SNKVYGSAIPSPPQSPQRAYSPCVALASIPDSPSEEASIKGSE GLEDPLHLLHNAQNTKVYDLVDFLVLNYQMKAFTTKAEM |
| | | LENIGREYEEYYPLIFSEASECLKMVFGLDMVEVDSSVHTY MLVTALGITYDGMMTDVQGMPKTGILIAVLSVIFMKGNYV SEEIIWEMLNNIGLCGGRDPYIHKDPRKLISEEFVQEGYLEY RQVPNSDPPSYGFLWGPRAFAETSKMKVLQFFASINKTHPR AYPEKYAEALQDEIDRTKTWILNRCSNSSDLHTF |
| 29 | MAGE-A12 PEP1360 | KMAELVHFL |
| 30 | Linker | GGGGS |
| 31 | Linker | GGGSG |
| 32 | Linker | GGSGG |
| 33 | Linker | GSGGG |
| 34 | Linker | GSGGGP |
| 35 | Linker | GGEPS |
| 36 | Linker | GGEGGGP |
| 37 | Linker | GGEGGGSEGGGS |
| 38 | Linker | GGGSGGGG |
| 39 | Linker | GGGGSGGGGSGGGGSGGGGSGGGS |
| 40 | Linker | GGGGSGGGGSGGGGSGGGGS |
| 41 | Linker | EAAAK |
| 42 | Linker | EAAAKEAAAKEAAAK |
| 43 | PEP1378 PRAME | SLLQHLIGL |
| 44 | PEP1359 | SAAELVHFL |
| 45 | mMA | YLEYEQVPG |
| 46 | PEP1350 | AVAELVHFL |
| 47 | PEP1351 | KAAELVHFL |
| 48 | PEP1352 | KVGELVHFL |
| 49 | PEP1353 | KVAALVHFL |
| 50 | PEP1354 | KVAEAVHFL |
| 51 | PEP1355 | KVAELAHFL |
| 52 | PEP1356 | KVAELVAFL |
| 53 | PEP1357 | KVAELVHAL |
| 54 | PEP1358 | KVAELVHFA |
| 55 | PEP1361 | KVADLGFL |
| 56 | PEP1362 | KMVELVHFL |
| 57 | PEP1363 | KVDELAHFL |
| 58 | PEP1364 | KVADLIHFL |
| 59 | PEP1365 | KVAKLVHFL |
| 60 | PEP1366 | KVAELVRFL |
| 61 | PEP1367 | KVTDLVQFL |
| 62 | PEP1368 | KIIDLVHLL |
| 63 | VR-33 - VH | QVQLVQSGAEVKKPGSSVKVSCKASGGTFSSYAISWVRQA PGQGLEWMGGIIPIFGTANYAQKFQGRVTITADESTSTAYM ELSSLRSEDTAVYYCARDMDTFSMVTLFDYWGQGTLVTV SS |
| 64 | VR-33 - VL | DIQMTQSPSSLSASVGDRVTITCRASQSISSYLNWYQQKPG KAPKLLIYAASSLQSGVPSRFSGSGSGTDFTLTISSLQPEDFA TYYCQQSYSWPLTFGGGTKVEIK |
| 65 | MAGE-A3 | FLWGPRALV |
| 66 | | SYAIS |
| 67 | | GIIPIFGTANYAQKFQG |
| 68 | | GIKGYAFDI |
| 69 | | SGSSSNIGAGYDVH |
| 70 | | GNSNRPS |
| 71 | | QSYDSSLTGV |
| 72 | | SYWMT |
| 73 | | NIQQDGSEKYYLDSVKG |
| 74 | | DWAYFDY |
| 75 | | SGDALPKKYAY |
| 76 | | GDSKRPS |
| 77 | | YSTDSSGNQGV |
| 78 | | SYAIS |
| 79 | | GIIPIFGTANYAQKFQG |
| 80 | | GGAPAVWSYFDY |
| 81 | | SGSSSNIGNNAVN |
| 82 | | YDDLLPS |
| 83 | | AAWDDSLNGLV |
| 84 | | SYYMH |
| 85 | | IINPSGGSTSYAQKFQG |
| 86 | | DKSLGWFDP |
| 87 | | RASQSISSYLN |
| 88 | | AASSLQS |
| 89 | | QQSYSTPFT |
| 90 | | SYAMS |
| 91 | | AISGSGGSTYYADSVKG |
| 92 | | ELGLRGAFDI |
| 93 | | SGSRSNIGTNTVN |
| 94 | | NNLQRPS |
| 95 | | ATWDDSLKGVV |
| 96 | | DYTMH |
| 97 | | LISWDGGSTYYADSVKG |
| 98 | | DKGWLQLGN |
| 99 | | IGTSSDVGGYNSVS |
| 100 | | DISERPS |
| 101 | | SSYTATSVI |
| 102 | **TPP-57** - **VH** (VR-32) | QVQLVQSGAEVKKPGSSVKVSCKASGGTFSSYAISWVRQA PGQGLEWMGGIIPIFGTANYAQKFQGRVTITADKSTSTAYM ELSSLRSEDTAVYYCARGIKGYAFDIWGQGTMVTVSS |
| 103 | **TPP-57** - **VL** (VR-32) | QSVLTQPPSVSGAPGQRVTISCSGSSSNIGAGYDVHWYQQL PGRAPKLLIYGNSNRPSGVPDRFSGSKSGTSASLAITGLQAE DEADYYCQSYDSSLTGVFGGGTKVTVL |
| 104 | **TPP-58** - **VH** (VR-31) | EVQLLESGGGLVQPGGSLRLSCAASGFNFDSYWMTWVRQ APGKGLEWVANIQQDGSEKYYLDSVKGRFTISRDNTKNSL YLQMNSLRDEDTAVYYCARDWAYFDYWGQGTLVTVSS |
| 105 | **TPP-58** - **VL** (VR-31) | SYELTQPPSVSVSPGQTARITCSGDALPKKYAYWYQQKSG QAPVLVIYGDSKRPSGIPERFSGSSSGTMATLTVSGAQVED EADYYCYSTDSSGNQGVFGGGTKLTVL |
| 106 | **TPP-59** - **VH** (VR-30) | QVQLVQSGAEVKKPGSSVKVSCKASGGTFSSYAISWVRQA PGQGLEWMGGIIPIFGTANYAQKFQGRVTITTDESMSTAY MELSSLRSEDTAVYYCARGGAPAVWSYFDYWGQGTLVTV SS |
| 107 | **TPP-59** - **VL** (VR-30) | QSVLTQPPSVSEAPRQRVTISCSGSSSNIGNNAVNWYQQLP GKAPKLLIYYDDLLPSGVSDRFSGSKSGTSASLAISGLQSED EADYYCAAWDDSLNGLVFGGGTKLTVL |
| 108 | **TPP-60** - **VH** (VR-29) | QVQLVQSGAEVKKPEASVKVSCKASGYTFTSYYMHWVRQ APGQGLEWMGIINPSGGSTSYAQKFQGRVTMTRDTSTSTV YMELSSLRSEDTAVYYCARDKSLGWFDPWGQGTLVTVSS |
| 109 | **TPP-60** - **VL** (VR-29) | DIQMTQSPSSLSASVGDRVTITCRASQSISSYLNWYQQKPG KAPKLLIYAASSLQSGVPSRFSGSGSGTDFTLTISSLQPEDFA TYYCQQSYSTPFTFGPGTKVDIK |
| 110 | **TPP-61** - **VH** (VR-28) | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKELGLRGAFDIWGQGTMVTVSS |
| 111 | **TPP-61** - **VL** (VR-28) | QSVLTQPPSASGTPGQRVTFSCSGSRSNIGTNTVNWYQQLP GTAPKLLIYNNLQRPSGVPDRFSGSRSGTSASLAISGLQSED EAEYYCATWDDSLKGVVFGGGTKVTVL |
| 112 | **TPP-62** - **VH** (VR-27) | EVQLVESGGVVVQPGGSLRLSCAASGFTFDDYTMHWVRQ APGKGLEWVSLISWDGGSTYYADSVKGRFTISRDNSKNSL YLQMNSLRTEDTALYYCAKDKGWLQLGNWGQGTLVTVS S |
| 113 | **TPP-62** - **VL** (VR-27) | QSALTQPASVSGSPGQSITISCIGTSSDVGGYNSVSWYQQYP GRAPKLMIYDISERPSGVSTRFSGSKSGNTASLTISGLQAED EADYYCSSYTATSVIFGGGTKVTVL |
| 114 | VR-106 - VH | QVQLVQSGAEVKKPGSSVKVSCKAPGGTFSSYAISWVRQA PGQGLEWMGGIIPIFGTANYAQKFQGRVTITADKSTSTAYM |
| | | ELSSLRSEDTAVYYCASQTGYSSGWPSPDAFDIWGQGTMV TVSS |
| 115 | VR-106 - VL | DIQMTQSPSSLSASVGDRVTITCRASQGISSYLAWYQQKPG KVPKLLIYAASTLQSGVPSRFSGSGSGTDFTLTISSLQPEDFA TYYCQQLNSYPPTFGPGTKVDIK |
| 116 | | SYAIS |
| 117 | | GIIPIFGTANYAQKFQG |
| 118 | | QTGYSSGWPSPDAFDI |
| 119 | | RASQGISSYLA |
| 120 | | AASTLQS |
| 121 | | QQLNSYPPT |
| 122 | VR-105 - VH | QVQLVQSGAEVKKPGSSVKVSCKASGGTFSSYAISWVRQA PGQGLEWMGGIIPIFGTANYAQKFQGRVTITADESTSTAYM ELSSLRSEDTAVYYCARAVGIFGAFDIWGQGTMVTVSS |
| 123 | VR-105 - VL | QSVLTQPPSASGTPGQRVTISCSGSNSNIGSNTVNWYQQLP GTAPKLLIYSNNQRPSGVPDRFSGSKSGTSASLAISGLQSED EADYYCAAWDDSLNGPYVVFGGGTKLTVL |
| 124 | | SYAIS |
| 125 | | GIIPIFGTANYAQKFQG |
| 126 | | AVGIFGAFDI |
| 127 | | SGSNSNIGSNTVN |
| 128 | | SNNQRPS |
| 129 | | AAWDDSLNGPYVV |
| 130 | VR-104 - VH | QVQLVQSGAEVKKPGSSVKVSCKASGGTFSSYAISWVRQA PGQGLEWMGGIIPIFGTANYAQKFQGRVTITADKSTSTAYM ELSSLRSEDTAVYYCARDSYDSSGYYYDWFDPWGQGTLV TVSS |
| 131 | VR-104 - VL | QSALTQPASVSGSPGQSITISCTGTSSDVGGYDFVSWYQQH PGKAPKLMIYEVSTRHSGVSNRFSASKSGNTASLTISGLQA EDEADYYCSSFTSSPSVVFGGGTKVTVL |
| 132 | | SYAIS |
| 133 | | GIIPIFGTANYAQKFQG |
| 134 | | DSYDSSGYYYDWFDP |
| 135 | | TGTSSDVGGYDFVS |
| 136 | | EVSTRHS |
| 137 | | SSFTSSPSVV |
| 138 | VR-103 - VH - | QIQLVQSGAEVKKPGSSVKVSCKASGGIFSNYAISWVRQAP GQGLEWMGGIIPIFGTANYAQKFQGRVTITADESTSTAYME LSSLRSEDTAVYYCARDYREGYSYGLYDAFDIWGQGTMV TVSS |
| 139 | VR-103 - VL | QSALTQPASVSGSPGQSITISCTGTSSDVGGYNYVSWYQQH PGKAPKLMIYDVSNRPSGVSNRFSGSKSGNTASLTISGLQA EDEADYYCSSYRSSYTLVFGGGTKLTVL |
| 140 | | NYAIS |
| 141 | | GIIPIFGTANYAQKFQG |
| 142 | | DYREGYSYGLYDAFDI |
| 143 | | TGTSSDVGGYNYVS |
| 144 | | DVSNRPS |
| 145 | | SSYRSSYTLV |
| 146 | VR-102 - VH - | QVQLVQSGAEVKKPGSSVKVSCKASGGTFSSYAISWVRQA PGQGLEWMGGIIPIFGTANYAQKFQGRVTITADESTSTAYM ELSSLRSEDTAVYYCARGGLESSYGMDVWGQGTTVTVSS |
| 147 | VR-102 - VL | QSVLTQPPSVSEAPRQRVTISCSGSSSNIGNNAVNWYQQLP GKAPKLLIYYDDLLPSGVSDRFSGSKSGTSASLAISGLQSED EADYYCAAWDDSLSGYVLGTGTKLTVL |
| 148 | | SYAIS |
| 149 | | GIIPIFGTANYAQKFQG |
| 150 | | GGLESSYGMDV |
| 151 | | SGSSSNIGNNAVN |
| 152 | | YDDLLPS |
| 153 | | AAWDDSLSGYV |
| 154 | MAGE-F1 | TVAELVQFV |
| 155 | MRVI1 | KLEELVHFL |
| 156 | EPS8 | SAADLVHFL |
| 157 | PPP2R1 B | GIAELVHFS |
| 158 | DDX28 | KVAELVHIL |
| 159 | MAGE-B18 | KVVSLVHFL |
| 160 | MAGE-C3 | KVAELVQFL |
| 161 | MAGE-A1 | KVADLVGFL |
| 162 | MAGE-C2 | KVAELVEFL |

### Examples

Anti-cancer antigen binding proteins that specifically bind to a MAGE-A3 antigenic peptide comprising or consisting of the amino acid sequence KVAELVHFL of SEQ ID NO: 1 are often of insufficient affinity and specificity when compared to binders targeting e.g. viral antigens, and this may be one possible explanation for tumor immune escape. Therefore, it is desirable to generate new antigen binding proteins with higher affinity and specificity for use as cancer antigen-targeting constructs in a recognition module of a soluble therapeutic drug, such as bispecific molecules (Hickman et al. 2016, J Biomol Screen. 2016 Sep;21(8):769-85). This invention thus relates to the modification and optimization of the properties of such antigen binding proteins. The overall binder may be of particular use in the context of targeted antibody drug conjugates (ADCs) or antibody-dependent cell-mediated cytotoxicity (ADCC) therapeutics.

For the Fc silenced construct, L234A/L235A (LALA), see, for example, Lund J, et al. Human Fc gamma RI and Fc gamma RII interact with distinct but overlapping sites on human IgG. J Immunol. 1991 Oct 15; 147(8):2657-62. PMID: 1833457).

### Identification of binding domains

In order to identify the binders according to the invention, four human scFv phage display libraries were screened. Three panning rounds against the target complex (recombinant HLA-A*0201/MAGE-A3₁₁₂₋₁₂₀) were performed. Hits were identified in ELISA against target complex HLA-A*0201/MAGE-A3₁₁₂₋₁₂₀ and HLA-A*0201 complexed with a non-related peptide with no sequence homology to MAGE-A3₁₁₂₋₁₂₀, HLA-A*0201/EW.

In detail, to select target binders, the phage display (scFv) libraries are screened against biotinylated HLA-A*0201 complexed with MAGE-A3₁₁₂₋₁₂₀ coupled to streptavidin-coated magnetic beads. After three panning rounds, single clones were tested in an ELISA for target complex binding (MAGE-A3₁₁₂₋₁₂₀ /HLA-A*0201) and negative complex binding (EW peptide/HLA-A*0201) to identify non-target specific binders (e.g. HLA-A*0201 only binders). Identified target-specific binders were subjected to DNA sequencing and re-cloned to produce MAGE-A3₁₁₂₋₁₂₀ /HLA-A*0201-specific binders in a desired antibody format.

### Provision of binding domains

The different anti-MAGE-A3 (KVAELVHFL)/HLA-A*02:01 CDR and V_{H} and V_{L}-antibody sequences were as follows:

| Na me | CDR -H1 | CDR-H2 | CDR-H3 | CDR-L1 | CDR-L2 | CDR-L3 |
|---|---|---|---|---|---|---|
| **VR-23** | SYG MH | VISYDGSNK YYADSVKG | DLVGGKTW DYGMDV | RASQSINS YLN | AASS LQS | QQSYSTP RT |
| SE Q ID | 2 | 3 | 4 | 5 | 6 | 7 |
| VR-24 | NY AM T | AISGSRGSTY YADSVKG | DNWNFPFY LDY | RASQGISN WLA | AAS NLQ G | QQGYSFP HT |
| SE Q ID | 8 | 9 | 10 | 11 | 12 | 13 |
| VR-22 | SYA IS | GIIPIFGAAN YAQKFQG | GGLESSYG MDV | SGSSSNIG NNAVN | YDD LLPS | AAWDDSL NGLV |
| SE Q ID | 14 | 15 | 16 | 17 | 18 | 19 |
| VR-32 | SYA IS | GIIPIFGTANY AQKFQG | GIKGYAFDI | SGSSSNIG AGYDVH | GNS NRPS | QSYDSSLT GV |
| SE Q ID | 66 | 67 | 68 | 69 | 70 | 71 |
| VR-31 | SY WM T | NIQQDGSEK YYLDSVKG | DWAYFDY | SGDALPK KYAY | GDS KRPS | YSTDSSG NQGV |
| SE Q ID | 72 | 73 | 74 | 75 | 76 | 77 |
| VR-30 | SYA IS | GIIPIFGTANY AQKFQG | GGAPAVWS YFDY | SGSSSNIG NNAVN | YDD LLPS | AAWDDSL NGLV |
| SE Q ID | 78 | 79 | 80 | 81 | 82 | 83 |
| VR-29 | SYY MH | IINPSGGSTS YAQKFQG | DKSLGWFD P | RASQSISS YLN | AASS LQS | QQSYSTPF T |
| SE Q ID | 84 | 85 | 86 | 87 | 88 | 89 |
| VR-28 | SYA MS | AISGSGGSTY YADSVKG | ELGLRGAF DI | SGSRSNIG TNTVN | NNL QRPS | ATWDDSL KGVV |
| SE Q ID | 90 | 91 | 92 | 93 | 94 | 95 |
| VR-27 | DYT MH | LISWDGGST YYADSVKG | DKGWLQLG N | IGTSSDVG GYNSVS | DISE RPS | SSYTATSV I |
| SE Q ID | 96 | 97 | 98 | 99 | 100 | 101 |
| VR-106 | SYA IS | GIIPIFGTANY AQKFQG | QTGYSSGW PSPDAFDI | RASQGISS YLA | AAST LQS | QQLNSYP PT |
| SE Q ID | 116 | 117 | 118 | 119 | 120 | 121 |
| VR-105 | SYA IS | GIIPIFGTANY AQKFQG | AVGIFGAFD I | SGSNSNIG SNTVN | SNN QRPS | AAWDDSL NGPYVV |
| SE Q ID | 124 | 125 | 126 | 127 | 128 | 129 |
| VR-104 | SYA IS | GIIPIFGTANY AQKFQG | DSYDSSGY YYDWFDP | TGTSSDVG GYDFVS | EVST RHS | SSFTSSPS VV |
| SE Q ID | 132 | 133 | 134 | 135 | 136 | 137 |
| VR-103 | NY AIS | GIIPIFGTANY AQKFQG | DYREGYSY GLYDAFDI | TGTSSDVG GYNYVS | DVS NRPS | SSYRSSYT LV |
| SE Q ID | 140 | 141 | 142 | 143 | 144 | 145 |
| VR-102 | SYA IS | GIIPIFGTANY AQKFQG | GGLESSYG MDV | SGSSSNIG NNAVN | YDD LLPS | AAWDDSL SGYV |
| SE Q ID | 148 | 149 | 150 | 151 | 152 | 153 |

Heavy and light chain CDR sequences of the anti-MAGE-A3/HLA antibodies under the Kabat definition

| Na me | Sequence - SEQ ID NO: |
|---|---|
| VR-23 - VH | QVQLVQSGGGVVQPGRSLRLSCAASGFTFSSYGMHWVRQAPGKGLE WVAVISYDGSNKYYADSVKGRFTISRDNSKNTLYLQMNSLRAEDTAV YYCAKDLVGGKTWDYGMDVWGQGTTVTVSS - SEQ ID NO: 20 |
| VR-23 - VL | DIVMTQPPSSLSASVGDRVTITCRASQSINSYLNWYQQKPGKAPKLLIY AASSLQSGVPSRFSGSGSGTDFTLTISSLQPEDFATYYCQQSYSTPRTFG QGTKVEIK - SEQ ID NO: 21 |
| VR-24 VH | QVQLVQSGGGLVQPGGSLRLSCAASGFTFNNYAMTWVRQAPGKGLE WVSAISGSRGSTYYADSVKGRFTISRDNSKNTLYLEMNSLRAEDTAVY FCAKDNWNFPFYLDYWGQGTLVTVSS - SEQ ID NO: 22 |
| VR-24 - VL | DIVMTQSPSSVSASVGDRVTITCRASQGISNWLAWYQQKPGKAPKLLI YAASNLQGGVPSRFSGSGSGTDFTLTISSLQPEDFATYFCQQGYSFPHT FGQGTKLEIK - SEQ ID NO: 23 |
| VR-22 - VH | QVQLVQSGAEVKKPGSSVKVSCKASGGTFSSYAISWVRQAPGQGLEW MGGIIPIFGAANYAQKFQGRVTITADESTSTAYMELSSLRSEDTAVYYC ARGGLESSYGMDVWGQGTTVTVSS - SEQ ID NO: 24 |
| VR-22 - VL | QSVLTQPPSVSEAPRQRVTISCSGSSSNIGNNAVNWYQQLPGKAPKLLI YYDDLLPSGVSDRFSGSKSGTSASLAISGLQSEDEADYYCAAWDDSLN GLVFGGGTKLTVL - SEQ ID NO: 25 |
| VR-32 - VH | QVQLVQSGAEVKKPGSSVKVSCKASGGTFSSYAISWVRQAPGQGLEW MGGIIPIFGTANYAQKFQGRVTITADKSTSTAYMELSSLRSEDTAVYYC ARGIKGYAFDIWGQGTMVTVSS - SEQ ID NO: 102 |
| VR-32 - VL | QSVLTQPPSVSGAPGQRVTISCSGSSSNIGAGYDVHWYQQLPGRAPKL LIYGNSNRPSGVPDRFSGSKSGTSASLAITGLQAEDEADYYCQSYDSSL TGVFGGGTKVTVL - SEQ ID NO: 103 |
| VR-31 - VH | EVQLLESGGGLVQPGGSLRLSCAASGFNFDSYWMTWVRQAPGKGLE WVANIQQDGSEKYYLDSVKGRFTISRDNTKNSLYLQMNSLRDEDTAV YYCARDWAYFDYWGQGTLVTVSS - SEQ ID NO: 104 |
| VR-31 - VL | SYELTQPPSVSVSPGQTARITCSGDALPKKYAYWYQQKSGQAPVLVIY GDSKRPSGIPERFSGSSSGTMATLTVSGAQVEDEADYYCYSTDSSGNQ GVFGGGTKLTVL - SEQ ID NO: 105 |
| VR-30 - VH | QVQLVQSGAEVKKPGSSVKVSCKASGGTFSSYAISWVRQAPGQGLEW MGGIIPIFGTANYAQKFQGRVTITTDESMSTAYMELSSLRSEDTAVYYC ARGGAPAVWSYFDYWGQGTLVTVSS - SEQ ID NO: 106 |
| VR-30 - VL | QSVLTQPPSVSEAPRQRVTISCSGSSSNIGNNAVNWYQQLPGKAPKLLI YYDDLLPSGVSDRFSGSKSGTSASLAISGLQSEDEADYYCAAWDDSLN GLVFGGGTKLTVL - SEQ ID NO: 107 |
| VR-29 - VH | QVQLVQSGAEVKKPEASVKVSCKASGYTFTSYYMHWVRQAPGQGLE WMGIINPSGGSTSYAQKFQGRVTMTRDTSTSTVYMELSSLRSEDTAVY YCARDKSLGWFDPWGQGTLVTVSS - SEQ ID NO: 108 |
| VR-29 - VL | DIQMTQSPSSLSASVGDRVTITCRASQSISSYLNWYQQKPGKAPKLLIY AASSLQSGVPSRFSGSGSGTDFTLTISSLQPEDFATYYCQQSYSTPFTFG PGTKVDIK - SEQ ID NO: 109 |
| VR-28 - VH | EVQLLESGGGLVQPGGSLRLSCAASGFTFSSYAMSWVRQAPGKGLEW VSAISGSGGSTYYADSVKGRFTISRDNSKNTLYLQMNSLRAEDTAVYY CAKELGLRGAFDIWGQGTMVTVSS - SEQ ID NO: 110 |
| VR-28 - VL | QSVLTQPPSASGTPGQRVTFSCSGSRSNIGTNTVNWYQQLPGTAPKLLI YNNLQRPSGVPDRFSGSRSGTSASLAISGLQSEDEAEYYCATWDDSLK GVVFGGGTKVTVL - SEQ ID NO: 111 |
| VR-27 - VH | EVQLVESGGVVVQPGGSLRLSCAASGFTFDDYTMHWVRQAPGKGLE WVSLISWDGGSTYYADSVKGRFTISRDNSKNSLYLQMNSLRTEDTALY YCAKDKGWLQLGNWGQGTLVTVSS - SEQ ID NO: 112 |
| VR-27 - VL | QSALTQPASVSGSPGQSITISCIGTSSDVGGYNSVSWYQQYPGRAPKLM IYDISERPSGVSTRFSGSKSGNTASLTISGLQAEDEADYYCSSYTATSVIF GGGTKVTVL - SEQ ID NO: 103 |
| VR-106 - VH | QVQLVQSGAEVKKPGSSVKVSCKAPGGTFSSYAISWVRQAPGQGL EWMGGIIPIFGTANYAQKFQGRVTITADKSTSTAYMELSSLRSEDTA VYYCASQTGYSSGWPSPDAFDIWGQGTMVTVSS - SEQ ID NO: 114 |
| VR-106 VL | DIQMTQSPSSLSASVGDRVTITCRASQGISSYLAWYQQKPGKVPKLL IYAASTLQSGVPSRFSGSGSGTDFTLTISSLQPEDFATYYCQQLNSYP PTFGPGTKVDIK - SEQ ID NO: 115 - |
| VR-105 - VH | QVQLVQSGAEVKKPGSSVKVSCKASGGTFSSYAISWVRQAPGQGL EWMGGIIPIFGTANYAQKFQGRVTITADESTSTAYMELSSLRSEDTA VYYCARAVGIFGAFDIWGQGTMVTVSS - SEQ ID NO: 122 |
| VR-105 - VL | QSVLTQPPSASGTPGQRVTISCSGSNSNIGSNTVNWYQQLPGTAPKL LIYSNNQRPSGVPDRFSGSKSGTSASLAISGLQSEDEADYYCAAWD DSLNGPYVVFGGGTKLTVL - SEQ ID NO: 123 |
| VR-104 - VH | QVQLVQSGAEVKKPGSSVKVSCKASGGTFSSYAISWVRQAPGQGL EWMGGIIPIFGTANYAQKFQGRVTITADKSTSTAYMELSSLRSEDTA VYYCARDSYDSSGYYYDWFDPWGQGTLVTVSS - SEQ ID NO: 130 |
| VR-104 - VL | QSALTQPASVSGSPGQSITISCTGTSSDVGGYDFVSWYQQHPGKAP KLMIYEVSTRHSGVSNRFSASKSGNTASLTISGLQAEDEADYYCSSF TSSPSVVFGGGTKVTVL - SEQ ID NO: 131 |
| VR-103 - VH | QIQLVQSGAEVKKPGSSVKVSCKASGGIFSNYAISWVRQAPGQGLE WMGGIIPIFGTANYAQKFQGRVTITADESTSTAYMELSSLRSEDTAV YYCARDYREGYSYGLYDAFDIWGQGTMVTVSS - SEQ ID NO: 138 |
| VR-103 - VL | QSALTQPASVSGSPGQSITISCTGTSSDVGGYNYVSWYQQHPGKAP KLMIYDVSNRPSGVSNRFSGSKSGNTASLTISGLQAEDEADYYCSSY RSSYTLVFGGGTKLTVL - SEQ ID NO: 139 |
| VR-102 - VH | QVQLVQSGAEVKKPGSSVKVSCKASGGTFSSYAISWVRQAPGQGL EWMGGIIPIFGTANYAQKFQGRVTITADESTSTAYMELSSLRSEDTA VYYCARGGLESSYGMDVWGQGTTVTVSS - SEQ ID NO: 146 |
| VR-102 - VL | QSVLTQPPSVSEAPRQRVTISCSGSSSNIGNNAVNWYQQLPGKAPKL LIYYDDLLPSGVSDRFSGSKSGTSASLAISGLQSEDEADYYCAAWD DSLSGYVLGTGTKLTVL - SEQ ID NO: 147 |

### Identification of reference binder molecule

The reference binder molecule (C001511_2, see WO2021/173674) was produced as IgG (Fc silenced, LALA_PG). T2 cells were loaded with MAGE-A3 target peptide at 100µM, and the reference molecule for pHLA-MAGE-A3 (VR-33, BCA0088 (=C-001511_2)) was tested for binding to T2 cells loaded with MAGE-A3 target peptide. The construct BCA0089 was used as isotype control. Antibodies were titrated from 250nM to 0.02nM (in 1:4 dilutions). The peptide loading efficiency was determined using anti-β2M-AF647, Antibody binding was determined using anti-IgG Fc-FITC. As a result, VR-33 (BCA0088 (=C-001511_2)) was used for further assays as reference antibody.

The sequences of the reference (VR-33; BCA0088) were as follows.
VH: 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARDMDTFSMVT
LFDYWGQGTLVTVSS (SEQ ID NO: 63)
VL:

DIQMTQSPSSLSASVGDRVTITCRASQSISSYLNWYQQKPGKAPKLLIYAASSLQ
SGVPSRFSGSGSGTDFTLTISSLQPEDFATYYCQQSYSWPLTFGGGTKVEIK (SEQ
ID NO: 64)

### Characterization of VR-23, VR-24, and VR-22)

In order to identify MAGE-A3 pHLA binders (MAGE-A3₁₁₂₋₁₂₀ /HLA-A*0201), scFv-libraries derived from healthy human donors were screened. After several panning rounds, scFv supernatants were tested in an ELISA for target complex binding (MAGE-A3₁₁₂₋₁₂₀ /HLA-A*0201) and counter-target complex binding (EW peptide/HLA-A*0201) to identify non-target specific binders (e.g. HLA-A*0201 only binders). After screening, target binders were converted to IgG (IgG1, Fc LALA-P/G) and subjected to further analysis.

After conversion to IgG1 (Fc silenced), selected IgG molecules were tested for binding to recombinant target and counter-target pHLA complex (see Figure 3). For all molecules, a binding to pHLA MAGE-A3₁₁₂₋₁₂₀ /HLA-A*0201 was confirmed, with VR-31 and VR-29 showing the weakest binding signals to the recombinant target complex. For VR-23, and to a lesser extent for VR-32, binding to the counter-target complex was detected.

Cancer therapy approaches directed against pHLA MAGE-A3₁₁₂₋₁₂₀ /HLA-A*0201 showed neurotoxicity in clinical development. Off-target activity against closely related peptides originating from MAGE-A12 (KMAELVHFL) and/or EPS8L2 (SAAELVHFL) are suspected to be responsible for this (Morgan RA et al., 2013; Martin AD et al., 2021; WO2021/173674). Selected MAGE-A3₁₁₂₋₁₂₀ /HLA-A*0201 binders were tested against T2 cells loaded with various peptides including discussed off-targets MAGE-A12 (KMAELVHFL) and EPS8L2 (SAAELVHFL) (Figure 4). An unrelated peptide derived from PRAME was used as control to evaluate binding to the HLA complex.

VR-33 (reference) was shown to be more selective for pHLA-A2/MAGE-A3 (KVAELVHFL) over pHLA-A2/EPS8L2 (SAAELVHFL). No major difference between binding to pHLA-A2/MAGE-A3 (KVAELVHFL) and pHLA-A2/MAGE-A12 (KMAELVHFL) was identified, and no binding to pHLA/PRAME (SLLQHLIGL) was found.

VR-27 was more selective for pHLA-A2/MAGE-A3 (KVAELVHFL) over pHLA-A2/EPS8L2 (SAAELVHFL), while no major difference between binding to pHLA-A2/MAGE-A3 (KVAELVHFL) and pHLA-A2/MAGE-A12 (KMAELVHFL) was identified. A lower EC50 value for target complex binding compared to reference molecule VR-33 was found. No binding to pHLA/PRAME (SLLQHLIGL) was identified.

For VR-30 a binding to the target pHLA complex at higher antibody concentrations was shown. Compared to reference molecule VR-33, no binding to off-target complexes pHLA-A2/EPS8L2 (SAAELVHFL) and pHLA-A2/MAGE-A12 (KMAELVHFL) could be found, demonstrating a specificity for pHLA-A2/MAGE-A3 (KVAELVHFL). No binding to pHLA/PRAME (SLLQHLIGL) was found.

VR-22 showed a target pHLA complex binding at higher antibody concentrations, similar to VR-30. Binding to pHLA-A2/MAGE-A12 (KMAELVHFL) was identified over pHLA-A2/MAGE-A3 (KVAELVHFL). When compared to reference molecule VR-33, no binding to the off-target complex pHLA-A2/EPS8L2 (SAAELVHFL) and no binding to pHLA/PRAME (SLLQHLIGL) was found.

For VR-24, a binding to the target pHLA-A2/MAGE-A3 (KVAELVHFL), and a binding to pHLA-A2/MAGE-A12 (KMAELVHFL) slightly above than pHLA-A2/MAGE-A3 (KVAELVHFL) was found. The binder was more selective for pHLA-A2/MAGE-A3 (KVAELVHFL) over pHLA-A2/EPS8L2 (SAAELVHFL). No binding to pHLA/PRAME (SLLQHLIGL) was idnetified.

VR-23 is more selective for pHLA-A2/MAGE-A3 (KVAELVHFL) over pHLA-A2/EPS8L2 (SAAELVHFL). No major difference between binding to pHLA-A2/MAGE-A3 (KVAELVHFL) and pHLA-A2/MAGE-A12 (KMAELVHFL) was fond. A binding to pHLA/PRAME (SLLQHLIGL) wa idnetified at high antibody concentrations.

### Differentiation of HLA-A*0201/MAGE-A3₁₁₂₋₁₂₀ binders according to the invention to reference molecule (Figure 5)

A relevance of positions 8 and 9 for a binding was found, except for VR-30 (only position 8 seems relevant). In VR-33 (reference), the central positions seem to be most relevant for the pHLA/antibody binding. In VR-23 and VR-24, a binding is affected, when the C-terminal amino acid positions of the target peptide were replaced by alanine. For VR-22, VR-30, and VR27, amino acids distributed across the target peptide sequence are important for pHLA/antibody binding (N-terminal-Central-C-terminal).

For VR-22, positions 1, 4, 6, and 8 and 9 are critical. For VR-30, almost all positions are involved in antibody binding, with a minor influence for position 3 and 6 and no influence of position 9. VR-27 shows a strong influence on binding of positions 1, 3, 5-9, and a minor influence by positions 2 and 4.

As can be seen in Figure 6, VR-33 (reference) showed no recognition of HLA-A*0201 in complex with MAGE-A1, -A4, -A10 and -A11, and a reduced binding to HLA-A*0201 in complex with MAGE-A2, -A5, -A6 and -A8. Not selectivity against HLA-A*0201/MAGE-A12 was found.

Compared to VR-33, VR-27 showed no binding to HLA-A*0201/MAGE-A5. No or only a minor recognition of HLA-A*0201 in complex with MAGE-A1, -A4, -A6, -A8, -A10 and -A11 was found. No selectivity for HLA-A*0201/MAGE-A2 and -A12 was identified.

VR-30 was found to be most selective. Besides binding to HLA-A*0201/MAGE-A3 target complex, only a binding to HLA-A*0201 in complex with MAGE-A4 was identified, and a reduced recognition of HLA-A*0201/MAGE-A2 and -A11. VR-30 is the only construct not binding to HLA-A*0201/MAGE-A12.

For VR-22, no binding to HLA-A*0201 in complex with MAGE-A1, -A4, -A5, -A6 and -A11 and no selectivity against MAGE-A2, -A8 -A10 and -A12 was found.

For VR-24, no binding to HLA-A*0201 in complex with MAGE-A4 and a reduced binding of MAGE-A1, -A5, -A6, -A8, -A11 and no selectivity for HLA-A*0201/MAGE-A12 was found.

For VR-23, only a minor selectivity against HLA-A*0201 in complex with MAGE-A-family members and a reduced binding only to HLA-A*0201 in complex with MAGE-A1, -A4 and -A8 was found.

As shown in Figure 7, the HLA-A*0201/MAGE-A3₁₁₂₋₁₂₀ binders according to the invention showed no or only minor recognition of the DDX28 peptide, compared to VR-33. When compared to reference VR-33, no binding to HLA-A*0201/MAGE-F1 was found for VR-22, VR-30, and VR-27.

## Claims

1. An antigen binding protein which specifically binds to a MAGE-A antigenic peptide comprising or consisting of the amino acid sequence KVAELVHFL of SEQ ID NO: 1, wherein said antigenic peptide is in a complex with a major histocompatibility complex (MHC) protein, the antigen binding protein comprising a polypeptide chain comprising a first variable domain comprising three complementary determining regions (CDRs) CDRH1, CDRH2 and CDRH3, wherein
i) the CDRH1 comprises or consists of the amino acid sequence DYTMH (SEQ ID NO: 96), or an amino acid sequence differing from SEQ ID NO: 96 by one or two amino acid substitutions,
the CDRH2 comprises or consists of the amino acid sequence LISWDGGSTYYADSVKG (SEQ ID NO: 97), or an amino acid sequence differing from SEQ ID NO: 97 by one or two amino acid substitutions,
the CDRH3 comprises or consists of the amino acid sequence DKGWLQLGN (SEQ ID NO: 98), or an amino acid sequence differing from SEQ ID NO: 98 by one or two amino acid substitutions, or
ii) the CDRH1 comprises or consists of the amino acid sequence SYAIS (SEQ ID NO: 78), or an amino acid sequence differing from SEQ ID NO: 78 by one or two amino acid substitutions,
the CDRH2 comprises or consists of the amino acid sequence GIIPIFGTANYAQKFQG (SEQ ID NO: 79), or an amino acid sequence differing from SEQ ID NO: 79 by one or two amino acid substitutions,
the CDRH3 comprises or consists of the amino acid sequence GGAPAVWSYFDY (SEQ ID NO: 80), or an amino acid sequence differing from SEQ ID NO: 80 by one or two amino acid substitutions, or
iii) the CDRH1 comprises or consists of the amino acid sequence SYGMH (SEQ ID NO: 2), or an amino acid sequence differing from SEQ ID NO: 2 by one or two amino acid substitutions,
the CDRH2 comprises or consists of the amino acid sequence VISYDGSNKYYADSVKG (SEQ ID NO: 3), or an amino acid sequence differing from SEQ ID NO: 3 by one or two amino acid substitutions,
the CDRH3 comprises or consists of the amino acid sequence DLVGGKTWDYGMDV (SEQ ID NO: 4), or an amino acid sequence differing from SEQ ID NO: 4 by one or two amino acid substitutions, or
iv) the CDRH1 comprises or consists of the amino acid sequence NYAMT (SEQ ID NO: 8), or an amino acid sequence differing from SEQ ID NO: 8 by one or two amino acid substitutions,
the CDRH2 comprises or consists of the amino acid sequence AISGSRGSTYYADSVKG (SEQ ID NO: 9), or an amino acid sequence differing from SEQ ID NO: 9 by one or two amino acid substitutions,
the CDRH3 comprises or consists of the amino acid sequence DNWNFPFYLDY (SEQ ID NO: 10), or an amino acid sequence differing from SEQ ID NO: 10 by one or two amino acid substitutions, or
v) the CDRH1 comprises or consists of the amino acid sequence SYAIS (SEQ ID NO: 14), or an amino acid sequence differing from SEQ ID NO: 14 by one or two amino acid substitutions,
the CDRH2 comprises or consists of the amino acid sequence GIIPIFGAANYAQKFQG (SEQ ID NO: 15), or an amino acid sequence differing from SEQ ID NO: 15 by one or two amino acid substitutions,
the CDRH3 comprises or consists of the amino acid sequence GGLESSYGMDV (SEQ ID NO: 16), or an amino acid sequence differing from SEQ ID NO: 16 by one or two amino acid substitutions, or
vi) the CDRH1 comprises or consists of the amino acid sequence SYAIS (SEQ ID NO: 66), or an amino acid sequence differing from SEQ ID NO: 66 by one or two amino acid substitutions,
the CDRH2 comprises or consists of the amino acid sequence GIIPIFGTANYAQKFQG (SEQ ID NO: 67), or an amino acid sequence differing from SEQ ID NO: 67 by one or two amino acid substitutions,
the CDRH3 comprises or consists of the amino acid sequence GIKGYAFDI (SEQ ID NO: 68), or an amino acid sequence differing from SEQ ID NO: 68 by one or two amino acid substitutions, or
vii) the CDRH1 comprises or consists of the amino acid sequence SYWMT (SEQ ID NO: 72), or an amino acid sequence differing from SEQ ID NO: 72 by one or two amino acid substitutions,
the CDRH2 comprises or consists of the amino acid sequence NIQQDGSEKYYLDSVKG (SEQ ID NO: 73), or an amino acid sequence differing from SEQ ID NO: 73 by one or two amino acid substitutions,
the CDRH3 comprises or consists of the amino acid sequence DWAYFDY (SEQ ID NO: 74), or an amino acid sequence differing from SEQ ID NO: 74 by one or two amino acid substitutions, or
viii) the CDRH1 comprises or consists of the amino acid sequence SYYMH (SEQ ID NO: 84), or an amino acid sequence differing from SEQ ID NO: 84 by one or two amino acid substitutions,
the CDRH2 comprises or consists of the amino acid sequence IINPSGGSTSYAQKFQG (SEQ ID NO: 85), or an amino acid sequence differing from SEQ ID NO: 85 by one or two amino acid substitutions,
the CDRH3 comprises or consists of the amino acid sequence DKSLGWFDP (SEQ ID NO: 86), or an amino acid sequence differing from SEQ ID NO: 86 by one or two amino acid substitutions, or
ix) the CDRH1 comprises or consists of the amino acid sequence SYAMS (SEQ ID NO: 90), or an amino acid sequence differing from SEQ ID NO: 90 by one or two amino acid substitutions,
the CDRH2 comprises or consists of the amino acid sequence AISGSGGSTYYADSVKG (SEQ ID NO: 91), or an amino acid sequence differing from SEQ ID NO: 91 by one or two amino acid substitutions,
the CDRH3 comprises or consists of the amino acid sequence ELGLRGAFDI (SEQ ID NO: 92), or an amino acid sequence differing from SEQ ID NO: 92 by one or two amino acid substitutions, or
x) the CDRH1 comprises or consists of the amino acid sequence SYAIS (SEQ ID NO: 116), or an amino acid sequence differing from SEQ ID NO: 116 by one or two amino acid substitutions,
the CDRH2 comprises or consists of the amino acid sequence GIIPIFGTANYAQKFQG (SEQ ID NO: 117), or an amino acid sequence differing from SEQ ID NO: 117 by one or two amino acid substitutions,
the CDRH3 comprises or consists of the amino acid sequence QTGYSSGWPSPDAFDI (SEQ ID NO: 118), or an amino acid sequence differing from SEQ ID NO: 118 by one or two amino acid substitutions, or
xi) the CDRH1 comprises or consists of the amino acid sequence SYAIS (SEQ ID NO: 124), or an amino acid sequence differing from SEQ ID NO: 124 by one or two amino acid substitutions,
the CDRH2 comprises or consists of the amino acid sequence GIIPIFGTANYAQKFQG (SEQ ID NO: 125), or an amino acid sequence differing from SEQ ID NO: 125 by one or two amino acid substitutions,
the CDRH3 comprises or consists of the amino acid sequence AVGIFGAFDI (SEQ ID NO: 126), or an amino acid sequence differing from SEQ ID NO: 126 by one or two amino acid substitutions, or
xii) the CDRH1 comprises or consists of the amino acid sequence SYAIS (SEQ ID NO: 132), or an amino acid sequence differing from SEQ ID NO: 132 by one or two amino acid substitutions,
the CDRH2 comprises or consists of the amino acid sequence GIIPIFGTANYAQKFQG (SEQ ID NO: 133), or an amino acid sequence differing from SEQ ID NO: 133 by one or two amino acid substitutions,
the CDRH3 comprises or consists of the amino acid sequence DSYDSSGYYYDWFDP (SEQ ID NO: 134), or an amino acid sequence differing from SEQ ID NO: 134 by one or two amino acid substitutions, or
xiii) the CDRH1 comprises or consists of the amino acid sequence NYAIS (SEQ ID NO: 140), or an amino acid sequence differing from SEQ ID NO: 140 by one or two amino acid substitutions,
the CDRH2 comprises or consists of the amino acid sequence GIIPIFGTANYAQKFQG (SEQ ID NO: 141), or an amino acid sequence differing from SEQ ID NO: 141 by one or two amino acid substitutions,
the CDRH3 comprises or consists of the amino acid sequence DYREGYSYGLYDAFDI (SEQ ID NO: 142), or an amino acid sequence differing from SEQ ID NO: 142 by one or two amino acid substitutions, or
xiv) the CDRH1 comprises or consists of the amino acid sequence SYAIS (SEQ ID NO: 148), or an amino acid sequence differing from SEQ ID NO: 148 by one or two amino acid substitutions,
the CDRH2 comprises or consists of the amino acid sequence GIIPIFGTANYAQKFQG (SEQ ID NO: 149), or an amino acid sequence differing from SEQ ID NO: 149 by one or two amino acid substitutions,
the CDRH3 comprises or consists of the amino acid sequence GGLESSYGMDV (SEQ ID NO: 150), or an amino acid sequence differing from SEQ ID NO: 150 by one or two amino acid substitutions.

2. The antigen binding protein according to claim 1, further comprising a second polypeptide chain comprising a second variable domain comprising three CDRs CDRLl, CDRL2 and CDRL3, wherein
a) the CDRLl comprises or consists of the amino acid sequence IGTSSDVGGYNSVS (SEQ ID NO: 99), or an amino acid sequence differing from SEQ ID NO: 99 by one or two amino acid substitutions,
the CDRL2 comprises or consists of the amino acid sequence DISERPS (SEQ ID NO: 100), or an amino acid sequence differing from SEQ ID NO: 100 by one or two amino acid substitutions, and
the CDRL3 comprises or consists of the amino acid sequence SSYTATSVI (SEQ ID NO: 101), or an amino acid sequence differing from SEQ ID NO: 101 by one or two amino acid substitutions, or
b) the CDRLl comprises or consists of the amino acid sequence SGSSSNIGNNAVN (SEQ ID NO: 81), or an amino acid sequence differing from SEQ ID NO: 81 by one or two amino acid substitutions,
the CDRL2 comprises or consists of the amino acid sequence YDDLLPS (SEQ ID NO: 82), or an amino acid sequence differing from SEQ ID NO: 82 by one or two amino acid substitutions, and
the CDRL3 comprises or consists of the amino acid sequence AAWDDSLNGLV (SEQ ID NO: 83), or an amino acid sequence differing from SEQ ID NO: 83 by one or two amino acid substitutions, or
c) the CDRLl comprises or consists of the amino acid sequence RASQSINSYLN (SEQ ID NO: 5), or an amino acid sequence differing from SEQ ID NO: 5 by one or two amino acid substitutions,
the CDRL2 comprises or consists of the amino acid sequence AASSLQS (SEQ ID NO: 6), or an amino acid sequence differing from SEQ ID NO: 6 by one or two amino acid substitutions,
and the CDRL3 comprises or consists of the amino acid sequence QQSYSTPRT (SEQ ID NO: 7), or an amino acid sequence differing from SEQ ID NO: 7 by one or two amino acid substitutions, or
d) the CDRLl comprises or consists of the amino acid sequence RASQGISNWLA (SEQ ID NO: 11), or an amino acid sequence differing from SEQ ID NO: 11 by one or two amino acid substitutions,
the CDRL2 comprises or consists of the amino acid sequence AASNLQG (SEQ ID NO: 12), or an amino acid sequence differing from SEQ ID NO: 12 by one or two amino acid substitutions,
and the CDRL3 comprises or consists of the amino acid sequence QQGYSFPHT (SEQ ID NO: 13), or an amino acid sequence differing from SEQ ID NO: 13 by one or two amino acid substitutions, or
e) the CDRLl comprises or consists of the amino acid sequence SGSSSNIGNNAVN (SEQ ID NO: 17), or an amino acid sequence differing from SEQ ID NO: 17 by one or two amino acid substitutions,
the CDRL2 comprises or consists of the amino acid sequence YDDLLPS (SEQ ID NO: 18), or an amino acid sequence differing from SEQ ID NO: 18 by one or two amino acid substitutions,
and the CDRL3 comprises or consists of the amino acid sequence AAWDDSLNGLV (SEQ ID NO: 19), or an amino acid sequence differing from SEQ ID NO: 19 by one or two amino acid substitutions, or
f) the CDRL1 comprises or consists of the amino acid sequence SGSSSNIGAGYDVH (SEQ ID NO: 69), or an amino acid sequence differing from SEQ ID NO: 69 by one or two amino acid substitutions,
the CDRL2 comprises or consists of the amino acid sequence GNSNRPS (SEQ ID NO: 70), or an amino acid sequence differing from SEQ ID NO: 70 by one or two amino acid substitutions, and
the CDRL3 comprises or consists of the amino acid sequence QSYDSSLTGV (SEQ ID NO: 71), or an amino acid sequence differing from SEQ ID NO: 71 by one or two amino acid substitutions, or
g) the CDRLl comprises or consists of the amino acid sequence SGDALPKKYAY (SEQ ID NO: 75), or an amino acid sequence differing from SEQ ID NO: 75 by one or two amino acid substitutions,
the CDRL2 comprises or consists of the amino acid sequence GDSKRPS (SEQ ID NO: 76), or an amino acid sequence differing from SEQ ID NO: 76 by one or two amino acid substitutions, and
the CDRL3 comprises or consists of the amino acid sequence YSTDSSGNQGV (SEQ ID NO: 77), or an amino acid sequence differing from SEQ ID NO: 77 by one or two amino acid substitutions, or
h) the CDRLl comprises or consists of the amino acid sequence RASQSISSYLN (SEQ ID NO: 87), or an amino acid sequence differing from SEQ ID NO: 87 by one or two amino acid substitutions,
the CDRL2 comprises or consists of the amino acid sequence AASSLQS (SEQ ID NO: 88), or an amino acid sequence differing from SEQ ID NO: 88 by one or two amino acid substitutions,
and the CDRL3 comprises or consists of the amino acid sequence QQSYSTPFT (SEQ ID NO: 89), or an amino acid sequence differing from SEQ ID NO: 89 by one or two amino acid substitutions, or
i) the CDRLl comprises or consists of the amino acid sequence SGSRSNIGTNTVN (SEQ ID NO: 93), or an amino acid sequence differing from SEQ ID NO: 93 by one or two amino acid substitutions,
the CDRL2 comprises or consists of the amino acid sequence NNLQRPS (SEQ ID NO: 94), or an amino acid sequence differing from SEQ ID NO: 94 by one or two amino acid substitutions, and
the CDRL3 comprises or consists of the amino acid sequence ATWDDSLKGVV (SEQ ID NO: 95), or an amino acid sequence differing from SEQ ID NO: 95 by one or two amino acid substitutions,, or
j) the CDRLl comprises or consists of the amino acid sequence RASQGISSYLA (SEQ ID NO: 119), or an amino acid sequence differing from SEQ ID NO: 119 by one or two amino acid substitutions,
the CDRL2 comprises or consists of the amino acid sequence AASTLQS (SEQ ID NO: 120), or an amino acid sequence differing from SEQ ID NO: 120 by one or two amino acid substitutions, and
the CDRL3 comprises or consists of the amino acid sequence QQLNSYPPT (SEQ ID NO: 121), or an amino acid sequence differing from SEQ ID NO: 121 by one or two amino acid substitutions, or
k) the CDRLl comprises or consists of the amino acid sequence SGSNSNIGSNTVN (SEQ ID NO: 127), or an amino acid sequence differing from SEQ ID NO: 127 by one or two amino acid substitutions,
the CDRL2 comprises or consists of the amino acid sequence SNNQRPS (SEQ ID NO: 128), or an amino acid sequence differing from SEQ ID NO: 128 by one or two amino acid substitutions, and
the CDRL3 comprises or consists of the amino acid sequence AAWDDSLNGPYVV (SEQ ID NO: 129), or an amino acid sequence differing from SEQ ID NO: 129 by one or two amino acid substitutions, or
l) the CDRLl comprises or consists of the amino acid sequence TGTSSDVGGYDFVS (SEQ ID NO: 135), or an amino acid sequence differing from SEQ ID NO: 135 by one or two amino acid substitutions,
the CDRL2 comprises or consists of the amino acid sequence EVSTRHS (SEQ ID NO: 136), or an amino acid sequence differing from SEQ ID NO: 136 by one or two amino acid substitutions, and
the CDRL3 comprises or consists of the amino acid sequence SSFTSSPSVV (SEQ ID NO: 137), or an amino acid sequence differing from SEQ ID NO: 137 by one or two amino acid substitutions, or
m) the CDRLl comprises or consists of the amino acid sequence TGTSSDVGGYNYVS (SEQ ID NO: 143), or an amino acid sequence differing from SEQ ID NO: 143 by one or two amino acid substitutions,
the CDRL2 comprises or consists of the amino acid sequence DVSNRPS (SEQ ID NO: 144), or an amino acid sequence differing from SEQ ID NO: 144 by one or two amino acid substitutions, and
the CDRL3 comprises or consists of the amino acid sequence SSYRSSYTLV (SEQ ID NO: 145), or an amino acid sequence differing from SEQ ID NO: 145 by one or two amino acid substitutions, or
n) the CDRLl comprises or consists of the amino acid sequence SGSSSNIGNNAVN (SEQ ID NO: 151), or an amino acid sequence differing from SEQ ID NO: 151 by one or two amino acid substitutions,
the CDRL2 comprises or consists of the amino acid sequence YDDLLPS (SEQ ID NO: 152), or an amino acid sequence differing from SEQ ID NO: 152 by one or two amino acid substitutions, and
the CDRL3 comprises or consists of the amino acid sequence AAWDDSLSGYV (SEQ ID NO: 153), or an amino acid sequence differing from SEQ ID NO: 153 by one or two amino acid substitutions.

3. The antigen binding protein according to claim 1 or 2, comprising an amino acid sequence having at least 75%, preferably at least 90%, and more preferably at least 95% or at least 97%, 98% or 99/ sequence identity to a sequence selected from
i) 
EVQLVESGGVVVQPGGSLRLSCAASGFTFDDYTMHWVRQAPGKGLEWVSLIS
WDGGSTYYADSVKGRFTISRDNSKNSLYLQMNSLRTEDTALYYCAKDKGWLQ
LGNWGQGTLVTVSS (SEQ ID NO: 112),
 and
a) 
QSALTQPASVSGSPGQSITISCIGTSSDVGGYNSVSWYQQYPGRAPKLMIYDISE
RPSGVSTRFSGSKSGNTASLTISGLQAEDEADYYCSSYTATSVIFGGGTKVTVL
(SEQ ID NO: 103),
ii) 
QVQLVQSGAEVKKPGSSVKVSCKASGGTFSSYAISWVRQAPGQGLEWMGGIIPI
FGTANYAQKFQGRVTITTDESMSTAYMELSSLRSEDTAVYYCARGGAPAVWS
YFDYWGQGTLVTVSS (SEQ ID NO: 106),
 and
b) 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 107),
iii) 
QVQLVQSGGGVVQPGRSLRLSCAASGFTFSSYGMHWVRQAPGKGLEWVAVIS
YDGSNKYYADSVKGRFTISRDNSKNTLYLQMNSLRAEDTAVYYCAKDLVGGK
TWDYGMDVWGQGTTVTVSS (SEQ ID NO: 20),
 and
c) 
DIVMTQPPSSLSASVGDRVTITCRASQSINSYLNWYQQKPGKAPKLLIYAASSLQ
SGVPSRFSGSGSGTDFTLTISSLQPEDFATYYCQQSYSTPRTFGQGTKVEIK (SEQ
ID NO: 21),
iv) QVQLVQSGGGLVQPGGSLRLSCAASGFTFNNYAMTWVRQAPGKGLEWVSAIS 
GSRGSTYYADSVKGRFTISRDNSKNTLYLEMNSLRAEDTAVYFCAKDNWNFPF
YLDYWGQGTLVTVSS (SEQ ID NO: 22),
 and
d) 
DIVMTQSPSSVSASVGDRVTITCRASQGISNWLAWYQQKPGKAPKLLIYAASNL
QGGVPSRFSGSGSGTDFTLTISSLQPEDFATYFCQQGYSFPHTFGQGTKLEIK(SE
Q ID NO: 23),
 and
v) 
QVQLVQSGAEVKKPGSSVKVSCKASGGTFSSYAISWVRQAPGQGLEWMGGIIPI
FGAANYAQKFQGRVTITADESTSTAYMELSSLRSEDTAVYYCARGGLESSYGM
DVWGQGTTVTVSS (SEQ ID NO: 24),
 and
e) 
QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLNGLVFGGGTKLT
VL (SEQ ID NO: 25),
vi) 
QVQLVQSGAEVKKPGSSVKVSCKASGGTFSSYAISWVRQAPGQGLEWMGGIIPI
FGTANYAQKFQGRVTITADKSTSTAYMELSSLRSEDTAVYYCARGIKGYAFDI
WGQGTMVTVSS (SEQ ID NO: 102),
 and
f) 
QSVLTQPPSVSGAPGQRVTISCSGSSSNIGAGYDVHWYQQLPGRAPKLLIYGNS
NRPSGVPDRFSGSKSGTSASLAITGLQAEDEADYYCQSYDSSLTGVFGGGTKVT
VL (SEQ ID NO: 103),
vii) 
EVQLLESGGGLVQPGGSLRLSCAASGFNFDSYWMTWVRQAPGKGLEWVANIQ
QDGSEKYYLDSVKGRFTISRDNTKNSLYLQMNSLRDEDTAVYYCARDWAYFD
YWGQGTLVTVSS (SEQ ID NO: 104),
 and
g) 
SYELTQPPSVSVSPGQTARITCSGDALPKKYAYWYQQKSGQAPVLVIYGDSKRP
SGIPERFSGSSSGTMATLTVSGAQVEDEADYYCYSTDSSGNQGVFGGGTKLTVL
(SEQ ID NO: 105),
viii) QVQLVQSGAEVKKPEASVKVSCKASGYTFTSYYMHWVRQAPGQGLEWMGIIN 
PSGGSTSYAQKFQGRVTMTRDTSTSTVYMELSSLRSEDTAVYYCARDKSLGWF
DPWGQGTLVTVSS (SEQ ID NO: 108),
 and
h) 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPFTFGPGTKVDIK (SEQ
ID NO: 109),
ix) 
EVQLLESGGGLVQPGGSLRLSCAASGFTFSSYAMSWVRQAPGKGLEWVSAISG
SGGSTYYADSVKGRFTISRDNSKNTLYLQMNSLRAEDTAVYYCAKELGLRGAF
DIWGQGTMVTVSS (SEQ ID NO: 110),
 and
i) 
QSVLTQPPSASGTPGQRVTFSCSGSRSNIGTNTVNWYQQLPGTAPKLLIYNNLQ
RPSGVPDRFSGSRSGTSASLAISGLQSEDEAEYYCATWDDSLKGVVFGGGTKVT
VL (SEQ ID NO: 111),
x) 
QVQLVQSGAEVKKPGSSVKVSCKAPGGTFSSYAISWVRQAPGQGLEWMGGIIPI
FGTANYAQKFQGRVTITADKSTSTAYMELSSLRSEDTAVYYCASQTGYSSGWP
SPDAFDIWGQGTMVTVSS (SEQ ID NO: 114),
 and
j) 
DIQMTQSPSSLSASVGDRVTITCRASQGISSYLAWYQQKPGKVPKLLIYAASTLQ
SGVPSRFSGSGSGTDFTLTISSLQPEDFATYYCQQLNSYPPTFGPGTKVDIK (SEQ
ID NO: 115),
xi) 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARAVGIFGAFDI
WGQGTMVTVSS (SEQ ID NO: 122),
 and
k) 
QSVLTQPPSASGTPGQRVTISCSGSNSNIGSNTVNWYQQLPGTAPKLLIYSNNQR
PSGVPDRFSGSKSGTSASLAISGLQSEDEADYYCAAWDDSLNGPYVVFGGGTK
LTVL (SEQ ID NO: 123),
xii) QVQLVQSGAEVKKPGSSVKVSCKASGGTFSSYAISWVRQAPGQGLEWMGGIIPI 
FGTANYAQKFQGRVTITADKSTSTAYMELSSLRSEDTAVYYCARDSYDSSGYY
YDWFDPWGQGTLVTVSS (SEQ ID NO: 130),
 and
l) 
QSALTQPASVSGSPGQSITISCTGTSSDVGGYDFVSWYQQHPGKAPKLMIYEVS
TRHSGVSNRFSASKSGNTASLTISGLQAEDEADYYCSSFTSSPSVVFGGGTKVTV
L (SEQ ID NO: 131),
 and
xiii) 
QIQLVQSGAEVKKPGSSVKVSCKASGGIFSNYAISWVRQAPGQGLEWMGGIIPI
FGTANYAQKFQGRVTITADESTSTAYMELSSLRSEDTAVYYCARDYREGYSYG
LYDAFDIWGQGTMVTVSS (SEQ ID NO: 138),
 and
m) 
QSALTQPASVSGSPGQSITISCTGTSSDVGGYNYVSWYQQHPGKAPKLMIYDVS
NRPSGVSNRFSGSKSGNTASLTISGLQAEDEADYYCSSYRSSYTLVFGGGTKLT
VL (SEQ ID NO: 139),
xiv) 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARGGLESSYGM
DVWGQGTTVTVSS (SEQ ID NO: 146),
 and
n)

QSVLTQPPSVSEAPRQRVTISCSGSSSNIGNNAVNWYQQLPGKAPKLLIYYDDL
LPSGVSDRFSGSKSGTSASLAISGLQSEDEADYYCAAWDDSLSGYVLGTGTKLT
VL (SEQ ID NO: 147).

4. The antigen binding protein according to any one of claims 1 to 3, wherein said antigen binding protein specifically binds to an epitope comprising or consisting of at least three amino acid positions of the MAGE-A3 antigenic peptide of SEQ ID NO: 1, preferably, at least three amino acid positions selected from the group of amino acid positions consisting of positions 1 to 6 and 8 or 9 or 6, 8 and 9, with a selection including position 6 being most preferred, of the amino acid sequence of SEQ ID NO: 1, and/or wherein said antigen binding protein specifically binds to the amino acid sequence KVAELVHFL of the antigenic peptide of SEQ ID NO: 1 and wherein said antigenic peptide is in a complex with a human leukocyte antigen (HLA) protein, preferably HLA-A*02, in particular HLA-A*02:01.

5. The antigen binding protein according to any one of claims 1 to 4, wherein said antigen binding protein has an EC₅₀ value for MAGE-A3/MHC complex presenting cells which is less than 200 nM, less than about 120 nM, such as less than 10 nM. or such as between 1 nM and 200 nM, or between 1 nM and 120 nM.

6. The antigen binding protein according to any one of claims 1 to 5, wherein the antigen binding protein is an antibody or a binding fragment thereof, a bispecific, trispecific (bi-paratopic), multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain or nanobody, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).

7. The antigen binding protein according to any one of claims 1 to 6, further comprising a half-life extending domain, such as, for example, an Fc fragment, preferably comprising one or more amino acid substitutions which remove a glycosylation site, wherein preferably the substitution is an N to Q substitution, more preferably wherein the half-life extending domain comprises one or more amino acid substitutions which facilitate dimerization of the amino acid sequences forming the Fc fragment, such as the C_{H}2 domains and/or C_{H}3 domains, in particular S228P (numbered according to the EU numbering scheme), in particular wherein one of the domains comprises one or more amino acid substitutions selected from the group consisting of S228P, F234A, L234A, L235A, R409K, M252Y, S254T, T256E, Y349C, T366S, L368A, Y407V, S354C, and T366W (numbered according to the EU numbering scheme).

8. The antigen binding protein according to claim 7, wherein the half-life extending domain comprises one or more amino acid substitutions which promote binding to FcRn, for example an M252Y, S254T and/or T256E amino acid substitution (numbered according to the EU numbering scheme).

9. A nucleic acid molecule encoding an antigen binding protein according to any one of claims 1 to 8 or at least one component thereof, a vector comprising the nucleic acid molecule, such as, for example, an expression vector comprising and expressing the nucleic acid molecule, or a recombinant or genetically modified cell comprising and optionally expressing the nucleic acid molecule or vector, wherein preferably the cell is a T cell, a hybridoma cell, or an antibody excreting cell.

10. A method of producing a pharmaceutical composition comprising formulating the antigen binding protein according to any one of claims 1 to 8 or the recombinant or genetically modified host cell according to claim 9 with a suitable pharmaceutically acceptable carrier, or a pharmaceutical composition as produced accordingly.

11. The antigen binding protein according to any one of claims 1 to 8, the recombinant or genetically modified host cell according to claim 9 or the pharmaceutical composition according to claim 10 for use as a medicament, preferably for use in the prevention or treatment of a cancer and/or a metastasis thereof, wherein preferably the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer.

12. A method for eliciting a CD8-positive T-cell response in a subject, comprising administering to said subject the pharmaceutical composition according to claim 10.

13. A method of preventing or treating a cancer cell expressing MAGE-A3 and/or a metastasis thereof, the method comprising administering to a subject suffering or prospectively suffering from said cancer and/or a metastasis thereof an effective amount of the antigen binding protein according to any one of claims 1 to 8, the recombinant or genetically modified host cell according to claim 9 or the pharmaceutical composition according to claim 10, wherein preferably the method is combined with a further anti-senescence and/or cancer treatment, wherein preferably the further treatment is selected from the group consisting of chemotherapy, radiotherapy, a treatment with immune-stimulating substances, gene therapy, a treatment with antibodies and a treatment using suitable dendritic cells, wherein preferably the cancer expresses MAGE-A3 and/or is selected from the group consisting of melanoma, head and neck cancer, bladder cancer, breast cancer, colorectal cancer, lung cancer, gastric cancer, non-small cell lung cancer, hematologic malignancies, ovarian cancer, osteosarcoma, hepatocarcinoma, and renal cell cancer.

14. A kit comprising at least one of the antigen binding proteins according to any one of claims 1 to 8, the recombinant or genetically modified host cell according to claim 9 or the pharmaceutical composition according to claim 10, and auxiliary agents and/or instructions for use thereof.

15. Use of a kit according to claim 14 for producing an antigen binding protein according to any one of claims 1 to 8, for producing a pharmaceutical composition according to claim 10, for eliciting a CD8-positive T-cell response in a subject according to claim 11, or for method of preventing or treating a cancer cell and/or a metastasis thereof according to claim 12.
